# EUROPEAN PATENT APPLICATION

(11) **EP 4 079 374 A1**
(43) Date of publication of application: **26.10.2022**
(21) Application number: 20903912.2
(22) Date of filing: 19.12.2020
(51) Int. Cl.: A61P 25/28, A61P 31/18, A61P 43/00, C12P 17/16, C12N 5/10, C12N 1/15, C12N 1/19, C12N 1/21, C12N 15/53, C12N 15/54, C12N 9/10, A61K 31/192, A61K 31/7056

(54) **METHOD FOR SYNTHESIZING CELOSIANIN II, METHOD FOR SYNTHESIZING BETAXANTHIN, AMYLOID-ß POLYMERIZATION INHIBITOR OR THERAPEUTIC OR PROPHYLACTIC AGENT FOR ALZHEIMER'S DISEASE, AMYLOID PEPTIDE AGGREGATION INHIBITOR, AND HIV-1 PROTEASE ACTIVITY INHIBITOR**

(30) Priority: 19.12.2019 JP 2019229647; 11.07.2020 JP 2020119563
(71) Applicant: Mori, Masashi, Nonoichi-shi, Ishikawa, 921-8833 (JP)
(72) Inventor: IMAMURA Tomohiro, Nonoichi-shi, Ishikawa 921-8836 (JP); TAKIZAKI Izumi, Nonoichi-shi, Ishikawa 921-8836 (JP); MIZUNO Akiko, Nonoichi-shi, Ishikawa 921-8836 (JP); HIGASHIMURA Yasuki, Nonoichi-shi, Ishikawa 921-8836 (JP); KOGA Hironori, Nonoichi-shi, Ishikawa 921-8836 (JP); MIZUKOSHI Hiroharu, Hakusan-shi, Ishikawa 924-0053 (JP); MORI Masashi, Nonoichi-shi, Ishikawa 921-8833 (JP)
(74) Representative: Brantsandpatents bvba
(86) International application number: PCT/JP2020/047581
(87) International publication number: WO 2021/125352

(57) **Abstract**

The present invention provides a method of synthesizing celosianin II, a method of synthesizing a betaxanthin, an amyloid-β polymerization inhibitor or a therapeutic or preventive agent for Alzheimer's, an amyloid peptide aggregation inhibitor, and an HIV-1 protease activity inhibitor. A gene having a celosianin II synthesis ability has been isolated from quinoa, and a method of synthesizing celosianin II of the present invention has been constructed. Besides, it has been recognized that celosianin II or the like serves as an active ingredient of each of an amyloid-β polymerization inhibitor or a therapeutic or preventive agent for Alzheimer's, an amyloid peptide aggregation inhibitor, and an HIV-1 protease activity inhibitor.

SEQUENCE LISTING

<110>  Mori, Masashi

<120>  Methods for synthesizing Celosianin II

<130>  GP20-1009PCT

<150>  JP2019-229647
<151>  2019-12-19

<150>  JP2020-119563
<151>  2020-07-11

<160>  69

<170>  PatentIn version 3.5

<210>  1
<211>  1383
<212>  DNA
<213>  Chenopodium quinoa

<400>  1
atgttgtctt tcacaaaagt cataaccttt gcaacccttt tattatgcaa ctttgcatta      60

caagtaactc cattatcatc tgaagatgat aaaatcacaa gcttaccagg gcaaccccct     120

gttaatttcc agcaattttc gggttatatt actgtcgacg aagtccaaaa aagaaatctt     180

ttttactatt tagttcaagc tgaaactaac ccttcttcca aaccccttgt tctttggctc     240

aatggagggc caggttgttc atctattgga gcaggagcat tcagtgaaca tggacccttt     300

agaccaagtg gaaatggatt gattaagaat gaatttagct ggaataaaga agcaaacatg     360

ttgtatcttg aatcaccagc aggagttggg ttttcttatt ctgctaataa aacattttat     420

gatggagtta atgatgaaat gacagcaaga gataatctgg tatttcttca aaaattccta     480

gcaaaatacc cagaatacaa gaatagagat ttattcctca ctggagagag ctatggagga     540

cattatgttc cacaacttgc taacctcgtg ctaaaatcaa gcctcaagtc aaatttgagg     600

ggagttgcta taggaaatcc attgctagag ttcaacaatg acttcaattc aatcggtgaa     660

tatctttggt ctcatggact aatatctgat gcaacttatg atctttctga aaaaatctgc     720

aattattcac aaattaggag acaagaacag aaattccttt cagcaccttg caaattagtt     780

aattctcaaa ttaatagtga gattggcaag tttattgact cgtacgatgt cactctcgac     840

gtgtgtttac attcggtttc tcaacaagct catgtcctca accaaatgat ggatgaagag     900

aaaattgatg tttgtctaga tgatgaaaca acccaatact tgaataggaa agatgtacaa     960

aaggctatgc atgcacacct tattggggta actcaatggg ctacttgtag caatgtgcta    1020

aactatcaca tggacgattt ggagattcct actcttccac tacttggcac tttcactaaa    1080

agcggcgtga gagttctggt ttacagtggt gaccaagatt caataatacc gttcattgga    1140

actagaacgc ttgtaaacgg attggcaaag gagttgggac taaacactac tgtgccttat    1200

cgaacttggc taacaaatag acaggttggt ggttggacgc aagtatatgg tgacattctt 1260

gcatatgcaa ctataagagg agcttcccat gaagcaccat tttcgcaacc tgctcgatct 1320

ctcgtgctct tccgctcctt cctggatggg aagccacttc cagatgtctc acttgctcaa 1380

taa 1383

<210> 2
<211> 460
<212> PRT
<213> Chenopodium quinoa

<400> 2

Met Leu Ser Phe Thr Lys Val Ile Thr Phe Ala Thr Leu Leu Leu Cys
1               5                   10                  15

Asn Phe Ala Leu Gln Val Thr Pro Leu Ser Ser Glu Asp Asp Lys Ile
            20                  25                  30

Thr Ser Leu Pro Gly Gln Pro Pro Val Asn Phe Gln Gln Phe Ser Gly
        35                  40                  45

Tyr Ile Thr Val Asp Glu Val Gln Lys Arg Asn Leu Phe Tyr Tyr Leu
    50                  55                  60

Val Gln Ala Glu Thr Asn Pro Ser Ser Lys Pro Leu Val Leu Trp Leu
65                  70                  75                  80

Asn Gly Gly Pro Gly Cys Ser Ser Ile Gly Ala Gly Ala Phe Ser Glu
                85                  90                  95

His Gly Pro Phe Arg Pro Ser Gly Asn Gly Leu Ile Lys Asn Glu Phe
            100                 105                 110

Ser Trp Asn Lys Glu Ala Asn Met Leu Tyr Leu Glu Ser Pro Ala Gly
        115                 120                 125

Val Gly Phe Ser Tyr Ser Ala Asn Lys Thr Phe Tyr Asp Gly Val Asn
    130                 135                 140

Asp Glu Met Thr Ala Arg Asp Asn Leu Val Phe Leu Gln Lys Phe Leu
145                 150                 155                 160

Ala Lys Tyr Pro Glu Tyr Lys Asn Arg Asp Leu Phe Leu Thr Gly Glu
                165                 170                 175

Ser Tyr Gly Gly His Tyr Val Pro Gln Leu Ala Asn Leu Val Leu Lys
            180                 185                 190

Ser Ser Leu Lys Ser Asn Leu Arg Gly Val Ala Ile Gly Asn Pro Leu
        195                 200                 205

Leu Glu Phe Asn Asn Asp Phe Asn Ser Ile Gly Glu Tyr Leu Trp Ser
    210                 215                 220

His Gly Leu Ile Ser Asp Ala Thr Tyr Asp Leu Ser Glu Lys Ile Cys
225                 230                 235                 240

Asn Tyr Ser Gln Ile Arg Arg Gln Glu Gln Lys Phe Leu Ser Ala Pro
                245                 250                 255

Cys Lys Leu Val Asn Ser Gln Ile Asn Ser Glu Ile Gly Lys Phe Ile
            260                 265                 270

Asp Ser Tyr Asp Val Thr Leu Asp Val Cys Leu His Ser Val Ser Gln
        275                 280                 285

Gln Ala His Val Leu Asn Gln Met Met Asp Glu Glu Lys Ile Asp Val
    290                 295                 300

Cys Leu Asp Asp Glu Thr Thr Gln Tyr Leu Asn Arg Lys Asp Val Gln
305                 310                 315                 320

Lys Ala Met His Ala His Leu Ile Gly Val Thr Gln Trp Ala Thr Cys
                325                 330                 335

Ser Asn Val Leu Asn Tyr His Met Asp Asp Leu Glu Ile Pro Thr Leu
            340                 345                 350

Pro Leu Leu Gly Thr Phe Thr Lys Ser Gly Val Arg Val Leu Val Tyr
        355                 360                 365

Ser Gly Asp Gln Asp Ser Ile Ile Pro Phe Ile Gly Thr Arg Thr Leu
    370                 375                 380

Val Asn Gly Leu Ala Lys Glu Leu Gly Leu Asn Thr Thr Val Pro Tyr
385                 390                 395                 400

Arg Thr Trp Leu Thr Asn Arg Gln Val Gly Gly Trp Thr Gln Val Tyr
                405                 410                 415

Gly Asp Ile Leu Ala Tyr Ala Thr Ile Arg Gly Ala Ser His Glu Ala
            420                 425                 430

Pro Phe Ser Gln Pro Ala Arg Ser Leu Val Leu Phe Arg Ser Phe Leu

435                 440                 445

Asp Gly Lys Pro Leu Pro Asp Val Ser Leu Ala Gln
    450                 455                 460

<210>  3
<211>  1440
<212>  DNA
<213>  Chenopodium quinoa

<400>  3
atgtctatat tacttgtttt ttttttcatt agtttctttc ttaagtgtag caaaatgtcg      60

tctttcacaa aactcataac ttttgtaacc cttttattat gcaactttgc attacaagta     120

actccattat catctcaaga tgataaaatc acaagcttac cagggcaacc ccctgttaat     180

ttccagcaat tttctggcta tattactgtc aacgaagtcg aaaaaagaaa tcttttttac     240

tatttagttc aagctgaaac taacccttct tccaaacccc ttgttctttg gctcaatgga     300

gggccaggtt gctcatctat tggagcagga gcatttagtg aacatggacc ctttagacca     360

agtggaaatg gattgattaa gaatgagttt agttggaata aagaagcaaa tatgttgtat     420

cttgaatcac cagcaggagt tgggttttct tattctgcta ataaatcatt ttataatgga     480

gttaatgatg aaatgacagc aagagataat ctggtatttc ttcaaaaatt cctagcaaaa     540

tacccagaat acaagaatag agatttgttc ctcactggag agagctatgg aggacattat     600

gttccacaac ttgctaacct cattctaaaa tcaagcctca agtcaaattt gaagggagtt     660

gctataggaa atccactggt ggagtttaac actgatttca attcaaggac agaatatatt     720

tggtctcatg gacttatttc agatgcaaca tatgagcttt ctgaaaagat atgcaattat     780

tcacaaatta ggagacaagc acagaaagga tttctctcag caccttgcaa attagttatt     840

tcacaaatta atagtgagat tggcaagttt attgactcgt acgatgtcac tctcgacgtt     900

tgtttacatt cggtttctca acaagctcat gtactacgcc aaatgatgga tgaagagaaa     960

attgatgttt gtctagatga tgaaacaacc caatacttga atagaaaaga tgtacaaaag    1020

gctatgcatg caaaccttat tggggtaact caatgggcta cttgtagcaa tgtgctcaac    1080

tatgacatgg acgatttgga ggttcctact cttccactac ttggtacatt cactaaaagt    1140

ggcgtaagag ttttggttta cagtggtgat caagattcag taataccatt cattggaact    1200

agaacgcttg taaacggatt ggcaaaggag ttgggactaa acactacggt tccttataga    1260

acttggctag cgaatagaca ggttggtgga tggacgcaag tgtatggtga cattcttgca    1320

tatgcaactg taagaggagc tgcccacgaa gtaccatttt cgcaacccgc tcgatctctc    1380

gtgctcttcc gctccttcct ggacgggaag ccactaccag atgtctcact tgctcaataa    1440

<210>  4

<211>  479
<212>  PRT
<213>  Chenopodium quinoa

<400>  4

Met Ser Ile Leu Leu Val Phe Phe Phe Ile Ser Phe Phe Leu Lys Cys
1               5                   10                  15

Ser Lys Met Ser Ser Phe Thr Lys Leu Ile Thr Phe Val Thr Leu Leu
            20                  25                  30

Leu Cys Asn Phe Ala Leu Gln Val Thr Pro Leu Ser Ser Gln Asp Asp
        35                  40                  45

Lys Ile Thr Ser Leu Pro Gly Gln Pro Pro Val Asn Phe Gln Gln Phe
    50                  55                  60

Ser Gly Tyr Ile Thr Val Asn Glu Val Glu Lys Arg Asn Leu Phe Tyr
65                  70                  75                  80

Tyr Leu Val Gln Ala Glu Thr Asn Pro Ser Ser Lys Pro Leu Val Leu
                85                  90                  95

Trp Leu Asn Gly Gly Pro Gly Cys Ser Ser Ile Gly Ala Gly Ala Phe
            100                 105                 110

Ser Glu His Gly Pro Phe Arg Pro Ser Gly Asn Gly Leu Ile Lys Asn
        115                 120                 125

Glu Phe Ser Trp Asn Lys Glu Ala Asn Met Leu Tyr Leu Glu Ser Pro
    130                 135                 140

Ala Gly Val Gly Phe Ser Tyr Ser Ala Asn Lys Ser Phe Tyr Asn Gly
145                 150                 155                 160

Val Asn Asp Glu Met Thr Ala Arg Asp Asn Leu Val Phe Leu Gln Lys
                165                 170                 175

Phe Leu Ala Lys Tyr Pro Glu Tyr Lys Asn Arg Asp Leu Phe Leu Thr
            180                 185                 190

Gly Glu Ser Tyr Gly Gly His Tyr Val Pro Gln Leu Ala Asn Leu Ile
        195                 200                 205

Leu Lys Ser Ser Leu Lys Ser Asn Leu Lys Gly Val Ala Ile Gly Asn
    210                 215                 220

Pro Leu Val Glu Phe Asn Thr Asp Phe Asn Ser Arg Thr Glu Tyr Ile

225                 230                 235                 240

Trp Ser His Gly Leu Ile Ser Asp Ala Thr Tyr Glu Leu Ser Glu Lys
                245                 250                 255

Ile Cys Asn Tyr Ser Gln Ile Arg Arg Gln Ala Gln Lys Gly Phe Leu
            260                 265                 270

Ser Ala Pro Cys Lys Leu Val Ile Ser Gln Ile Asn Ser Glu Ile Gly
        275                 280                 285

Lys Phe Ile Asp Ser Tyr Asp Val Thr Leu Asp Val Cys Leu His Ser
    290                 295                 300

Val Ser Gln Gln Ala His Val Leu Arg Gln Met Met Asp Glu Glu Lys
305                 310                 315                 320

Ile Asp Val Cys Leu Asp Asp Glu Thr Thr Gln Tyr Leu Asn Arg Lys
                325                 330                 335

Asp Val Gln Lys Ala Met His Ala Asn Leu Ile Gly Val Thr Gln Trp
            340                 345                 350

Ala Thr Cys Ser Asn Val Leu Asn Tyr Asp Met Asp Asp Leu Glu Val
        355                 360                 365

Pro Thr Leu Pro Leu Leu Gly Thr Phe Thr Lys Ser Gly Val Arg Val
    370                 375                 380

Leu Val Tyr Ser Gly Asp Gln Asp Ser Val Ile Pro Phe Ile Gly Thr
385                 390                 395                 400

Arg Thr Leu Val Asn Gly Leu Ala Lys Glu Leu Gly Leu Asn Thr Thr
                405                 410                 415

Val Pro Tyr Arg Thr Trp Leu Ala Asn Arg Gln Val Gly Gly Trp Thr
            420                 425                 430

Gln Val Tyr Gly Asp Ile Leu Ala Tyr Ala Thr Val Arg Gly Ala Ala
        435                 440                 445

His Glu Val Pro Phe Ser Gln Pro Ala Arg Ser Leu Val Leu Phe Arg
    450                 455                 460

Ser Phe Leu Asp Gly Lys Pro Leu Pro Asp Val Ser Leu Ala Gln
465                 470                 475

<210>  5
<211>  1362
<212>  DNA
<213>  Chenopodium quinoa

<400>  5
atgaaaaata ccaaatttgg ttgttttttc ttggtttttc tgggtatttt ttctgctaca      60

tttgcaaatc aagagaaaga tagaataaca tggttaccag gccaaccaga aaatgtgaat     120

tttgctcaat attctggtta tgtaacagta gataaatcag ctggtaaagc attgttttat     180

tggttaactg aatcaccttc aaatccagag tcaaaaccat tgttattatg gcttaatggc     240

ggtcctggtt gttcatcagt tgcttatggt gctgcagaag agattggtcc atttcatatt     300

aaaaatgatg gaaaaagtct ttacttgaat aaatattcat ggaataagga ggcaaatttg     360

ctatttcttg agtctcctgt tggtgttggg ttctcatact caaatagatc atctgatttg     420

tatgctcttg gtgatagcag gacagctaaa gatgcctata aatttcttgt taaatggttc     480

aataggtttc cccagtacaa gcacagagaa ttctacattg ctggggaaag ttatgcaggg     540

cattatgttc ctcaattgtc tcaacttatt tacagaagaa acaaaggaat tgcacaacca     600

gaaataaact tcaagggatt tatggtggga aatgcagtta cagatgatta ttatgattac     660

attggtacat ttgagtactg gtggacacat ggtttaattt cagattccac ctacaagagc     720

cttaaaacag cttgtttggc tggatcatct cagcatccta gtattgtatg caacagagct     780

ctggaaatgg ctgattctga gttcggaaac atcgatccat acagtatcta tacccgtcct     840

tgcaactcga ctgcctcgat gaagcgcagt ctaaggggcc gttatccttg gatgacgaga     900

gcatacgatc catgcaccga gaggtactcg aaccagtact tcaatcttcc tgaagttcaa     960

ctcgctatgc atgctaatgt gaccaaattg tcttatccat ggagcacttg cagtgacata    1020

gttggaactt actggtctga ctctccacaa tcgatgcttc caatttatcg agaactcata    1080

gcaggaggct tgaaaatatg ggtattcagc ggcgacactg atgctgtagt gcctattaca    1140

gcaactcgat attctattga tgcactgaga ctccctactg tgaacaattg gtccccttgg    1200

tatgacaatg gaagggttgg tggatggagc caagaataca agggtctaac atatgtgaca    1260

ataacaggag caggacatga agtacctctt catagacctc gacaagcttt tatacttttc    1320

aatcatttct taaaggacag atctatgcca agccgaagct aa                       1362


<210>  6
<211>  453
<212>  PRT
<213>  Chenopodium quinoa

<400>  6

Met Lys Asn Thr Lys Phe Gly Cys Phe Phe Leu Val Phe Leu Gly Ile
1               5                   10                  15

Phe Ser Ala Thr Phe Ala Asn Gln Glu Lys Asp Arg Ile Thr Trp Leu
            20                  25                  30

Pro Gly Gln Pro Glu Asn Val Asn Phe Ala Gln Tyr Ser Gly Tyr Val
        35                  40                  45

Thr Val Asp Lys Ser Ala Gly Lys Ala Leu Phe Tyr Trp Leu Thr Glu
    50                  55                  60

Ser Pro Ser Asn Pro Glu Ser Lys Pro Leu Leu Leu Trp Leu Asn Gly
65                  70                  75                  80

Gly Pro Gly Cys Ser Ser Val Ala Tyr Gly Ala Ala Glu Glu Ile Gly
                85                  90                  95

Pro Phe His Ile Lys Asn Asp Gly Lys Ser Leu Tyr Leu Asn Lys Tyr
            100                 105                 110

Ser Trp Asn Lys Glu Ala Asn Leu Leu Phe Leu Glu Ser Pro Val Gly
        115                 120                 125

Val Gly Phe Ser Tyr Ser Asn Arg Ser Ser Asp Leu Tyr Ala Leu Gly
    130                 135                 140

Asp Ser Arg Thr Ala Lys Asp Ala Tyr Lys Phe Leu Val Lys Trp Phe
145                 150                 155                 160

Asn Arg Phe Pro Gln Tyr Lys His Arg Glu Phe Tyr Ile Ala Gly Glu
                165                 170                 175

Ser Tyr Ala Gly His Tyr Val Pro Gln Leu Ser Gln Leu Ile Tyr Arg
            180                 185                 190

Arg Asn Lys Gly Ile Ala Gln Pro Glu Ile Asn Phe Lys Gly Phe Met
        195                 200                 205

Val Gly Asn Ala Val Thr Asp Asp Tyr Tyr Asp Tyr Ile Gly Thr Phe
    210                 215                 220

Glu Tyr Trp Trp Thr His Gly Leu Ile Ser Asp Ser Thr Tyr Lys Ser
225                 230                 235                 240

Leu Lys Thr Ala Cys Leu Ala Gly Ser Ser Gln His Pro Ser Ile Val
                245                 250                 255

Cys Asn Arg Ala Leu Glu Met Ala Asp Ser Glu Phe Gly Asn Ile Asp
            260                 265                 270

Pro Tyr Ser Ile Tyr Thr Arg Pro Cys Asn Ser Thr Ala Ser Met Lys
        275                 280                 285

Arg Ser Leu Arg Gly Arg Tyr Pro Trp Met Thr Arg Ala Tyr Asp Pro
    290                 295                 300

Cys Thr Glu Arg Tyr Ser Asn Gln Tyr Phe Asn Leu Pro Glu Val Gln
305                 310                 315                 320

Leu Ala Met His Ala Asn Val Thr Lys Leu Ser Tyr Pro Trp Ser Thr
                325                 330                 335

Cys Ser Asp Ile Val Gly Thr Tyr Trp Ser Asp Ser Pro Gln Ser Met
            340                 345                 350

Leu Pro Ile Tyr Arg Glu Leu Ile Ala Gly Gly Leu Lys Ile Trp Val
        355                 360                 365

Phe Ser Gly Asp Thr Asp Ala Val Val Pro Ile Thr Ala Thr Arg Tyr
    370                 375                 380

Ser Ile Asp Ala Leu Arg Leu Pro Thr Val Asn Asn Trp Ser Pro Trp
385                 390                 395                 400

Tyr Asp Asn Gly Arg Val Gly Gly Trp Ser Gln Glu Tyr Lys Gly Leu
                405                 410                 415

Thr Tyr Val Thr Ile Thr Gly Ala Gly His Glu Val Pro Leu His Arg
            420                 425                 430

Pro Arg Gln Ala Phe Ile Leu Phe Asn His Phe Leu Lys Asp Arg Ser
        435                 440                 445

Met Pro Ser Arg Ser
    450

<210>  7
<211>  1428
<212>  DNA
<213>  Chenopodium quinoa

<220>
<221>  misc_feature
<222>  (196)..(196)
<223>  n is a, c, g, or t

<400>  7
atgatgagca gcataactaa aattctttct cttctcatct ttattgtatt ctatattaca      60

ttcattaaag catctgaaac attcgctgtg tcagaagaag atgacagcaa gatactaaat 120

ttaccaggtc aaccatcaaa cccaccaata tcacagtact caggttacat cactgtcaac 180

caaaataatg gcaggnccct attttactgg ttctttgaag ctctttcaca gccttccgat 240

aaacctctca tactctggct gaatggagga cctggttgct catcagtagg atatggcgct 300

gttgtggaat taggtcctct taaagttgat gaaactggtg ttggtttgct cttcaataac 360

ttttcatgga taacagaagc aaatttgctc ttcgtggagt cccctgttgg agtagggttc 420

tcttatacca acacatcctc tgacctcacc aaattggatg atgaatttgt agctgaggat 480

gcatacagtt tcttagtaaa ttggtttggg agatacccac agttcaagaa ccaagacttc 540

ttcctagccg gagaaagtta tgcaggtcac tatgttcctc aactggcaga gcttattttt 600

gacaggaaca aggatcagac tatgaacctg aagattaatc tcaaaggttt cctagtaggg 660

aaccctgaaa ctgatgattt ttatgactca aagggtctgt tagaatatgc ttggagtcac 720

actgtaataa gtgatgatat gtataataag actaaagaag tgtgtgattt caagacctta 780

tattggccga aggaatgcgt gactgccatg aatttggttt atggaaaata caaagaaatc 840

gacttttaca acatatatgc tccaatttgt ctttcaaaca gcacttcatc agaagcttat 900

tatactaata aggaggaaaa ttatggattg aggaggccaa gaatatattc aggggggtat 960

gacccttgct attcaaacta cgcagaaacg tacttcaaca gggcagatgt acaaacatcg 1020

cttcgtgcaa atgctaacag agggacttca aatactagtg tcaagtggaa tgtttgcaac 1080

gacaacattc tgaatacata caacgctaca gttcaatctg tcctatcaat ctacaagaaa 1140

ctgatgcaag gtggtcttaa gatctgggtt tacagcggag atgtcgatgg aagagtcccg 1200

gtgattggaa ctaggtactg ggttgagtca cttgggttgc caatcaagac accttggcgt 1260

tcctggtacc atcaacatca ggtaggagga agattagtgg agtacgaagg tttaactttg 1320

gtgactgttc gaggggcagg tcatttggta cctctgaata agcctagtga agcacttgct 1380

ctgattcact cttttttaac aggagaagat cttccagcaa atagatga 1428

<210> 8
<211> 475
<212> PRT
<213> Chenopodium quinoa

<220>
<221> misc_feature
<222> (66)..(66)
<223> Xaa can be any naturally occurring amino acid

<400> 8

Met Met Ser Ser Ile Thr Lys Ile Leu Ser Leu Leu Ile Phe Ile Val
1               5                   10                  15

Phe Tyr Ile Thr Phe Ile Lys Ala Ser Glu Thr Phe Ala Val Ser Glu
            20                  25                  30

Glu Asp Asp Ser Lys Ile Leu Asn Leu Pro Gly Gln Pro Ser Asn Pro
        35                  40                  45

Pro Ile Ser Gln Tyr Ser Gly Tyr Ile Thr Val Asn Gln Asn Asn Gly
    50                  55                  60

Arg Xaa Leu Phe Tyr Trp Phe Phe Glu Ala Leu Ser Gln Pro Ser Asp
65                  70                  75                  80

Lys Pro Leu Ile Leu Trp Leu Asn Gly Gly Pro Gly Cys Ser Ser Val
                85                  90                  95

Gly Tyr Gly Ala Val Val Glu Leu Gly Pro Leu Lys Val Asp Glu Thr
            100                 105                 110

Gly Val Gly Leu Leu Phe Asn Asn Phe Ser Trp Ile Thr Glu Ala Asn
        115                 120                 125

Leu Leu Phe Val Glu Ser Pro Val Gly Val Gly Phe Ser Tyr Thr Asn
    130                 135                 140

Thr Ser Ser Asp Leu Thr Lys Leu Asp Asp Glu Phe Val Ala Glu Asp
145                 150                 155                 160

Ala Tyr Ser Phe Leu Val Asn Trp Phe Gly Arg Tyr Pro Gln Phe Lys
                165                 170                 175

Asn Gln Asp Phe Phe Leu Ala Gly Glu Ser Tyr Ala Gly His Tyr Val
            180                 185                 190

Pro Gln Leu Ala Glu Leu Ile Phe Asp Arg Asn Lys Asp Gln Thr Met
        195                 200                 205

Asn Leu Lys Ile Asn Leu Lys Gly Phe Leu Val Gly Asn Pro Glu Thr
    210                 215                 220

Asp Asp Phe Tyr Asp Ser Lys Gly Leu Leu Glu Tyr Ala Trp Ser His
225                 230                 235                 240

Thr Val Ile Ser Asp Asp Met Tyr Asn Lys Thr Lys Glu Val Cys Asp
                245                 250                 255

Phe Lys Thr Leu Tyr Trp Pro Lys Glu Cys Val Thr Ala Met Asn Leu

260                 265                 270

Val Tyr Gly Lys Tyr Lys Glu Ile Asp Phe Tyr Asn Ile Tyr Ala Pro
        275                 280                 285

Ile Cys Leu Ser Asn Ser Thr Ser Ser Glu Ala Tyr Tyr Thr Asn Lys
    290                 295                 300

Glu Glu Asn Tyr Gly Leu Arg Arg Pro Arg Ile Tyr Ser Gly Gly Tyr
305                 310                 315                 320

Asp Pro Cys Tyr Ser Asn Tyr Ala Glu Thr Tyr Phe Asn Arg Ala Asp
                325                 330                 335

Val Gln Thr Ser Leu Arg Ala Asn Ala Asn Arg Gly Thr Ser Asn Thr
            340                 345                 350

Ser Val Lys Trp Asn Val Cys Asn Asp Asn Ile Leu Asn Thr Tyr Asn
        355                 360                 365

Ala Thr Val Gln Ser Val Leu Ser Ile Tyr Lys Lys Leu Met Gln Gly
    370                 375                 380

Gly Leu Lys Ile Trp Val Tyr Ser Gly Asp Val Asp Gly Arg Val Pro
385                 390                 395                 400

Val Ile Gly Thr Arg Tyr Trp Val Glu Ser Leu Gly Leu Pro Ile Lys
                405                 410                 415

Thr Pro Trp Arg Ser Trp Tyr His Gln His Gln Val Gly Gly Arg Leu
            420                 425                 430

Val Glu Tyr Glu Gly Leu Thr Leu Val Thr Val Arg Gly Ala Gly His
        435                 440                 445

Leu Val Pro Leu Asn Lys Pro Ser Glu Ala Leu Ala Leu Ile His Ser
    450                 455                 460

Phe Leu Thr Gly Glu Asp Leu Pro Ala Asn Arg
465                 470                 475

<210>  9
<211>  1428
<212>  DNA
<213>  Chenopodium quinoa

<400>  9
atgatgagca gcataactaa aattctttct cttctcatct ttattgtatt ctatattaca        60

ttcattaaag catctgaaac attcgctgtg tcagaagaag atgacagcaa gatactaaat 120

ttaccaggtc aaccatcaaa cccaccaata tcacagtact caggttacat cactgtcaac 180

caaaataatg gcagggccct attttactgg ttctttgaag ctctttcaca gccttccgat 240

aaacctctca tactctggct gaatggagga cctggttgct catcagtagg atatggcgct 300

gttgtggaat taggtcctct taaagttgat gaaactggtg ttggtttgct cttcaataac 360

ttttcatgga taacagaagc aaatttgctc ttcgtggagt cccctgttgg agtagggttc 420

tcttatacca acacatcctc tgacctcacc aaattggatg atgaatttgt agctgaggat 480

gcatacagtt tcttagtaaa ttggtttggg agatacccac agttcaagaa ccaagacttc 540

ttcctagccg gagaaagtta tgcaggtcac tatgttcctc aactggcaga gcttattttt 600

gacaggaaca aggatcagac tatgaacctg aagattaatc tcaaaggttt cctagtaggg 660

aaccctgaaa ctgatgattt ttatgactca aagggtctgt tagaatatgc ttggagtcac 720

actgtaataa gtgatgatat gtataataag actaaagaag tgtgtgattt caagacctta 780

tattggccga aggaatgcgt gactgccatg aatttggttt atggaaaata caaagaaatc 840

gacttttaca acatatatgc tccaatttgt ctttcaaaca gcacttcatc agaagcttat 900

tatactaata aggaggaaaa ttatggattg aggaggccaa gaatatattc aggggggtat 960

gacccttgct attcaaacta cgcagaaacg tacttcaaca gggcagatgt acaaacatcg 1020

cttcgtgcaa atgctaacag agggacttca aatactagtg tcaagtggaa tgtttgcaac 1080

gacaacattc tgaatacata caacgctaca gttcaatctg tcctatcaat ctacaagaaa 1140

ctgatgcaag gtggtcttaa gatctgggtt tacagcggag atgtcgatgg aagagtcccg 1200

gtgattggaa ctaggtactg ggttgagtca cttgggttgc caatcaagac accttggcgt 1260

tcctggtacc atcaacatca ggtaggagga agattagtgg agtacgaagg tttaactttg 1320

gtgactgttc gaggggcagg tcatttggta cctctgaata agcctagtga agcacttgct 1380

ctgattcact cttttttaac aggagaagat cttccagcaa atagatga 1428

<210> 10
<211> 475
<212> PRT
<213> Chenopodium quinoa

<400> 10

Met Met Ser Ser Ile Thr Lys Ile Leu Ser Leu Leu Ile Phe Ile Val
1               5                   10                  15

Phe Tyr Ile Thr Phe Ile Lys Ala Ser Glu Thr Phe Ala Val Ser Glu
            20                  25                  30

Glu Asp Asp Ser Lys Ile Leu Asn Leu Pro Gly Gln Pro Ser Asn Pro

35                  40                  45

Pro Ile Ser Gln Tyr Ser Gly Tyr Ile Thr Val Asn Gln Asn Asn Gly
    50                  55                  60

Arg Ala Leu Phe Tyr Trp Phe Phe Glu Ala Leu Ser Gln Pro Ser Asp
65                  70                  75                  80

Lys Pro Leu Ile Leu Trp Leu Asn Gly Gly Pro Gly Cys Ser Ser Val
                85                  90                  95

Gly Tyr Gly Ala Val Val Glu Leu Gly Pro Leu Lys Val Asp Glu Thr
            100                 105                 110

Gly Val Gly Leu Leu Phe Asn Asn Phe Ser Trp Ile Thr Glu Ala Asn
        115                 120                 125

Leu Leu Phe Val Glu Ser Pro Val Gly Val Gly Phe Ser Tyr Thr Asn
    130                 135                 140

Thr Ser Ser Asp Leu Thr Lys Leu Asp Asp Glu Phe Val Ala Glu Asp
145                 150                 155                 160

Ala Tyr Ser Phe Leu Val Asn Trp Phe Gly Arg Tyr Pro Gln Phe Lys
                165                 170                 175

Asn Gln Asp Phe Phe Leu Ala Gly Glu Ser Tyr Ala Gly His Tyr Val
            180                 185                 190

Pro Gln Leu Ala Glu Leu Ile Phe Asp Arg Asn Lys Asp Gln Thr Met
        195                 200                 205

Asn Leu Lys Ile Asn Leu Lys Gly Phe Leu Val Gly Asn Pro Glu Thr
    210                 215                 220

Asp Asp Phe Tyr Asp Ser Lys Gly Leu Leu Glu Tyr Ala Trp Ser His
225                 230                 235                 240

Thr Val Ile Ser Asp Asp Met Tyr Asn Lys Thr Lys Glu Val Cys Asp
                245                 250                 255

Phe Lys Thr Leu Tyr Trp Pro Lys Glu Cys Val Thr Ala Met Asn Leu
            260                 265                 270

Val Tyr Gly Lys Tyr Lys Glu Ile Asp Phe Tyr Asn Ile Tyr Ala Pro
        275                 280                 285

Ile Cys Leu Ser Asn Ser Thr Ser Ser Glu Ala Tyr Tyr Thr Asn Lys
    290                 295                 300

Glu Glu Asn Tyr Gly Leu Arg Arg Pro Arg Ile Tyr Ser Gly Gly Tyr
305                 310                 315                 320

Asp Pro Cys Tyr Ser Asn Tyr Ala Glu Thr Tyr Phe Asn Arg Ala Asp
                325                 330                 335

Val Gln Thr Ser Leu Arg Ala Asn Ala Asn Arg Gly Thr Ser Asn Thr
            340                 345                 350

Ser Val Lys Trp Asn Val Cys Asn Asp Asn Ile Leu Asn Thr Tyr Asn
        355                 360                 365

Ala Thr Val Gln Ser Val Leu Ser Ile Tyr Lys Lys Leu Met Gln Gly
    370                 375                 380

Gly Leu Lys Ile Trp Val Tyr Ser Gly Asp Val Asp Gly Arg Val Pro
385                 390                 395                 400

Val Ile Gly Thr Arg Tyr Trp Val Glu Ser Leu Gly Leu Pro Ile Lys
                405                 410                 415

Thr Pro Trp Arg Ser Trp Tyr His Gln His Gln Val Gly Gly Arg Leu
            420                 425                 430

Val Glu Tyr Glu Gly Leu Thr Leu Val Thr Val Arg Gly Ala Gly His
        435                 440                 445

Leu Val Pro Leu Asn Lys Pro Ser Glu Ala Leu Ala Leu Ile His Ser
    450                 455                 460

Phe Leu Thr Gly Glu Asp Leu Pro Ala Asn Arg
465                 470                 475

<210>  11
<211>  1512
<212>  DNA
<213>  Chenopodium quinoa

<400>  11
atggctaaac cttgtcatta tattcttaat cacatattaa taccaatttt aatggtgttt      60

tttctctgtt ttataattac tgaatctgcc cctcaaagtt ctattgttac tcaacttcct     120

ggatttaatg gcacttttcc ctccaaacac tactctgggt atgtgacaat tgatgagagt     180

cacgggaaga aattgtttta ctactttgtt gaatcagaga gaaacccatc agaggatcct     240

gttgttcttt ggcttaatgg aggacccgga tgctcgagtt tagatggctt cgtgtacgag     300

catggtcctt tcaattttga ggcagcaaag acaaaaggag gcttgccaaa gttgcatatt 360

aatccataca gctggtctaa ggtttccaat gttatatact tggattctcc agcaggagtt 420

gggtattctt atgcacaaaa cattagtgag tatgataccg gggaccttaa gaccgcctct 480

gatacccata cattcctgct caagtggttt gatatctata aagagtttgt tgcaaaccca 540

tttttcatcg cgggtgagtc ttatgctggg atttatgtgc caacccttgc ttcagatgtt 600

gtgaaaggac ttgatgccgg tgtaaagcct gttatcaatt taaagggtta tttggtagga 660

aatggtgtta cagatgaaac ctttgatgga aatgctttag tgccttttgc acatggaatg 720

ggcttgatct ctgatcaatt atatcaggag gttgtcactc agtgtgaagg aaactattac 780

aatctgaaat ctgatagttg tgatgccttg ctttcaaaag ttaaccagga tgttgatgga 840

ttgaatatat atgatattct ggagccttgt tatcactccc ctgactcaga tatgcagagt 900

actgctcatc taagactacc ttcgaaaatt tccagcttca aggcattagg agaaactgaa 960

agacctcttc ctgtgaggaa aagaattttt ggccgtgctt ggcccctcag agctcctgtg 1020

agagatggac atgttcctac ctggccggaa cttgctaaca gcgcttctgt tccttgcatt 1080

aatgatgaag ttgcaacttt gtggctgaat gacaaggcag tgagaaaagc aatccatgct 1140

gaagaggaaa gtgtgtcggg tccctgggaa ctttgcacag gcaggatttc atttagccat 1200

gatgctggaa gcatgattac ataccataag aacctcactt ctcgaggtta tcgagccctc 1260

atatacagtg gagaccatga tatgtgtgtt ccatacactg ggagcgaggc atggacaaaa 1320

tctgttgggt acaaagtagt agatgattgg aggccatggg tttgcaatga acaagtttct 1380

gggttcacac aggggtatga gaagaatctc actttcctta ctataaaggg ggcaggacat 1440

acggtaccag aatacaaacc gcgagaagca ctgtactttt atagccagtt tctggcaggg 1500

aaaagtatat aa 1512

<210> 12
<211> 503
<212> PRT
<213> Chenopodium quinoa

<400> 12

Met Ala Lys Pro Cys His Tyr Ile Leu Asn His Ile Leu Ile Pro Ile
1               5                   10                  15

Leu Met Val Phe Phe Leu Cys Phe Ile Ile Thr Glu Ser Ala Pro Gln
            20                  25                  30

Ser Ser Ile Val Thr Gln Leu Pro Gly Phe Asn Gly Thr Phe Pro Ser
        35                  40                  45

Lys His Tyr Ser Gly Tyr Val Thr Ile Asp Glu Ser His Gly Lys Lys
    50                  55                  60

Leu Phe Tyr Tyr Phe Val Glu Ser Glu Arg Asn Pro Ser Glu Asp Pro
65                  70                  75                  80

Val Val Leu Trp Leu Asn Gly Gly Pro Gly Cys Ser Ser Leu Asp Gly
                85                  90                  95

Phe Val Tyr Glu His Gly Pro Phe Asn Phe Glu Ala Ala Lys Thr Lys
            100                 105                 110

Gly Gly Leu Pro Lys Leu His Ile Asn Pro Tyr Ser Trp Ser Lys Val
        115                 120                 125

Ser Asn Val Ile Tyr Leu Asp Ser Pro Ala Gly Val Gly Tyr Ser Tyr
    130                 135                 140

Ala Gln Asn Ile Ser Glu Tyr Asp Thr Gly Asp Leu Lys Thr Ala Ser
145                 150                 155                 160

Asp Thr His Thr Phe Leu Leu Lys Trp Phe Asp Ile Tyr Lys Glu Phe
                165                 170                 175

Val Ala Asn Pro Phe Phe Ile Ala Gly Glu Ser Tyr Ala Gly Ile Tyr
            180                 185                 190

Val Pro Thr Leu Ala Ser Asp Val Val Lys Gly Leu Asp Ala Gly Val
        195                 200                 205

Lys Pro Val Ile Asn Leu Lys Gly Tyr Leu Val Gly Asn Gly Val Thr
    210                 215                 220

Asp Glu Thr Phe Asp Gly Asn Ala Leu Val Pro Phe Ala His Gly Met
225                 230                 235                 240

Gly Leu Ile Ser Asp Gln Leu Tyr Gln Glu Val Val Thr Gln Cys Glu
                245                 250                 255

Gly Asn Tyr Tyr Asn Leu Lys Ser Asp Ser Cys Asp Ala Leu Leu Ser
            260                 265                 270

Lys Val Asn Gln Asp Val Asp Gly Leu Asn Ile Tyr Asp Ile Leu Glu
        275                 280                 285

Pro Cys Tyr His Ser Pro Asp Ser Asp Met Gln Ser Thr Ala His Leu
    290                 295                 300

Arg Leu Pro Ser Lys Ile Ser Ser Phe Lys Ala Leu Gly Glu Thr Glu
305                 310                 315                 320

Arg Pro Leu Pro Val Arg Lys Arg Ile Phe Gly Arg Ala Trp Pro Leu
                325                 330                 335

Arg Ala Pro Val Arg Asp Gly His Val Pro Thr Trp Pro Glu Leu Ala
            340                 345                 350

Asn Ser Ala Ser Val Pro Cys Ile Asn Asp Glu Val Ala Thr Leu Trp
        355                 360                 365

Leu Asn Asp Lys Ala Val Arg Lys Ala Ile His Ala Glu Glu Glu Ser
    370                 375                 380

Val Ser Gly Pro Trp Glu Leu Cys Thr Gly Arg Ile Ser Phe Ser His
385                 390                 395                 400

Asp Ala Gly Ser Met Ile Thr Tyr His Lys Asn Leu Thr Ser Arg Gly
                405                 410                 415

Tyr Arg Ala Leu Ile Tyr Ser Gly Asp His Asp Met Cys Val Pro Tyr
            420                 425                 430

Thr Gly Ser Glu Ala Trp Thr Lys Ser Val Gly Tyr Lys Val Val Asp
        435                 440                 445

Asp Trp Arg Pro Trp Val Cys Asn Glu Gln Val Ser Gly Phe Thr Gln
    450                 455                 460

Gly Tyr Glu Lys Asn Leu Thr Phe Leu Thr Ile Lys Gly Ala Gly His
465                 470                 475                 480

Thr Val Pro Glu Tyr Lys Pro Arg Glu Ala Leu Tyr Phe Tyr Ser Gln
                485                 490                 495

Phe Leu Ala Gly Lys Ser Ile
            500

<210>  13
<211>  1437
<212>  DNA
<213>  Chenopodium quinoa

<400>  13
atgagtaagc tagcatcaag ggttatagct tactatgttg aagtggtagt aatattgatg      60

agtttggtat cattggcatt atccaattat cctcatcatg ccacaacaaa aacatcggaa     120

ttatattcta caattgttga ttttctgcct ggatttcccg gaaaactccc tttcactctt 180

gaaactgggt acattgaagt ttgggaagaa gtgcagctat tttactactt catacaatct 240

gagcaaaacc cagaaataga tccaattttg atttggtttg ctggaggtcc tggttgttct 300

tcctttactg atcttgcttt tgggcatatt gggcctttat ccttcaactt tacgggaatt 360

aatttcaact atgcaacatc aagttgggag gttgatctac caagacttca attaaatcct 420

tattcttgga ccaaggttgc aaacgtaata tttatagatt cacctgctgg cgctggattc 480

tcctatgcaa ctaacaaatc ttattaccct agtgatacaa agacatcaaa acaagcacat 540

gggtttttga agaagtggct tattgagcat cctaaatttg caagaaaccc tctttacatt 600

gctggtgctt catatggggg aaaagttgtt ccatctctta catgggagat tgtaaaagga 660

aatgaagctg gaattgggcc acaaatgaat cttcagggat atattcttgt aaatccaata 720

actgcttggt accttgatga ccaaaagaaa gttgaatatg ctcatcgcat ctctcttata 780

tccgatgaac tatacaagtc tgccaaatta agctgcaatg aagattatcg gacaaatgag 840

gctaataaga gtaatgcaca atgcaacaag attcttgaag atgtatcaaa ttgcattgat 900

ccactagaag accaacatgt actggatcct aaatgcaccc attcttcttc gccaaatcat 960

tggtgtcggg gaaatgatcg tatgattgct agccagtggg caaataacat taatgtccaa 1020

gaagctcttc atgttcgaga gggaactatt agccagtgga ttcgttgtcg cggtcacgca 1080

aaatctgatg tgtttatgta caataaagat atcgacagta gcatcattta tcatcagaat 1140

cttaccaccc aacctctcag ggcactaatc ttcagtggtg accaagatat gagtgttcca 1200

tatgtgacta cactaaaatg gatcaaggag cttaatatcg ccccaaatga tgattattgg 1260

aagccttggt atcttaataa tggccaagtt tcaggatatg taactgaata ttccaatggc 1320

tacttcaatt tgacatttac tacaatcaag ggagcagggc atgttgcctc cgagtataag 1380

ccgcgggaat gttttacaat gtttcaaaga tggatcacat ttagtcctct atcataa 1437

<210> 14
<211> 478
<212> PRT
<213> Chenopodium quinoa

<400> 14

Met Ser Lys Leu Ala Ser Arg Val Ile Ala Tyr Tyr Val Glu Val Val
1               5                   10                  15

Val Ile Leu Met Ser Leu Val Ser Leu Ala Leu Ser Asn Tyr Pro His
            20                  25                  30

His Ala Thr Thr Lys Thr Ser Glu Leu Tyr Ser Thr Ile Val Asp Phe
        35                  40                  45

Leu Pro Gly Phe Pro Gly Lys Leu Pro Phe Thr Leu Glu Thr Gly Tyr
    50                  55                  60

Ile Glu Val Trp Glu Glu Val Gln Leu Phe Tyr Tyr Phe Ile Gln Ser
65                  70                  75                  80

Glu Gln Asn Pro Glu Ile Asp Pro Ile Leu Ile Trp Phe Ala Gly Gly
                85                  90                  95

Pro Gly Cys Ser Ser Phe Thr Asp Leu Ala Phe Gly His Ile Gly Pro
            100                 105                 110

Leu Ser Phe Asn Phe Thr Gly Ile Asn Phe Asn Tyr Ala Thr Ser Ser
        115                 120                 125

Trp Glu Val Asp Leu Pro Arg Leu Gln Leu Asn Pro Tyr Ser Trp Thr
    130                 135                 140

Lys Val Ala Asn Val Ile Phe Ile Asp Ser Pro Ala Gly Ala Gly Phe
145                 150                 155                 160

Ser Tyr Ala Thr Asn Lys Ser Tyr Tyr Pro Ser Asp Thr Lys Thr Ser
                165                 170                 175

Lys Gln Ala His Gly Phe Leu Lys Lys Trp Leu Ile Glu His Pro Lys
            180                 185                 190

Phe Ala Arg Asn Pro Leu Tyr Ile Ala Gly Ala Ser Tyr Gly Gly Lys
        195                 200                 205

Val Val Pro Ser Leu Thr Trp Glu Ile Val Lys Gly Asn Glu Ala Gly
    210                 215                 220

Ile Gly Pro Gln Met Asn Leu Gln Gly Tyr Ile Leu Val Asn Pro Ile
225                 230                 235                 240

Thr Ala Trp Tyr Leu Asp Asp Gln Lys Lys Val Glu Tyr Ala His Arg
                245                 250                 255

Ile Ser Leu Ile Ser Asp Glu Leu Tyr Lys Ser Ala Lys Leu Ser Cys
            260                 265                 270

Asn Glu Asp Tyr Arg Thr Asn Glu Ala Asn Lys Ser Asn Ala Gln Cys
        275                 280                 285

Asn Lys Ile Leu Glu Asp Val Ser Asn Cys Ile Asp Pro Leu Glu Asp
    290                 295                 300

Gln His Val Leu Asp Pro Lys Cys Thr His Ser Ser Ser Pro Asn His
305                 310                 315                 320

Trp Cys Arg Gly Asn Asp Arg Met Ile Ala Ser Gln Trp Ala Asn Asn
                325                 330                 335

Ile Asn Val Gln Glu Ala Leu His Val Arg Glu Gly Thr Ile Ser Gln
            340                 345                 350

Trp Ile Arg Cys Arg Gly His Ala Lys Ser Asp Val Phe Met Tyr Asn
        355                 360                 365

Lys Asp Ile Asp Ser Ser Ile Ile Tyr His Gln Asn Leu Thr Thr Gln
    370                 375                 380

Pro Leu Arg Ala Leu Ile Phe Ser Gly Asp Gln Asp Met Ser Val Pro
385                 390                 395                 400

Tyr Val Thr Thr Leu Lys Trp Ile Lys Glu Leu Asn Ile Ala Pro Asn
                405                 410                 415

Asp Asp Tyr Trp Lys Pro Trp Tyr Leu Asn Asn Gly Gln Val Ser Gly
            420                 425                 430

Tyr Val Thr Glu Tyr Ser Asn Gly Tyr Phe Asn Leu Thr Phe Thr Thr
        435                 440                 445

Ile Lys Gly Ala Gly His Val Ala Ser Glu Tyr Lys Pro Arg Glu Cys
    450                 455                 460

Phe Thr Met Phe Gln Arg Trp Ile Thr Phe Ser Pro Leu Ser
465                 470                 475

<210>  15
<211>  1419
<212>  DNA
<213>  Chenopodium quinoa

<400>  15
atgattagca gggcttgcat ccattggtgg ctgatgctgg taatttttgt tgggattggt      60

tgtttgaatg ggtatcctct cgaggatatt gtggtaggat tgcctggcca accaaaagtt     120

ggattcaaac agtttgctgg ctacattgat ttggatgtta agactgggaa gagcttgttt     180

tactactttg ttgaagctga gaaagatcca gaccataagc ctctcactct ttggctcaat     240

ggaggccctg gttgctcgtc acttggtggt ggtgccttta cggaattggg accattcttt     300

cctacaggtg atggtagggg tcttcgaaaa aatccaaaat cttggaataa agcatcaaat     360

ctcctatttg tggagtctcc tgctggagta ggctggtcat attcaaatac aacggctgat 420

tacaatgccg ggataccctc aacagcaaaa gacatgcaac tttttatgat gaagtggtat 480

gagaaatttc cagctctgaa atcgagggat ttctttctta ctggagaaag ctatgcaggg 540

cattacatac cacaattagc tgttgcttta ctggatcata atgcccattc tactcgtttt 600

aagttcaaca taaaaggagt tgctattgga aatccattgc tcagacttga tcgagatact 660

cgggcaacat atgagttcct atggtcacat gggatgattt ctgatgaaat aggcctcacc 720

ataataaagg aatgtgattt cagtgattat agttttggta gtcctgatca taatgtttct 780

ggtaaatgct atgatgcaat aacagaagct cagctcagcg ttagtagcta cgttgataac 840

tataatgtca ttgtagatgt ttgttaccca tcgatagtgg agcaagaatt tcggttgaga 900

aaagcggcca ctaaaataag cttgggtatt gatatttgca ttaattacga atctggattc 960

tatttcaatc ttccagaggt ccaaagcgct ctccatgcaa atcgaacaaa attgccctac 1020

agctggtcca tgtgtagcga agttttgaac tataccaaga ctgacgtcaa gatgaatatg 1080

cttcccttgc ttaaaggaat catccaaaaa ggtgttccgg tttgggttta cagcggagat 1140

caagattctg tagttccact gctgggatcc cgccagatga ttcatgaact tgctcgtgaa 1200

ctaaacttta cagtcactgc tccttatgga gcttggtttc acaaagggca ggtaggaggt 1260

tggtcaactg agtatggaaa tttgttgacc tttgccacag taaggggtgc tgcacatatg 1320

gtgccctatt cacagccagc cagggcttta cacctcttta gttcttttgt tcgtggtcta 1380

agattgccta attcaacacg cctgcccata gatgactag 1419

<210> 16
<211> 472
<212> PRT
<213> Chenopodium quinoa

<400> 16

Met Ile Ser Arg Ala Cys Ile His Trp Trp Leu Met Leu Val Ile Phe
1               5                   10                  15

Val Gly Ile Gly Cys Leu Asn Gly Tyr Pro Leu Glu Asp Ile Val Val
            20                  25                  30

Gly Leu Pro Gly Gln Pro Lys Val Gly Phe Lys Gln Phe Ala Gly Tyr
        35                  40                  45

Ile Asp Leu Asp Val Lys Thr Gly Lys Ser Leu Phe Tyr Tyr Phe Val
    50                  55                  60

Glu Ala Glu Lys Asp Pro Asp His Lys Pro Leu Thr Leu Trp Leu Asn
65                  70                  75                  80

Gly Gly Pro Gly Cys Ser Ser Leu Gly Gly Gly Ala Phe Thr Glu Leu
                85                  90                  95

Gly Pro Phe Phe Pro Thr Gly Asp Gly Arg Gly Leu Arg Lys Asn Pro
            100                 105                 110

Lys Ser Trp Asn Lys Ala Ser Asn Leu Leu Phe Val Glu Ser Pro Ala
        115                 120                 125

Gly Val Gly Trp Ser Tyr Ser Asn Thr Thr Ala Asp Tyr Asn Ala Gly
    130                 135                 140

Asp Thr Ser Thr Ala Lys Asp Met Gln Leu Phe Met Met Lys Trp Tyr
145                 150                 155                 160

Glu Lys Phe Pro Ala Leu Lys Ser Arg Asp Phe Phe Leu Thr Gly Glu
                165                 170                 175

Ser Tyr Ala Gly His Tyr Ile Pro Gln Leu Ala Val Ala Leu Leu Asp
            180                 185                 190

His Asn Ala His Ser Thr Arg Phe Lys Phe Asn Ile Lys Gly Val Ala
        195                 200                 205

Ile Gly Asn Pro Leu Leu Arg Leu Asp Arg Asp Thr Arg Ala Thr Tyr
    210                 215                 220

Glu Phe Leu Trp Ser His Gly Met Ile Ser Asp Glu Ile Gly Leu Thr
225                 230                 235                 240

Ile Ile Lys Glu Cys Asp Phe Ser Asp Tyr Ser Phe Gly Ser Pro Asp
                245                 250                 255

His Asn Val Ser Gly Lys Cys Tyr Asp Ala Ile Thr Glu Ala Gln Leu
            260                 265                 270

Ser Val Ser Ser Tyr Val Asp Asn Tyr Asn Val Ile Val Asp Val Cys
        275                 280                 285

Tyr Pro Ser Ile Val Glu Gln Glu Phe Arg Leu Arg Lys Ala Ala Thr
    290                 295                 300

Lys Ile Ser Leu Gly Ile Asp Ile Cys Ile Asn Tyr Glu Ser Gly Phe
305                 310                 315                 320

Tyr Phe Asn Leu Pro Glu Val Gln Ser Ala Leu His Ala Asn Arg Thr

325                 330                 335

Lys Leu Pro Tyr Ser Trp Ser Met Cys Ser Glu Val Leu Asn Tyr Thr
            340                 345                 350

Lys Thr Asp Val Lys Met Asn Met Leu Pro Leu Leu Lys Gly Ile Ile
        355                 360                 365

Gln Lys Gly Val Pro Val Trp Val Tyr Ser Gly Asp Gln Asp Ser Val
    370                 375                 380

Val Pro Leu Leu Gly Ser Arg Gln Met Ile His Glu Leu Ala Arg Glu
385                 390                 395                 400

Leu Asn Phe Thr Val Thr Ala Pro Tyr Gly Ala Trp Phe His Lys Gly
                405                 410                 415

Gln Val Gly Gly Trp Ser Thr Glu Tyr Gly Asn Leu Leu Thr Phe Ala
            420                 425                 430

Thr Val Arg Gly Ala Ala His Met Val Pro Tyr Ser Gln Pro Ala Arg
        435                 440                 445

Ala Leu His Leu Phe Ser Ser Phe Val Arg Gly Leu Arg Leu Pro Asn
    450                 455                 460

Ser Thr Arg Leu Pro Ile Asp Asp
465                 470

<210>  17
<211>  1494
<212>  DNA
<213>  Chenopodium quinoa

<400>  17
atggatcatg caacactagc aatgatactt gctatttggt ttgttgtttt tcatttcatt      60

aaaatgttat tcactagcca aactaccaaa cttcttcccc ccggtcctaa accattgcca     120

ctaatcggaa acatccttga agttggtgag aaacctcacc agtcatttgc taatcttgct     180

aagattcatg gtccattgat atcattacgt ctaggaagtg tcacaactat tgttgtatcc     240

tcggctgaag tagccaaaga aatgttctta aaaaaagacc acccactttc taaccgcact     300

gttcctaatt ctgtcactgc tggggatcac cataagctga ctatgtcgtg gttgcctgtt     360

tctccaaagt ggcggaactt tagaaaaatc accgccgttc atttactttc tcctcaaaga     420

cttgatgctt gccaaaccct aagacatgcc aaagtgcaac agcttttcca atatgtacaa     480

gaatgtgcac aaaaaggaca agctgttgat attggcaagg cagcatttac aacctcccct     540

aatttgttat caaaattatt cttttcggtc gaattagccc accataaatc ccatacatct 600

caacaattca aagaacttat atggaatatt atggaagata ttggcaaacc taactacgct 660

gattattttc caatcttagg atgcttagac ccatcaggaa ttcgacgtcg attggcgtct 720

aattttgaca agttgattgc agtttttcaa agcataatat gtcaaaggat tggcaacggc 780

caggattctg cttcgacgaa gacgaccgat gatgtgctag acattcttct tgacctccac 840

aaacaaaaag agctcagtat gggcgagata aatcatctgc tcgtggatat ttttgatgct 900

gggactgaca ctacatcaag tacttttgaa tgggtaatgg cagagttaat tcgaaatcct 960

aagatgatgg aaaaagctca agaagaaatt gagcaagtct tgggcaagga tagacaaatt 1020

caagaatcag acattattaa gctaccttac ttacaagcca ttatcaaaga aacattgcgg 1080

ctacacccac caactgtatt tctcttgcct cgtaaagctg atagtgatgt tgaattatat 1140

ggctatgttg tgccgaaaga tgcacaaata cttgttaatt tatgggccat tggaagagac 1200

cctcaagcgt gggtgaaacc tgacgtgttt ttacctgaga ggtttttagg atccgaaatt 1260

gatgtgaaag gaagagattt tggactctta ccttttggag ctggaaggag aatatgcccc 1320

gggatgaatt tggctattag aatgttaact ttgatgttag ctacgcttct tcaattcttc 1380

aattggaagc ttgaagaagg tatgaaagca gaagatctag acatggatga gaaatttggg 1440

attgccttac aaaagactaa acctcttcag attattcccg ttcttaggta ttga 1494

<210> 18
<211> 497
<212> PRT
<213> Chenopodium quinoa

<400> 18

Met Asp His Ala Thr Leu Ala Met Ile Leu Ala Ile Trp Phe Val Val
1               5                   10                  15

Phe His Phe Ile Lys Met Leu Phe Thr Ser Gln Thr Thr Lys Leu Leu
            20                  25                  30

Pro Pro Gly Pro Lys Pro Leu Pro Leu Ile Gly Asn Ile Leu Glu Val
        35                  40                  45

Gly Glu Lys Pro His Gln Ser Phe Ala Asn Leu Ala Lys Ile His Gly
    50                  55                  60

Pro Leu Ile Ser Leu Arg Leu Gly Ser Val Thr Thr Ile Val Val Ser
65                  70                  75                  80

Ser Ala Glu Val Ala Lys Glu Met Phe Leu Lys Lys Asp His Pro Leu
                85                  90                  95

Ser Asn Arg Thr Val Pro Asn Ser Val Thr Ala Gly Asp His His Lys
            100                 105                 110

Leu Thr Met Ser Trp Leu Pro Val Ser Pro Lys Trp Arg Asn Phe Arg
        115                 120                 125

Lys Ile Thr Ala Val His Leu Leu Ser Pro Gln Arg Leu Asp Ala Cys
    130                 135                 140

Gln Thr Leu Arg His Ala Lys Val Gln Gln Leu Phe Gln Tyr Val Gln
145                 150                 155                 160

Glu Cys Ala Gln Lys Gly Gln Ala Val Asp Ile Gly Lys Ala Ala Phe
                165                 170                 175

Thr Thr Ser Leu Asn Leu Leu Ser Lys Leu Phe Phe Ser Val Glu Leu
            180                 185                 190

Ala His His Lys Ser His Thr Ser Gln Gln Phe Lys Glu Leu Ile Trp
        195                 200                 205

Asn Ile Met Glu Asp Ile Gly Lys Pro Asn Tyr Ala Asp Tyr Phe Pro
    210                 215                 220

Ile Leu Gly Cys Leu Asp Pro Ser Gly Ile Arg Arg Arg Leu Ala Ser
225                 230                 235                 240

Asn Phe Asp Lys Leu Ile Ala Val Phe Gln Ser Ile Ile Cys Gln Arg
                245                 250                 255

Ile Gly Asn Gly Gln Asp Ser Ala Ser Thr Lys Thr Thr Asp Asp Val
            260                 265                 270

Leu Asp Ile Leu Leu Asp Leu His Lys Gln Lys Glu Leu Ser Met Gly
        275                 280                 285

Glu Ile Asn His Leu Leu Val Asp Ile Phe Asp Ala Gly Thr Asp Thr
    290                 295                 300

Thr Ser Ser Thr Phe Glu Trp Val Met Ala Glu Leu Ile Arg Asn Pro
305                 310                 315                 320

Lys Met Met Glu Lys Ala Gln Glu Glu Ile Glu Gln Val Leu Gly Lys
                325                 330                 335

Asp Arg Gln Ile Gln Glu Ser Asp Ile Ile Lys Leu Pro Tyr Leu Gln
            340                 345                 350

Ala Ile Ile Lys Glu Thr Leu Arg Leu His Pro Pro Thr Val Phe Leu
        355                 360                 365

Leu Pro Arg Lys Ala Asp Ser Asp Val Glu Leu Tyr Gly Tyr Val Val
    370                 375                 380

Pro Lys Asp Ala Gln Ile Leu Val Asn Leu Trp Ala Ile Gly Arg Asp
385                 390                 395                 400

Pro Gln Ala Trp Val Lys Pro Asp Val Phe Leu Pro Glu Arg Phe Leu
                405                 410                 415

Gly Ser Glu Ile Asp Val Lys Gly Arg Asp Phe Gly Leu Leu Pro Phe
            420                 425                 430

Gly Ala Gly Arg Arg Ile Cys Pro Gly Met Asn Leu Ala Ile Arg Met
        435                 440                 445

Leu Thr Leu Met Leu Ala Thr Leu Leu Gln Phe Phe Asn Trp Lys Leu
    450                 455                 460

Glu Glu Gly Met Lys Ala Glu Asp Leu Asp Met Asp Glu Lys Phe Gly
465                 470                 475                 480

Ile Ala Leu Gln Lys Thr Lys Pro Leu Gln Ile Ile Pro Val Leu Arg
                485                 490                 495

Tyr

<210>  19
<211>  1476
<212>  DNA
<213>  Chenopodium quinoa

<400>  19
atggagtcat caacaaaaga acaagaacaa caacacataa tcctcctccc atttttagca      60

caaggccatc tccgaccatt cctccacctc gctcaccgcc ttctctctct aacacccttc     120

aatctctctc tcctcactac ccctttaaac gccgctaatc tccgccgtca atccgacaac     180

ttcaacatca accttaacat catcgaactc cccttcacta gcaccgacca cggtctccca     240

cccaacaccg aaaataccga taaactctca ttaacctcca tcatcaaact cttccatgca     300

tccacttcac tcgaacctca tgtacgtgac tacctcacgc gccaccacct gcacgaccca     360

cccgtgtgca taatcttcga cgtgttcttg ggctgggccg ataacgtggc ccgatcagtt     420

gggtccaccg gaatctgctt caacactggt ggggcctacg gtgtcggtgc gtatgtttca     480

atctggagca atctacctca ccggaatgtc ggcgatgatg aggagttttc attggctggt 540

ttcccggaag atcggaaact ccgacgtaat cagcttcatc ggtttttaag gtttgctgat 600

gggtccgatg aatggtcgag gtttttccaa cctcagatta aatcatcctt gaattgttct 660

gggtggttgt gtaattccat cgaggagatt gaaccgttag ggtttcaagt ttttaggaat 720

ttcacgaaat ctcctatttg gggaattggt cctttgatta tgacatcttc gaaaaaagat 780

gatgatgaga aggaagaggc ttgtttgagg tggttgaatc aatttgaaaa tgactcagtt 840

ttgtacattt gtttcgggtc acagaataca gtgactccga ttcagatgat ggagttagcg 900

aaaggtttgg aggagagtaa gatcccgttc ttgtgggtga ttcggccgcc attcgggttc 960

gatttcaatg gggagtttaa acccgaatgg ctgcctgaga agtttgagga aagaatgatg 1020

gagaaaaaac aggggatgtt ggtgagagat tgggtgcctc agttggatat tttgaggcat 1080

gaggctactg gtggattttt gagtcattgt ggttggaatt cggtattgga aggtttaaga 1140

gaaggagtgc caatactcgg gtggccgttg gcggccgagc aagcgtataa ttcaaagatg 1200

atggtggagg aaatgggggt ggcggtcgag ttgacgaggg ggttggaagg ggaggtgaag 1260

aaagagtggg tgaagagtgt ggtggaaatg gtgttggata gaaaggaggg gagttgtggg 1320

tgggaaatga agaagaaagc tgttgagatt ggggagaaat tgaaggatgc ttggaaaatt 1380

gagggagatt acaaaggatc ttctgttaag gcaatggata attttgtaga gtttatagtt 1440

tcttgtaggg gaaagaaatc taaagaagat gcttga 1476

<210> 20
<211> 491
<212> PRT
<213> Chenopodium quinoa

<400> 20

Met Glu Ser Ser Thr Lys Glu Gln Glu Gln Gln His Ile Ile Leu Leu
1               5                   10                  15

Pro Phe Leu Ala Gln Gly His Leu Arg Pro Phe Leu His Leu Ala His
            20                  25                  30

Arg Leu Leu Ser Leu Thr Pro Phe Asn Leu Ser Leu Leu Thr Thr Pro
        35                  40                  45

Leu Asn Ala Ala Asn Leu Arg Arg Gln Ser Asp Asn Phe Asn Ile Asn
    50                  55                  60

Leu Asn Ile Ile Glu Leu Pro Phe Thr Ser Thr Asp His Gly Leu Pro
65                  70                  75                  80

Pro Asn Thr Glu Asn Thr Asp Lys Leu Ser Leu Thr Ser Ile Ile Lys
                85                  90                  95

Leu Phe His Ala Ser Thr Ser Leu Glu Pro His Val Arg Asp Tyr Leu
            100                 105                 110

Thr Arg His His Leu His Asp Pro Pro Val Cys Ile Ile Phe Asp Val
        115                 120                 125

Phe Leu Gly Trp Ala Asp Asn Val Ala Arg Ser Val Gly Ser Thr Gly
    130                 135                 140

Ile Cys Phe Asn Thr Gly Gly Ala Tyr Gly Val Gly Ala Tyr Val Ser
145                 150                 155                 160

Ile Trp Ser Asn Leu Pro His Arg Asn Val Gly Asp Asp Glu Glu Phe
                165                 170                 175

Ser Leu Ala Gly Phe Pro Glu Asp Arg Lys Leu Arg Arg Asn Gln Leu
            180                 185                 190

His Arg Phe Leu Arg Phe Ala Asp Gly Ser Asp Glu Trp Ser Arg Phe
        195                 200                 205

Phe Gln Pro Gln Ile Lys Ser Ser Leu Asn Cys Ser Gly Trp Leu Cys
    210                 215                 220

Asn Ser Ile Glu Glu Ile Glu Pro Leu Gly Phe Gln Val Phe Arg Asn
225                 230                 235                 240

Phe Thr Lys Ser Pro Ile Trp Gly Ile Gly Pro Leu Ile Met Thr Ser
                245                 250                 255

Ser Lys Lys Asp Asp Asp Glu Lys Glu Glu Ala Cys Leu Arg Trp Leu
            260                 265                 270

Asn Gln Phe Glu Asn Asp Ser Val Leu Tyr Ile Cys Phe Gly Ser Gln
        275                 280                 285

Asn Thr Val Thr Pro Ile Gln Met Met Glu Leu Ala Lys Gly Leu Glu
    290                 295                 300

Glu Ser Lys Ile Pro Phe Leu Trp Val Ile Arg Pro Pro Phe Gly Phe
305                 310                 315                 320

Asp Phe Asn Gly Glu Phe Lys Pro Glu Trp Leu Pro Glu Lys Phe Glu
                325                 330                 335

Glu Arg Met Met Glu Lys Lys Gln Gly Met Leu Val Arg Asp Trp Val
            340                 345                 350

Pro Gln Leu Asp Ile Leu Arg His Glu Ala Thr Gly Gly Phe Leu Ser
        355                 360                 365

His Cys Gly Trp Asn Ser Val Leu Glu Gly Leu Arg Glu Gly Val Pro
    370                 375                 380

Ile Leu Gly Trp Pro Leu Ala Ala Glu Gln Ala Tyr Asn Ser Lys Met
385                 390                 395                 400

Met Val Glu Glu Met Gly Val Ala Val Glu Leu Thr Arg Gly Leu Glu
                405                 410                 415

Gly Glu Val Lys Lys Glu Trp Val Lys Ser Val Val Glu Met Val Leu
            420                 425                 430

Asp Arg Lys Glu Gly Ser Cys Gly Trp Glu Met Lys Lys Lys Ala Val
        435                 440                 445

Glu Ile Gly Glu Lys Leu Lys Asp Ala Trp Lys Ile Glu Gly Asp Tyr
    450                 455                 460

Lys Gly Ser Ser Val Lys Ala Met Asp Asn Phe Val Glu Phe Ile Val
465                 470                 475                 480

Ser Cys Arg Gly Lys Lys Ser Lys Glu Asp Ala
                485                 490

<210>  21
<211>  1446
<212>  DNA
<213>  Cleretum bellidiforme

<400>  21
atgagcaaaa tagagctggt gttggttcca accccaggga tgggccactt gctctccgcc      60

gtcgagctat ccaagctcat tatccgccgt gagaaccgta tctcagtcct catcctaatc     120

ttaagcttcc cctttgactc cggtttggtt aatgcctacg ttgatttcca gtcacgtgac     180

cctgacaact ccggtagcct aaccttcatc actctcccac cactctccaa catacctgac     240

tgcacttcga gcaccttctt caccaccgtc attgaactcc acaagccaaa tgtcaagcag     300

gttgtcgagg aacgagtcag gtccgggtca cccaagcctg ctgggtttgt tatagacatg     360

ctttgccctg ctatgatgga tgtcgcggag gagcttgagg taccttctta catattgttc     420

acctctgggg ctaacttgtt gaatgtagtg tttcatttcc tgtcactcgc tgataatggc     480

gtggatatcg ccactgaagt taatgatccg gataaggagg tggatgttcc cgggttcagg 540

aaccgggtgc cctgcaaggt cttacccttg cccttccttg agaaagattt tctagtgaag 600

cgcggaagga ggtttagacg atccaacggt attctagtga acacttccaa tgagctggaa 660

tcgtatgcga ttcagacgtt gcttgagcag gctaaggata ataagatccc acccgtttat 720

ccggtaggtc ccattttgga gctgaatagc aaaagccgtt gtgggactaa ggaagatgag 780

gaagtctcga tcatgaggtg gctagacgag cagccagtaa actcggtgct gttcgtttgc 840

tttggaagca tgggaacatt cgatgaggat caagtaaagg agatagccaa tggtctagag 900

cagtcggggt attgctttct atggtcctta cgccagccac ccccggaagg gaaggcgaca 960

ccaagcgagg aggccttcct ggacaccctt ccagaggggt tcgtagagcg tacatcccat 1020

aaggggaaga tcatcgggtg ggctccgcag gtatcaatac tagcccacaa ggctgttggg 1080

ggattcgtgt cacattgtgg ttggaactcg actttagaga gtctctggtt tggggtccca 1140

atggcaacgt ggccaattag cgcggagcag cagctgaacg catttgagct agtgaaagag 1200

ttcgggatgg cggtagagat tcggatggat ttttggcgag attgtaggaa aaatactcag 1260

agctttgtag taacgagtga ggagatagag aatggggtga agaagctgat gagtatggat 1320

gaagaaatgg tcgagaaagt aaagaagatg agtgataaga gtaggaagac tttagaggat 1380

ggcggatctt cgcatcactc gttgggccgt ttcattaatg atctcttgga gaacgctggc 1440

ttctga 1446

<210> 22
<211> 481
<212> PRT
<213> Cleretum bellidiforme

<400> 22

Met Ser Lys Ile Glu Leu Val Leu Val Pro Thr Pro Gly Met Gly His
1               5                   10                  15

Leu Leu Ser Ala Val Glu Leu Ser Lys Leu Ile Ile Arg Arg Glu Asn
            20                  25                  30

Arg Ile Ser Val Leu Ile Leu Ile Leu Ser Phe Pro Phe Asp Ser Gly
        35                  40                  45

Leu Val Asn Ala Tyr Val Asp Phe Gln Ser Arg Asp Pro Asp Asn Ser
    50                  55                  60

Gly Ser Leu Thr Phe Ile Thr Leu Pro Pro Leu Ser Asn Ile Pro Asp
65                  70                  75                  80

Cys Thr Ser Ser Thr Phe Phe Thr Thr Val Ile Glu Leu His Lys Pro

85                  90                  95

Asn Val Lys Gln Val Val Glu Glu Arg Val Arg Ser Gly Ser Pro Lys
            100                 105                 110

Pro Ala Gly Phe Val Ile Asp Met Leu Cys Pro Ala Met Met Asp Val
        115                 120                 125

Ala Glu Glu Leu Glu Val Pro Ser Tyr Ile Leu Phe Thr Ser Gly Ala
    130                 135                 140

Asn Leu Leu Asn Val Val Phe His Phe Leu Ser Leu Ala Asp Asn Gly
145                 150                 155                 160

Val Asp Ile Ala Thr Glu Val Asn Asp Pro Asp Lys Glu Val Asp Val
                165                 170                 175

Pro Gly Phe Arg Asn Arg Val Pro Cys Lys Val Leu Pro Leu Pro Phe
            180                 185                 190

Leu Glu Lys Asp Phe Leu Val Lys Arg Gly Arg Arg Phe Arg Arg Ser
        195                 200                 205

Asn Gly Ile Leu Val Asn Thr Ser Asn Glu Leu Glu Ser Tyr Ala Ile
    210                 215                 220

Gln Thr Leu Leu Glu Gln Ala Lys Asp Asn Lys Ile Pro Pro Val Tyr
225                 230                 235                 240

Pro Val Gly Pro Ile Leu Glu Leu Asn Ser Lys Ser Arg Cys Gly Thr
                245                 250                 255

Lys Glu Asp Glu Glu Val Ser Ile Met Arg Trp Leu Asp Glu Gln Pro
            260                 265                 270

Val Asn Ser Val Leu Phe Val Cys Phe Gly Ser Met Gly Thr Phe Asp
        275                 280                 285

Glu Asp Gln Val Lys Glu Ile Ala Asn Gly Leu Glu Gln Ser Gly Tyr
    290                 295                 300

Cys Phe Leu Trp Ser Leu Arg Gln Pro Pro Pro Glu Gly Lys Ala Thr
305                 310                 315                 320

Pro Ser Glu Glu Ala Phe Leu Asp Thr Leu Pro Glu Gly Phe Val Glu
                325                 330                 335

Arg Thr Ser His Lys Gly Lys Ile Ile Gly Trp Ala Pro Gln Val Ser
            340                 345                 350

Ile Leu Ala His Lys Ala Val Gly Gly Phe Val Ser His Cys Gly Trp
        355                 360                 365

Asn Ser Thr Leu Glu Ser Leu Trp Phe Gly Val Pro Met Ala Thr Trp
    370                 375                 380

Pro Ile Ser Ala Glu Gln Gln Leu Asn Ala Phe Glu Leu Val Lys Glu
385                 390                 395                 400

Phe Gly Met Ala Val Glu Ile Arg Met Asp Phe Trp Arg Asp Cys Arg
                405                 410                 415

Lys Asn Thr Gln Ser Phe Val Val Thr Ser Glu Glu Ile Glu Asn Gly
            420                 425                 430

Val Lys Lys Leu Met Ser Met Asp Glu Glu Met Val Glu Lys Val Lys
        435                 440                 445

Lys Met Ser Asp Lys Ser Arg Lys Thr Leu Glu Asp Gly Gly Ser Ser
    450                 455                 460

His His Ser Leu Gly Arg Phe Ile Asn Asp Leu Leu Glu Asn Ala Gly
465                 470                 475                 480

Phe

<210>  23
<211>  1389
<212>  DNA
<213>  Chenopodium quinoa

<400>  23
atgtcacaaa acaaagacaa ccaaattcta aacgttacat tttacccatg gtttgcacta      60

ggccacctta cttcttttct ccgcttagcc aacaaacttg ccgaaagagg tcacaatgtc     120

tcttattttc tcccaccaaa aacacaatca aaattagctt ctcacaacca ctacccaacc     180

caccttacct tcatccccat ccccgtccca cccgtcgaag gcctccctcc tggggccgag     240

acaaccaacg atgtacccgc ctcattaggc cctctcatca tgaccgccat ggacatgaca     300

cgtgacacta tcgagtctca tctagtccgt ctcaaacccg atattgtttt ttacgacttc     360

acttgttgga tgcccgaact aggccgaaaa cacgggttta aagccatgca ctatatcaca     420

gcatacatag caagatatgc atatcttgca ccatacaaaa aaataccagg atatcaccct     480

aatgcggatg atctattgac accaccacca gagtttccgt cacaatcaat caggatgctc     540

ccccaagagg ctgagatcat tgcgggtgcc cttaagacac cattcggtct aggaggacta 600

acactagctg agaggctagg tgtttcgttt cgagaatgcg acgcattcgg agttaagact 660

tgtgcggaga tggaagggga gtattgtaaa ttttttgaga aaatatttgg taagccagtg 720

ttactagcag ggccaatggt acctaaacgg ccatcttcgg aacttgataa ttattttgat 780

gattggctaa atagttttcg tacgtctagt gttatatatt gtgcacttgg aagtgaatgc 840

gctctgaatt tgaaccaatt tcaagagctt gttcttggat tggagcttac aggtaggcca 900

tttttggcag cgttaaagcc accaatgaat taccaaacaa tagaatcagc attgccagaa 960

ggacttgcgg aaagaataaa aggcagggga ctaattcatg ggggttgggt gcaacagcag 1020

ctcatcttac aacatccttc ggtaggatgt tttattacac attgtggtgc aggatctcta 1080

tccgaagcta tggtaagcga atgtcaagtc gtgctgatgc ctcaagcaat cgaccagttc 1140

atcagtgcgc ggatgatgag cttagagtgg aaggttgggg tcgaagttga gaagagaaag 1200

aattatggtt tgttcaccaa ggaggccgtc cataaggcgg tctccttggt gatggaagaa 1260

gatagtgaag ttgggagaga tgtaagggct aatcatgcta aatggagaga gtttatttta 1320

actgaaggtc ttgaggattc ttatattagt agcttcatac agagtttgca acaattgata 1380

ggatcataa 1389

<210> 24
<211> 462
<212> PRT
<213> Chenopodium quinoa

<400> 24

Met Ser Gln Asn Lys Asp Asn Gln Ile Leu Asn Val Thr Phe Tyr Pro
1               5                   10                  15

Trp Phe Ala Leu Gly His Leu Thr Ser Phe Leu Arg Leu Ala Asn Lys
            20                  25                  30

Leu Ala Glu Arg Gly His Asn Val Ser Tyr Phe Leu Pro Pro Lys Thr
        35                  40                  45

Gln Ser Lys Leu Ala Ser His Asn His Tyr Pro Thr His Leu Thr Phe
    50                  55                  60

Ile Pro Ile Pro Val Pro Pro Val Glu Gly Leu Pro Pro Gly Ala Glu
65                  70                  75                  80

Thr Thr Asn Asp Val Pro Ala Ser Leu Gly Pro Leu Ile Met Thr Ala
                85                  90                  95

Met Asp Met Thr Arg Asp Thr Ile Glu Ser His Leu Val Arg Leu Lys
            100                 105                 110

Pro Asp Ile Val Phe Tyr Asp Phe Thr Cys Trp Met Pro Glu Leu Gly
        115                 120                 125

Arg Lys His Gly Phe Lys Ala Met His Tyr Ile Thr Ala Tyr Ile Ala
    130                 135                 140

Arg Tyr Ala Tyr Leu Ala Pro Tyr Lys Lys Ile Pro Gly Tyr His Pro
145                 150                 155                 160

Asn Ala Asp Asp Leu Leu Thr Pro Pro Pro Glu Phe Pro Ser Gln Ser
                165                 170                 175

Ile Arg Met Leu Pro Gln Glu Ala Glu Ile Ile Ala Gly Ala Leu Lys
            180                 185                 190

Thr Pro Phe Gly Leu Gly Gly Leu Thr Leu Ala Glu Arg Leu Gly Val
        195                 200                 205

Ser Phe Arg Glu Cys Asp Ala Phe Gly Val Lys Thr Cys Ala Glu Met
    210                 215                 220

Glu Gly Glu Tyr Cys Lys Phe Phe Glu Lys Ile Phe Gly Lys Pro Val
225                 230                 235                 240

Leu Leu Ala Gly Pro Met Val Pro Lys Arg Pro Ser Ser Glu Leu Asp
                245                 250                 255

Asn Tyr Phe Asp Asp Trp Leu Asn Ser Phe Arg Thr Ser Ser Val Ile
            260                 265                 270

Tyr Cys Ala Leu Gly Ser Glu Cys Ala Leu Asn Leu Asn Gln Phe Gln
        275                 280                 285

Glu Leu Val Leu Gly Leu Glu Leu Thr Gly Arg Pro Phe Leu Ala Ala
    290                 295                 300

Leu Lys Pro Pro Met Asn Tyr Gln Thr Ile Glu Ser Ala Leu Pro Glu
305                 310                 315                 320

Gly Leu Ala Glu Arg Ile Lys Gly Arg Gly Leu Ile His Gly Gly Trp
                325                 330                 335

Val Gln Gln Gln Leu Ile Leu Gln His Pro Ser Val Gly Cys Phe Ile
            340                 345                 350

Thr His Cys Gly Ala Gly Ser Leu Ser Glu Ala Met Val Ser Glu Cys
        355                 360                 365

Gln Val Val Leu Met Pro Gln Ala Ile Asp Gln Phe Ile Ser Ala Arg
    370                 375                 380

Met Met Ser Leu Glu Trp Lys Val Gly Val Glu Val Glu Lys Arg Lys
385                 390                 395                 400

Asn Tyr Gly Leu Phe Thr Lys Glu Ala Val His Lys Ala Val Ser Leu
                405                 410                 415

Val Met Glu Glu Asp Ser Glu Val Gly Arg Asp Val Arg Ala Asn His
            420                 425                 430

Ala Lys Trp Arg Glu Phe Ile Leu Thr Glu Gly Leu Glu Asp Ser Tyr
        435                 440                 445

Ile Ser Ser Phe Ile Gln Ser Leu Gln Gln Leu Ile Gly Ser
    450                 455                 460

<210>  25
<211>  1389
<212>  DNA
<213>  Chenopodium quinoa

<400>  25
atgtcacaaa acaaagacac ccaaattcta aacgttgcat tttacccatg gtttgcacta      60

ggccacctta cttcttttct ccgcttagcc aacaaactag ccgaaagagg tcacaaagtc     120

tcctatttta tcccaccaaa aacacgatca aagttagctt ctcacaacca ctacccaacc     180

caccttacct tcatccccat ccccgtccca ccggtcgaag gcctccctcc cggggccgag     240

acaaccaacg atgtacccgc ctcatccggc ccactcatca tgaacgccat ggacatgaca     300

cgtgacacca tcgagaccca tctagtccga ctcaaacccg atattgtttt ttacgacttc     360

acttgttgga tgcccgaact agcccgaaaa cacgggttta aagcaataca ctatatcaca     420

gcatacatag caagatatgc atatcttgca ccatacaaaa aaataccagg gtatcaccct     480

aatgcgaatg atctattaac accaccacca gagtttccgt ctcaatcaat caggatgctc     540

ccccaagagg ccgagatcat ggcgggtgcc ggtaagacgc catttggtct agggggacta     600

acactggctg agaggctagg tgtttcgttt cgagaatgcg acgcattcgg ggttaagact     660

tgtacggaga tggaagggga gtattgtaag ttttttgaga aaatatttgg taagccagtg     720

ttactagcag ggccaatggt gcctaaaacg ccatcttcga aacttgataa gtattttgat     780

gattggttga atagttttgg taatgctagt gttatatatt gtgcacttgg aagtgaatgt     840

gctctgaaat tgaaccaatt tcaagagctt gttcttggac tagagcttac aggtaggcca 900

tttttggcag ccttaaagcc accaatgaat taccaaacaa tagaatcagc attaccagaa 960

ggatttgcgg aaagaacaaa aagcagggga cttattcatg gtggttgggt gcaacaacaa 1020

ctcatcgtac aacatccttc ggtaggatgt tttataacac attgtggtgc aggatctcta 1080

tccgaagcta tggtaagcga atgtcaagtc gtgttgatgc ctcaagcaat cgaccagttc 1140

atcagtgcgc gaatgatgag cttagagtgg aaggttgggg tcgaagttga gaagagaaag 1200

aatgatggtt tgttcaccaa ggaggccgtc cataaggcgg tctccttggt gatggaagaa 1260

gacagtgaag ttggaagaga tgtaagggct aatcatgcta aatggagaga gtttatatta 1320

actgaaggtc ttgaggattc ttatattagt agcttcatta tgagtttgca acaattgatt 1380

ggatcatga 1389

<210> 26
<211> 462
<212> PRT
<213> Chenopodium quinoa

<400> 26

Met Ser Gln Asn Lys Asp Thr Gln Ile Leu Asn Val Ala Phe Tyr Pro
1               5                   10                  15

Trp Phe Ala Leu Gly His Leu Thr Ser Phe Leu Arg Leu Ala Asn Lys
            20                  25                  30

Leu Ala Glu Arg Gly His Lys Val Ser Tyr Phe Ile Pro Pro Lys Thr
        35                  40                  45

Arg Ser Lys Leu Ala Ser His Asn His Tyr Pro Thr His Leu Thr Phe
    50                  55                  60

Ile Pro Ile Pro Val Pro Pro Val Glu Gly Leu Pro Pro Gly Ala Glu
65                  70                  75                  80

Thr Thr Asn Asp Val Pro Ala Ser Ser Gly Pro Leu Ile Met Asn Ala
                85                  90                  95

Met Asp Met Thr Arg Asp Thr Ile Glu Thr His Leu Val Arg Leu Lys
            100                 105                 110

Pro Asp Ile Val Phe Tyr Asp Phe Thr Cys Trp Met Pro Glu Leu Ala
        115                 120                 125

Arg Lys His Gly Phe Lys Ala Ile His Tyr Ile Thr Ala Tyr Ile Ala
    130                 135                 140

Arg Tyr Ala Tyr Leu Ala Pro Tyr Lys Lys Ile Pro Gly Tyr His Pro
145                 150                 155                 160

Asn Ala Asn Asp Leu Leu Thr Pro Pro Pro Glu Phe Pro Ser Gln Ser
                165                 170                 175

Ile Arg Met Leu Pro Gln Glu Ala Glu Ile Met Ala Gly Ala Gly Lys
            180                 185                 190

Thr Pro Phe Gly Leu Gly Gly Leu Thr Leu Ala Glu Arg Leu Gly Val
        195                 200                 205

Ser Phe Arg Glu Cys Asp Ala Phe Gly Val Lys Thr Cys Thr Glu Met
    210                 215                 220

Glu Gly Glu Tyr Cys Lys Phe Phe Glu Lys Ile Phe Gly Lys Pro Val
225                 230                 235                 240

Leu Leu Ala Gly Pro Met Val Pro Lys Thr Pro Ser Ser Lys Leu Asp
                245                 250                 255

Lys Tyr Phe Asp Asp Trp Leu Asn Ser Phe Gly Asn Ala Ser Val Ile
            260                 265                 270

Tyr Cys Ala Leu Gly Ser Glu Cys Ala Leu Lys Leu Asn Gln Phe Gln
        275                 280                 285

Glu Leu Val Leu Gly Leu Glu Leu Thr Gly Arg Pro Phe Leu Ala Ala
    290                 295                 300

Leu Lys Pro Pro Met Asn Tyr Gln Thr Ile Glu Ser Ala Leu Pro Glu
305                 310                 315                 320

Gly Phe Ala Glu Arg Thr Lys Ser Arg Gly Leu Ile His Gly Gly Trp
                325                 330                 335

Val Gln Gln Gln Leu Ile Val Gln His Pro Ser Val Gly Cys Phe Ile
            340                 345                 350

Thr His Cys Gly Ala Gly Ser Leu Ser Glu Ala Met Val Ser Glu Cys
        355                 360                 365

Gln Val Val Leu Met Pro Gln Ala Ile Asp Gln Phe Ile Ser Ala Arg
    370                 375                 380

Met Met Ser Leu Glu Trp Lys Val Gly Val Glu Val Glu Lys Arg Lys
385                 390                 395                 400

Asn Asp Gly Leu Phe Thr Lys Glu Ala Val His Lys Ala Val Ser Leu
                405                 410                 415

Val Met Glu Glu Asp Ser Glu Val Gly Arg Asp Val Arg Ala Asn His
            420                 425                 430

Ala Lys Trp Arg Glu Phe Ile Leu Thr Glu Gly Leu Glu Asp Ser Tyr
        435                 440                 445

Ile Ser Ser Phe Ile Met Ser Leu Gln Gln Leu Ile Gly Ser
    450                 455                 460

<210>  27
<211>  1359
<212>  DNA
<213>  Beta vulgaris

<400>  27
atgggtgaaa acaaagaatc gcaagttttg aaggtagcat tctacccttg gtttgcactt      60

ggccacctta cttcattcct tcgcttagcc aacaaacttg ctgaaagagg tcaccaggtc     120

tcatattttc tgccgaagaa tacacaactt aaattagctt ctcaaaacca cttcccagac     180

catctcacct tcatccccat caccgttcca tccgtagacg gccttcctcc tggggccgag     240

acgaccaacg atgtatcctt gtcgggtgcg caccttatca tggctgccat ggacatgact     300

cgtgatacta ttgatgccca tctggccctt ctcaagcccg actttgtttt ctatgatttc     360

gcttattgga tgcctcaact ggctcggaaa catgggatca aatcagtgca ctacatcact     420

ggattaatag caagatatac tctagctatc tcccgtatca caggggcgta tccacccgat     480

ctctcgtccc caatttttca gatgcgtgcc catgagatga aggttatgga gcatataagt     540

gagaagccat tcgcaccagg aggtgtgacg ttgccggaga tgttcaggat atcgttcgaa     600

gaatgtgatg ctgtcggagt caagacttgt aaggaaatgg aaggaattta ctgtgagttt     660

gctgagaaga cgttagataa gcctgtgctt ctcgcagggc ctgttgtccc aaaactacca     720

tcttctaaac ttgatgacta tgttgatgcc tggctaagta cttttggttc tggtactgtt     780

atattttgtg ctcttggaag tgaatgtatt cttgaacaaa accagtttca agatattctt     840

cttggactcg agctcacagg taagccattt ttggcggccc tgaagccgcc caagaattgc     900

aaaagtttag agtcgggttt accagaagga ttcagtgaga gagtaagagg aagaggaatg     960

atccatggag gttgggtgca gcaacaactg atcttacaac atccatcagt gggatgtttc    1020

ataactcatt gtggggttgg atctctatca gaagctatgg taagccaatg tcaagttgta    1080

cttatgcctc aagcagtcga tcaattcatg aatgcgaggc agatgagttt agaattgaag    1140

atcggagtta aggtcgagtc cacagagact gatggttttt tcactaggga agccctctgt    1200

aaggcggtct cccttgtgat ggatgaacaa agtgaagttg cgagagaagt gaaggcaaat 1260

catgctaaat ggagagattt tattttaacc gaaggtcttg aggactctta tattagtagc 1320

ttcattcaga gtctacaaca cttgcttcag atgaactga 1359

<210> 28
<211> 452
<212> PRT
<213> Beta vulgaris

<400> 28

Met Gly Glu Asn Lys Glu Ser Gln Val Leu Lys Val Ala Phe Tyr Pro
1               5                   10                  15

Trp Phe Ala Leu Gly His Leu Thr Ser Phe Leu Arg Leu Ala Asn Lys
            20                  25                  30

Leu Ala Glu Arg Gly His Gln Val Ser Tyr Phe Leu Pro Lys Asn Thr
        35                  40                  45

Gln Leu Lys Leu Ala Ser Gln Asn His Phe Pro Asp His Leu Thr Phe
    50                  55                  60

Ile Pro Ile Thr Val Pro Ser Val Asp Gly Leu Pro Pro Gly Ala Glu
65                  70                  75                  80

Thr Thr Asn Asp Val Ser Leu Ser Gly Ala His Leu Ile Met Ala Ala
                85                  90                  95

Met Asp Met Thr Arg Asp Thr Ile Asp Ala His Leu Ala Leu Leu Lys
            100                 105                 110

Pro Asp Phe Val Phe Tyr Asp Phe Ala Tyr Trp Met Pro Gln Leu Ala
        115                 120                 125

Arg Lys His Gly Ile Lys Ser Val His Tyr Ile Thr Gly Leu Ile Ala
    130                 135                 140

Arg Tyr Thr Leu Ala Ile Ser Arg Ile Thr Gly Ala Tyr Pro Pro Asp
145                 150                 155                 160

Leu Ser Ser Pro Ile Phe Gln Met Arg Ala His Glu Met Lys Val Met
                165                 170                 175

Glu His Ile Ser Glu Lys Pro Phe Ala Pro Gly Gly Val Thr Leu Pro
            180                 185                 190

Glu Met Phe Arg Ile Ser Phe Glu Glu Cys Asp Ala Val Gly Val Lys
        195                 200                 205

Thr Cys Lys Glu Met Glu Gly Ile Tyr Cys Glu Phe Ala Glu Lys Thr
    210                 215                 220

Leu Asp Lys Pro Val Leu Leu Ala Gly Pro Val Val Pro Lys Leu Pro
225                 230                 235                 240

Ser Ser Lys Leu Asp Asp Tyr Val Asp Ala Trp Leu Ser Thr Phe Gly
                245                 250                 255

Ser Gly Thr Val Ile Phe Cys Ala Leu Gly Ser Glu Cys Ile Leu Glu
            260                 265                 270

Gln Asn Gln Phe Gln Asp Ile Leu Leu Gly Leu Glu Leu Thr Gly Lys
        275                 280                 285

Pro Phe Leu Ala Ala Leu Lys Pro Pro Lys Asn Cys Lys Ser Leu Glu
    290                 295                 300

Ser Gly Leu Pro Glu Gly Phe Ser Glu Arg Val Arg Gly Arg Gly Met
305                 310                 315                 320

Ile His Gly Gly Trp Val Gln Gln Gln Leu Ile Leu Gln His Pro Ser
                325                 330                 335

Val Gly Cys Phe Ile Thr His Cys Gly Val Gly Ser Leu Ser Glu Ala
            340                 345                 350

Met Val Ser Gln Cys Gln Val Val Leu Met Pro Gln Ala Val Asp Gln
        355                 360                 365

Phe Met Asn Ala Arg Gln Met Ser Leu Glu Leu Lys Ile Gly Val Lys
    370                 375                 380

Val Glu Ser Thr Glu Thr Asp Gly Phe Phe Thr Arg Glu Ala Leu Cys
385                 390                 395                 400

Lys Ala Val Ser Leu Val Met Asp Glu Gln Ser Glu Val Ala Arg Glu
                405                 410                 415

Val Lys Ala Asn His Ala Lys Trp Arg Asp Phe Ile Leu Thr Glu Gly
            420                 425                 430

Leu Glu Asp Ser Tyr Ile Ser Ser Phe Ile Gln Ser Leu Gln His Leu
        435                 440                 445

Leu Gln Met Asn
    450

<210>  29
<211>  1371
<212>  DNA
<213>  Amaranthus hypochondriacus

<400>  29
atgggttaca aagaagagtc ctctgttatg catatagcat tttacccatg gctagctcta      60

ggccatataa cctccttcct tcgtttagcc aacaaacttg cacaaaaagg tcatactatc     120

tcattcttca tccctaccaa aacccaatcc aaattagctt ctcaaaacca cttccctaac     180

cacctcacct tcgtccccgt cgatatcccg tctatcgacg ggcttcctcc aggggccgag     240

accaccaacg atgtgtcggt ctcggccgca ccccttatca tgtccgctat ggacatgacc     300

cgtgactcta tcgaaaccca attagtccat cttaaacccg attttgtttt ctatgacttt     360

gcgtattgga tgcctgaatt gggtaaaaaa catgggttca aatccgtgca ttacatcaca     420

ggttatatag caagatacgc tgcttttgct gcttacatta cagcacccga ccatactaac     480

actttagacc caccaccagg gctttcttcc ccaatattta agatgcaggc ccatgagtcc     540

cggattctat cagcggtaag taaacggccg tttgggtcaa caggtaaaac gatgactgaa     600

atgtttggta tatcgtttag ggaatgcgat gcgattggag ctaaaacttg tacggaaatg     660

gaaggggaat attatgagtt tgttaaaaag actttaggta agccattgtt gttagccggg     720

cctgttgttc ctattcaacc agcatctaag ttagatgaga aagttgatga atggttaaat     780

ggttttggag ctgaaactgt gatttattgt gcacttggaa gtgaatgtgt tctagaactt     840

tctcagtttc aacagattct tcttggatta gagctaacag gtaggccatt ttttgcagcc     900

ctaaagcccc ccaagaatta tgaaactata gagtcagcct tgccgaaaga gttcgaggag     960

agaataaaag gaaaaggaat cattgatagt ggttgggttc aacaacaact aatattaaaa    1020

catccatcaa taggatgttt tataacacat tgtggagttg gatctctgtc tgaagctatg    1080

gtaagccatt gtcaagtggt gtttatgcct caagccgtgg accaattcat caacgcaagg    1140

caaatgagcc ttgagttgaa gatcggggtg gaagttgagt cgcgagagga ggatggtttt    1200

tacaccaagg aggccgtcag taaagcggtc tccttggtga tggatgaaca tagtgaagtt    1260

ggtagagaag taagagctaa acatgctaaa tggagagatt ttattttaaa acaaggtctt    1320

gaggattctt atattactag ctttatcaat agtttacgac aattgcttta a             1371

<210>  30
<211>  456
<212>  PRT
<213>  Amaranthus hypochondriacus

<400>  30

Met Gly Tyr Lys Glu Glu Ser Ser Val Met His Ile Ala Phe Tyr Pro
1               5                   10                  15

Trp Leu Ala Leu Gly His Ile Thr Ser Phe Leu Arg Leu Ala Asn Lys
            20                  25                  30

Leu Ala Gln Lys Gly His Thr Ile Ser Phe Phe Ile Pro Thr Lys Thr
        35                  40                  45

Gln Ser Lys Leu Ala Ser Gln Asn His Phe Pro Asn His Leu Thr Phe
    50                  55                  60

Val Pro Val Asp Ile Pro Ser Ile Asp Gly Leu Pro Pro Gly Ala Glu
65                  70                  75                  80

Thr Thr Asn Asp Val Ser Val Ser Ala Ala Pro Leu Ile Met Ser Ala
                85                  90                  95

Met Asp Met Thr Arg Asp Ser Ile Glu Thr Gln Leu Val His Leu Lys
            100                 105                 110

Pro Asp Phe Val Phe Tyr Asp Phe Ala Tyr Trp Met Pro Glu Leu Gly
        115                 120                 125

Lys Lys His Gly Phe Lys Ser Val His Tyr Ile Thr Gly Tyr Ile Ala
    130                 135                 140

Arg Tyr Ala Ala Phe Ala Ala Tyr Ile Thr Ala Pro Asp His Thr Asn
145                 150                 155                 160

Thr Leu Asp Pro Pro Pro Gly Leu Ser Ser Pro Ile Phe Lys Met Gln
                165                 170                 175

Ala His Glu Ser Arg Ile Leu Ser Ala Val Ser Lys Arg Pro Phe Gly
            180                 185                 190

Ser Thr Gly Lys Thr Met Thr Glu Met Phe Gly Ile Ser Phe Arg Glu
        195                 200                 205

Cys Asp Ala Ile Gly Ala Lys Thr Cys Thr Glu Met Glu Gly Glu Tyr
    210                 215                 220

Tyr Glu Phe Val Lys Lys Thr Leu Gly Lys Pro Leu Leu Leu Ala Gly
225                 230                 235                 240

Pro Val Val Pro Ile Gln Pro Ala Ser Lys Leu Asp Glu Lys Val Asp

245                 250                 255

Glu Trp Leu Asn Gly Phe Gly Ala Glu Thr Val Ile Tyr Cys Ala Leu
            260                 265                 270

Gly Ser Glu Cys Val Leu Glu Leu Ser Gln Phe Gln Gln Ile Leu Leu
        275                 280                 285

Gly Leu Glu Leu Thr Gly Arg Pro Phe Phe Ala Ala Leu Lys Pro Pro
    290                 295                 300

Lys Asn Tyr Glu Thr Ile Glu Ser Ala Leu Pro Lys Glu Phe Glu Glu
305                 310                 315                 320

Arg Ile Lys Gly Lys Gly Ile Ile Asp Ser Gly Trp Val Gln Gln Gln
                325                 330                 335

Leu Ile Leu Lys His Pro Ser Ile Gly Cys Phe Ile Thr His Cys Gly
            340                 345                 350

Val Gly Ser Leu Ser Glu Ala Met Val Ser His Cys Gln Val Val Phe
        355                 360                 365

Met Pro Gln Ala Val Asp Gln Phe Ile Asn Ala Arg Gln Met Ser Leu
    370                 375                 380

Glu Leu Lys Ile Gly Val Glu Val Glu Ser Arg Glu Glu Asp Gly Phe
385                 390                 395                 400

Tyr Thr Lys Glu Ala Val Ser Lys Ala Val Ser Leu Val Met Asp Glu
                405                 410                 415

His Ser Glu Val Gly Arg Glu Val Arg Ala Lys His Ala Lys Trp Arg
            420                 425                 430

Asp Phe Ile Leu Lys Gln Gly Leu Glu Asp Ser Tyr Ile Thr Ser Phe
        435                 440                 445

Ile Asn Ser Leu Arg Gln Leu Leu
    450                 455

<210>  31
<211>  1386
<212>  DNA
<213>  Amaranthus hypochondriacus

<400>  31
atgtctcaca acaaagaatc caacccaaat cttcacgtag cattttaccc ttggtttgca        60

ttaggccact taacttcatt ccttcgttta gccaacaaac tagccgaaag aggccttcaa 120

gtctcttatt ttatcccatc caaaacccaa ccaaaactat cccctcacaa ccaccaccct 180

aaccacctta ccttcatacc catcaccgtc ccacacgtcg acggcctccc acccggagcc 240

gagaccacca acgatgtccc cggctcagcc gtacccctca tcatgaccgc aatggactta 300

acccaagaca taattgaggc ccatctagcc caactcaaac ccaactttgt tttctatgac 360

ttcacttatt ggatacctaa actaggccaa aaacttgggt ttaaatctat ccactatttt 420

accgcattta tatcaagata tggttatctt gcaccttaca aaaaggaagg ataccttcca 480

acggcggccg atcttcttcg gccgccacca ggctacccat ccccaataag gatgaagcct 540

tacgaagcca aaattatggc gggcgcaggt aaaaccgcct ttggactagg cgggtttacg 600

ctggccgaga gattagccgt gtcatttata gagtgtgatg catttggggt taagacttgt 660

aaggagatgg aaggtgaaca ttgtaagttt tttgaggatg tttttggtaa gccagtgctt 720

ttagctggac cagttgtgcc taaacttcca tcttctaaac tagatgaaca ttttgatgag 780

tggttaaatg ggtttgatga atctagtgtg attttttgtg cacttggaag tgaatgttct 840

ttggaaatta accaatttca tgagcttctt cttggattag agctcacagg tagaccattt 900

ttggcggctc taaagccgcc aaaaaattac aaaacaatag aatcagcatt accagaaggt 960

tttgcaagca gaactagaga aagaggaata gttcatgaag gttgggtgca acaacaacta 1020

atcttacaac atcgatcagt aggatgtttt ataactcatt gtggagttgg ttctttatcc 1080

gaagctatga taagcaaatg tcaagtagtg atgatacctc aagcaattga tcaattcatc 1140

aatgcaagga tgatgagttt agagtggaag attggagtcg aaatcgagac gagggaggat 1200

gatggttggt tcactaggga ggatgttcac aaggcaatta caatggtgat ggatggagaa 1260

agtgatgttg ggagagaagt tagggctaac catgctaaat ggagggactt tattttaaca 1320

caaggtgttg aagattctta tattagtagc tttatagaga gtttacaaca attgttaatt 1380

gtatag 1386

<210> 32
<211> 461
<212> PRT
<213> Amaranthus hypochondriacus

<400> 32

Met Ser His Asn Lys Glu Ser Asn Pro Asn Leu His Val Ala Phe Tyr
1               5                   10                  15

Pro Trp Phe Ala Leu Gly His Leu Thr Ser Phe Leu Arg Leu Ala Asn
            20                  25                  30

Lys Leu Ala Glu Arg Gly Leu Gln Val Ser Tyr Phe Ile Pro Ser Lys

        35                  40                  45

Thr Gln Pro Lys Leu Ser Pro His Asn His His Pro Asn His Leu Thr
    50                  55                  60

Phe Ile Pro Ile Thr Val Pro His Val Asp Gly Leu Pro Pro Gly Ala
65                  70                  75                  80

Glu Thr Thr Asn Asp Val Pro Gly Ser Ala Val Pro Leu Ile Met Thr
                85                  90                  95

Ala Met Asp Leu Thr Gln Asp Ile Ile Glu Ala His Leu Ala Gln Leu
            100                 105                 110

Lys Pro Asn Phe Val Phe Tyr Asp Phe Thr Tyr Trp Ile Pro Lys Leu
        115                 120                 125

Gly Gln Lys Leu Gly Phe Lys Ser Ile His Tyr Phe Thr Ala Phe Ile
    130                 135                 140

Ser Arg Tyr Gly Tyr Leu Ala Pro Tyr Lys Lys Glu Gly Tyr Leu Pro
145                 150                 155                 160

Thr Ala Ala Asp Leu Leu Arg Pro Pro Pro Gly Tyr Pro Ser Pro Ile
                165                 170                 175

Arg Met Lys Pro Tyr Glu Ala Lys Ile Met Ala Gly Ala Gly Lys Thr
            180                 185                 190

Ala Phe Gly Leu Gly Gly Phe Thr Leu Ala Glu Arg Leu Ala Val Ser
        195                 200                 205

Phe Ile Glu Cys Asp Ala Phe Gly Val Lys Thr Cys Lys Glu Met Glu
    210                 215                 220

Gly Glu His Cys Lys Phe Phe Glu Asp Val Phe Gly Lys Pro Val Leu
225                 230                 235                 240

Leu Ala Gly Pro Val Val Pro Lys Leu Pro Ser Ser Lys Leu Asp Glu
                245                 250                 255

His Phe Asp Glu Trp Leu Asn Gly Phe Asp Glu Ser Ser Val Ile Phe
            260                 265                 270

Cys Ala Leu Gly Ser Glu Cys Ser Leu Glu Ile Asn Gln Phe His Glu
        275                 280                 285

Leu Leu Leu Gly Leu Glu Leu Thr Gly Arg Pro Phe Leu Ala Ala Leu
    290                 295                 300

Lys Pro Pro Lys Asn Tyr Lys Thr Ile Glu Ser Ala Leu Pro Glu Gly
305                 310                 315                 320

Phe Ala Ser Arg Thr Arg Glu Arg Gly Ile Val His Glu Gly Trp Val
                325                 330                 335

Gln Gln Gln Leu Ile Leu Gln His Arg Ser Val Gly Cys Phe Ile Thr
            340                 345                 350

His Cys Gly Val Gly Ser Leu Ser Glu Ala Met Ile Ser Lys Cys Gln
        355                 360                 365

Val Val Met Ile Pro Gln Ala Ile Asp Gln Phe Ile Asn Ala Arg Met
    370                 375                 380

Met Ser Leu Glu Trp Lys Ile Gly Val Glu Ile Glu Thr Arg Glu Asp
385                 390                 395                 400

Asp Gly Trp Phe Thr Arg Glu Asp Val His Lys Ala Ile Thr Met Val
                405                 410                 415

Met Asp Gly Glu Ser Asp Val Gly Arg Glu Val Arg Ala Asn His Ala
            420                 425                 430

Lys Trp Arg Asp Phe Ile Leu Thr Gln Gly Val Glu Asp Ser Tyr Ile
        435                 440                 445

Ser Ser Phe Ile Glu Ser Leu Gln Gln Leu Leu Ile Val
    450                 455                 460

<210>  33
<211>  1263
<212>  DNA
<213>  Arabidopsis thaliana

<400>  33
atgggttaca aagaagagtc ctctgttatg cacatagcat tttacccatg gttagctcta        60

ggccatataa cctccttcct tcgtttagcc aacaaacttg cacaaaaagg tcacactatc       120

tcattcttca tccctaccaa aacccaatcc aaattagctt ctcaaaacca cttccctaac       180

cacctcacct tcatccccgt cgatatcccg tctatcgacg ggcttcctct aggggccgag       240

actaacgatg tgtcggtctc ggccgcaccc cttatcatgt ccgctatgga catgacccgt       300

gaatctatcg aaacccaatt agtccatctt aaacccgact ttgttttcta tgactttgct       360

tattggatgc ctgaattggg taaaaaacat gggttcaaat ccgtgcatta catcacagga       420

tatttaacaa gatacgctgc ttttgctgct tacattacag cacccgatca tactgacatt 480

ttgggcccac caccagggct ttcttcccca atctttaaga cacgggccca cgaggcccgg 540

gtcggtctat cagcagtgat caaaaaaccg tttggattaa caggtaaaac attgattgaa 600

atgtttggta tatcgtttag ggaatgcgat gcaattggag caaatacttg tatggaaatg 660

gaaggggaat attatgagtt tgtaaacaag actttgggta agccattgtt gttagccggg 720

cctgttgttc caattcaacc agcatctaag ttagatgaga aggttaatga atggttaaat 780

ggttttggtg ctgaaactgt ggtttattgt gcacttggaa agtcagcctt gtcagaagag 840

tttaaggaga gaataaaagg gaaaggaatc attgacggtg gttgggttca gcaacaacta 900

atattgaaac atccatcagt aggatgtttt gtaacacatt gtggaattgg atctctatct 960

gaagctatgg taagccattg tcaaatggtg tttatgcctc aagctgtgga ccagttcatc 1020

aatgcaaggc aaatgagcct tgagttgaag atcggggcgg aagttgagtc gcgagaggag 1080

gatggttttt acaccaagga ggccatcagt aaggcggtct cctcggtgat ggatgaacat 1140

agtgaagttg gtcgagaagt aagagctaac catgctaaat ggagagactt tattttaaaa 1200

caaggtcttg aggactctta tattattagc ttcatcaata gtttacaaca attgcttcat 1260

taa 1263

<210> 34
<211> 420
<212> PRT
<213> Arabidopsis thaliana

<400> 34

Met Gly Tyr Lys Glu Glu Ser Ser Val Met His Ile Ala Phe Tyr Pro
1               5                   10                  15

Trp Leu Ala Leu Gly His Ile Thr Ser Phe Leu Arg Leu Ala Asn Lys
            20                  25                  30

Leu Ala Gln Lys Gly His Thr Ile Ser Phe Phe Ile Pro Thr Lys Thr
        35                  40                  45

Gln Ser Lys Leu Ala Ser Gln Asn His Phe Pro Asn His Leu Thr Phe
    50                  55                  60

Ile Pro Val Asp Ile Pro Ser Ile Asp Gly Leu Pro Leu Gly Ala Glu
65                  70                  75                  80

Thr Asn Asp Val Ser Val Ser Ala Ala Pro Leu Ile Met Ser Ala Met
                85                  90                  95

Asp Met Thr Arg Glu Ser Ile Glu Thr Gln Leu Val His Leu Lys Pro
        100                 105                 110

Asp Phe Val Phe Tyr Asp Phe Ala Tyr Trp Met Pro Glu Leu Gly Lys
    115                 120                 125

Lys His Gly Phe Lys Ser Val His Tyr Ile Thr Gly Tyr Leu Thr Arg
    130                 135                 140

Tyr Ala Ala Phe Ala Ala Tyr Ile Thr Ala Pro Asp His Thr Asp Ile
145                 150                 155                 160

Leu Gly Pro Pro Pro Gly Leu Ser Ser Pro Ile Phe Lys Thr Arg Ala
                165                 170                 175

His Glu Ala Arg Val Gly Leu Ser Ala Val Ile Lys Lys Pro Phe Gly
        180                 185                 190

Leu Thr Gly Lys Thr Leu Ile Glu Met Phe Gly Ile Ser Phe Arg Glu
    195                 200                 205

Cys Asp Ala Ile Gly Ala Asn Thr Cys Met Glu Met Glu Gly Glu Tyr
    210                 215                 220

Tyr Glu Phe Val Asn Lys Thr Leu Gly Lys Pro Leu Leu Leu Ala Gly
225                 230                 235                 240

Pro Val Val Pro Ile Gln Pro Ala Ser Lys Leu Asp Glu Lys Val Asn
                245                 250                 255

Glu Trp Leu Asn Gly Phe Gly Ala Glu Thr Val Val Tyr Cys Ala Leu
        260                 265                 270

Gly Lys Ser Ala Leu Ser Glu Glu Phe Lys Glu Arg Ile Lys Gly Lys
    275                 280                 285

Gly Ile Ile Asp Gly Gly Trp Val Gln Gln Gln Leu Ile Leu Lys His
    290                 295                 300

Pro Ser Val Gly Cys Phe Val Thr His Cys Gly Ile Gly Ser Leu Ser
305                 310                 315                 320

Glu Ala Met Val Ser His Cys Gln Met Val Phe Met Pro Gln Ala Val
                325                 330                 335

Asp Gln Phe Ile Asn Ala Arg Gln Met Ser Leu Glu Leu Lys Ile Gly
        340                 345                 350

Ala Glu Val Glu Ser Arg Glu Glu Asp Gly Phe Tyr Thr Lys Glu Ala
        355                 360                 365

Ile Ser Lys Ala Val Ser Ser Val Met Asp Glu His Ser Glu Val Gly
    370                 375                 380

Arg Glu Val Arg Ala Asn His Ala Lys Trp Arg Asp Phe Ile Leu Lys
385                 390                 395                 400

Gln Gly Leu Glu Asp Ser Tyr Ile Ile Ser Phe Ile Asn Ser Leu Gln
                405                 410                 415

Gln Leu Leu His
            420

<210>  35
<211>  272
<212>  PRT
<213>  Chenopodium quinoa

<400>  35

Met Lys Met Gly Gly Glu Asn Asn Asn Ile Ile Asn Glu Thr Phe Phe
1               5                   10                  15

Ile Thr His Gly Asn Pro Ile Leu Thr Val Glu Asp Thr His Pro Leu
            20                  25                  30

Arg Pro Phe Phe Glu Thr Trp Thr Lys Lys Ile Phe Ser Lys Lys Pro
        35                  40                  45

Lys Ala Ile Leu Val Ile Ser Gly His Trp Glu Thr Asp His Pro Ala
    50                  55                  60

Val Asn Ala Val His Leu Asn Asp Thr Ile Tyr Asp Phe Asp Asp Tyr
65                  70                  75                  80

Pro Gln Ala Met Tyr Lys Leu Lys Tyr Pro Ala Pro Gly Ser Pro Asp
                85                  90                  95

Leu Ala Ser Arg Ile Glu Glu Ile Leu Lys Lys Ser Gly Phe Asp Thr
            100                 105                 110

Val His Ile Asp Lys Lys Arg Gly Leu Asp His Gly Ala Trp Val Pro
        115                 120                 125

Leu Met Tyr Met Tyr Pro Glu Ala Asp Ile Pro Val Cys Gln Leu Ser
    130                 135                 140

Val Gln Pro Lys Met Asp Gly Thr Tyr His Tyr Asn Leu Gly Arg Ala
145                 150                 155                 160

Leu Ala Pro Leu Lys Asp Glu Gly Val Leu Ile Ile Gly Ser Gly Ser
                165                 170                 175

Ala Thr His Pro Leu Asp Glu Thr Pro His Tyr His Gly Gly Val Ala
            180                 185                 190

Pro Trp Ala Ala Asp Phe Asp Ser Trp Leu Asp Leu Ala Leu Thr Lys
        195                 200                 205

Gly Arg Phe Glu Glu Val Asn Thr Tyr Glu Thr Lys Ala Pro Asn Trp
    210                 215                 220

Glu Leu Ala His Pro Phe Pro Glu His Phe Tyr Pro Leu His Val Val
225                 230                 235                 240

Val Gly Ala Ala Gly Glu Lys Trp Lys Ala Glu Leu Ile His Thr Ser
                245                 250                 255

Trp Asp His Gly Thr Leu Cys His Ala Ser Tyr Lys Phe Thr Ser Thr
            260                 265                 270

<210>  36
<211>  819
<212>  DNA
<213>  Chenopodium quinoa

<400>  36
atgaaaatgg gtggggaaaa taacaatata atcaatgaaa ccttcttcat aacccatgga      60

aacccgattt tgaccgtaga agacacacat cccttaaggc cattctttga aacttggact     120

aaaaaaatat tttcgaagaa gccgaaggcg atacttgtga tctccggtca ctgggaaact     180

gatcatcctg ctgttaatgc tgttcatctt aatgatacta tctacgattt tgatgactat     240

cctcaagcaa tgtacaagtt gaagtatcca gcaccagggt caccagactt ggctagcaga     300

atagaagaaa ttctgaaaaa gtccgggttc gacacggtgc acattgacaa aaaacgtggg     360

cttgatcatg gtgcatgggt gcctcttatg tacatgtatc cggaggctga cattccggtg     420

tgtcagctct ccgtgcagcc gaaaatggac ggaacgtacc actacaactt gggacgggcg     480

ttggctccgc tgaaagacga aggtgtactc attattggtt ccggaagtgc aacacaccca     540

ttggacgaaa cccctcatta tcatggtggt gttgctcctt gggctgctga ctttgactca     600

tggcttgacc tagctctcac taaaggaagg tttgaagaag tgaatacata tgaaaccaaa     660

gcaccaaact gggaattggc acatccattc ccggagcatt tttatccatt gcacgtcgtc     720

gttggggcgg ctggtgaaaa gtggaaggct gagcttattc atactagttg ggaccatggt 780

accttatgtc atgcctccta caagtttact tccacctaa 819

<210> 37
<211> 276
<212> PRT
<213> Chenopodium quinoa

<400> 37

Met Lys Met Val Val Glu Glu Gly Lys Asn Asn Glu Met Lys Phe Asn
1               5                   10                  15

Glu Thr Phe Phe Ile Thr His Gly Asn Pro Ile Leu Thr Val Glu Asp
            20                  25                  30

Thr His Pro Leu Arg Pro Phe Phe Glu Thr Trp Thr Glu Lys Ile Phe
        35                  40                  45

Ser Lys Lys Pro Lys Ala Ile Leu Val Ile Ser Gly His Trp Glu Thr
    50                  55                  60

Asp His Pro Ala Val Asn Ala Val His Ile Asn Asp Thr Ile Tyr Asp
65                  70                  75                  80

Phe Asp Asp Tyr Pro Glu Ala Met Tyr Lys Phe Lys Tyr Pro Ala Pro
                85                  90                  95

Gly Ser Leu Glu Leu Ala Lys Gly Val Gln Gln Leu Leu Asn Lys Ser
            100                 105                 110

Lys Phe Glu Thr Val His Ile Asp Gln Lys Arg Gly Leu Asp His Gly
        115                 120                 125

Val Trp Val Pro Leu Met Tyr Met Tyr Pro Glu Ala Asp Ile Pro Val
    130                 135                 140

Cys Gln Leu Ser Val Gln Pro Lys Met Asp Gly Thr Tyr His Tyr Asn
145                 150                 155                 160

Leu Gly Arg Ala Leu Ala Pro Leu Arg Asp Glu Gly Ile Leu Ile Ile
                165                 170                 175

Gly Ser Gly Ser Ala Thr His Pro Leu Asp Glu Thr Pro His Tyr His
            180                 185                 190

Gly Gly Val Ala Pro Trp Ala Ala Glu Phe Asp Ser Trp Leu Asp Thr
        195                 200                 205

Ala Leu Thr Asn Gly Arg Ile Glu Glu Val Asn Thr Tyr Glu Thr Lys
    210                 215                 220

Ala Pro Asn Trp Glu Leu Ala His Pro Phe Pro Glu His Phe Tyr Pro
225                 230                 235                 240

Leu His Val Val Val Gly Ala Ala Gly Glu Lys Trp Lys Ala Glu Leu
                245                 250                 255

Ile His Thr Ser Trp Asp His Gly Thr Leu Cys His Gly Ala Tyr Lys
            260                 265                 270

Phe Thr Ser Ser
        275

<210>  38
<211>  831
<212>  DNA
<213>  Chenopodium quinoa

<400>  38
atgaaaatgg ttgttgaaga gggaaaaaat aacgaaatga aattcaatga aaccttcttc      60

ataacccatg gaaacccaat tttgacagta gaagacacac atcccttaag gccattcttt     120

gaaacttgga ctgaaaaaat attttcgaag aagccgaagg cgatacttgt gatctccggt     180

cactgggaaa ctgatcatcc tgctgttaat gctgttcata tcaatgatac tatctacgat     240

tttgatgact atcctgaagc catgtacaag tttaagtatc cggctcccgg gtctctagaa     300

ttggcaaaag gggtgcaaca actactcaac aaatccaagt tcgaaaccgt gcacattgac     360

caaaaacgtg ggcttgatca tggtgtatgg gtgcctctta tgtacatgta tccggaggcc     420

gacatcccgg tatgtcagct ctccgttcag ccgaaaatgg acggaacata ccactacaac     480

ttgggacgtg cgttagcgcc tctaagagac gaaggtatac tcatcattgg ttccggaagt     540

gcaacacacc ccttagacga aacgcctcat tatcatggtg gtgttgctcc ttgggctgcc     600

gagtttgatt cttggctcga cactgctctc actaatggaa ggattgaaga agtgaataca     660

tatgaaacca aagctccaaa ctgggaatta gcacatccgt tcccggagca tttttatcca     720

ttgcacgtcg tcgttggggc ggctggtgaa aagtggaagg ctgagcttat tcatactagt     780

tgggaccatg gtaccctatg tcatggcgct tacaagttca cttccagtta a              831

<210>  39
<211>  270
<212>  PRT
<213>  Chenopodium quinoa

<400>  39

Met Gly Gly Glu Asp Lys Asn Ile Ile Asn Glu Thr Phe Phe Ile Thr
1               5                   10                  15

His Gly Asn Pro Ile Leu Thr Val Glu Asp Thr His Pro Leu Arg Pro
            20                  25                  30

Phe Phe Glu Thr Trp Thr Glu Lys Ile Phe Ser Lys Lys Pro Lys Ala
        35                  40                  45

Ile Leu Val Ile Ser Gly His Trp Glu Thr Asp His Pro Ala Val Asn
    50                  55                  60

Ala Val His Ile Asn Asp Thr Ile Tyr Asp Phe Asp Asp Tyr Pro Glu
65                  70                  75                  80

Ala Met Tyr Lys Val Lys Tyr Pro Ala Pro Gly Ser Pro Asp Leu Ala
                85                  90                  95

Ser Arg Val Glu Glu Ile Leu Lys Lys Ser Gly Phe Asp Thr Val His
            100                 105                 110

Ile Asp Lys Lys Arg Gly Leu Asp His Gly Ala Trp Val Pro Leu Met
        115                 120                 125

Tyr Met Tyr Pro Glu Ala Asp Ile Pro Val Cys Gln Leu Ser Val Gln
    130                 135                 140

Pro Lys Met Asp Gly Thr Tyr His Tyr Asn Leu Gly Arg Ala Leu Ala
145                 150                 155                 160

Pro Leu Lys Asp Glu Gly Val Leu Ile Ile Gly Ser Gly Ser Ala Thr
                165                 170                 175

His Pro Leu Asp Glu Thr Pro His Tyr His Gly Gly Val Ala Pro Trp
            180                 185                 190

Ala Ala Asp Phe Asp Ser Trp Leu Asp Val Ala Leu Thr Lys Gly Arg
        195                 200                 205

Phe Glu Glu Val Asn Thr Tyr Glu Thr Lys Ala Pro Asn Trp Glu Leu
    210                 215                 220

Ala His Pro Phe Pro Glu His Phe Tyr Pro Leu His Val Val Val Gly
225                 230                 235                 240

Ala Ala Gly Glu Lys Trp Lys Ala Glu Leu Ile His Thr Ser Trp Asp
                245                 250                 255

His Gly Thr Leu Cys His Ala Ser Tyr Lys Phe Thr Ser Thr
            260                 265                 270

<210>  40
<211>  813
<212>  DNA
<213>  Chenopodium quinoa

<400>  40
atgggtgggg aagataagaa tataatcaat gaaaccttct tcataaccca tggaaaccca      60

attttaacag tagaagacac gcatccctta aggccattct ttgaaacttg gactgaaaaa     120

atattttcga agaagccgaa ggcgatactt gtgatctccg gtcactggga aactgatcat     180

cctgctgtta atgctgttca tatcaatgat actatctacg attttgatga ttatcctgaa     240

gccatgtaca aagtgaagta tccagctcca gggtcaccag acttggctag ccgagtagaa     300

gaaattctga aaaagtccgg gttcgacacg gtgcacattg acaaaaaacg tgggcttgat     360

catggtgcat gggtgcctct tatgtacatg tatccggagg ctgacattcc ggtatgtcag     420

ctctccgttc agccgaaaat ggacggaacg taccactaca acttggggcg ggcgttggcg     480

ccactgaaag acgaaggtgt tctcattatt ggctccggaa gtgcaacaca ccctttggac     540

gaaacccctc attatcatgg tggtgttgct ccttgggctg ctgactttga ctcgtggctt     600

gacgttgctc tcactaaagg aaggtttgaa gaagtgaata catatgaaac caaagcacca     660

aactgggaat tggcacatcc attcccggag catttttatc cattgcacgt cgtcgtcggg     720

gcggctggtg aaaagtggaa ggctgagctt attcatacta gttgggacca tggtacctta     780

tgtcatgcct cctacaagtt tacttccacc taa                                  813

<210>  41
<211>  270
<212>  PRT
<213>  Chenopodium quinoa

<400>  41

Met Gly Gly Glu Asp Asn Asn Met Ile Lys Glu Thr Phe Phe Ile Thr
1               5                   10                  15

His Gly Asn Pro Ile Leu Thr Val Val Asp Thr His Pro Leu Arg Pro
            20                  25                  30

Phe Phe Glu Thr Trp Ser Glu Lys Ile Phe Leu Lys Lys Pro Lys Ala
        35                  40                  45

Ile Leu Val Ile Ser Gly His Trp Glu Thr Asn His Pro Ala Val Asn
    50                  55                  60

Ala Val His Ile Asn Asp Val Ile Tyr Asp Phe Asp Asp Tyr Pro Glu
65                  70                  75                  80

Ala Met Tyr Gln Leu Lys Tyr Pro Ala Pro Gly Ser Pro Glu Leu Ala
                85                  90                  95

Ser Ser Val Glu Glu Asn Leu Lys Lys Ser Gly Phe Asp Thr Val His
            100                 105                 110

Ile Asp Lys Lys Arg Gly Leu Asp His Gly Ala Trp Val Pro Leu Met
        115                 120                 125

Tyr Met Tyr Pro Glu Ala Asp Ile Pro Val Cys Gln Leu Ser Val Gln
    130                 135                 140

Pro Lys Leu Asp Gly Thr Tyr His Tyr Asn Leu Gly Arg Ala Leu Ala
145                 150                 155                 160

Pro Leu Lys Asp Glu Gly Val Leu Ile Ile Gly Ser Gly Ser Ala Thr
                165                 170                 175

His Pro Leu Asp Glu Thr Pro His Tyr His Gly Gly Val Ala Pro Trp
            180                 185                 190

Ala Ala Asp Phe Asp Ser Trp Leu Asp Val Ala Leu Thr Lys Gly Arg
        195                 200                 205

Phe Glu Glu Val Asn Thr Tyr Glu Thr Lys Ala Pro Asn Trp Glu Leu
    210                 215                 220

Ala His Pro Phe Pro Glu Asn Phe Tyr Pro Leu His Val Val Ile Gly
225                 230                 235                 240

Ala Ala Gly Glu Met Trp Lys Ala Glu Leu Ile His Asn Ser Trp Asp
                245                 250                 255

His Gly Thr Leu Cys His Gly Ser Tyr Lys Phe Thr Ser Thr
            260                 265                 270

<210>  42
<211>  813
<212>  DNA
<213>  Chenopodium quinoa

<400>  42
atgggaggag aagataacaa tatgatcaaa gaaaccttct tcataaccca tggaaacccg      60

attttgaccg tggtagacac tcatccctta aggccattct ttgaaacatg gagtgaaaaa     120

atatttttga aaaagccgaa ggcgatactt gtgatctctg gtcactggga aactaatcat     180

cctgctgtta atgctgttca tatcaatgat gttatctatg atttcgatga ctatcctgaa 240

gccatgtacc agttgaaata tccagctcca gggtcaccag agttggctag cagtgtagaa 300

gaaaatctga aaaaatccgg gttcgacacg gtgcacattg acaaaaaacg tgggcttgat 360

catggtgcat gggtgcctct tatgtacatg tatccggagg ctgacattcc ggtatgtcag 420

ctctccgttc agccaaaatt ggacggaacg taccactaca acttgggacg ggcgttggcg 480

ccgctgaaag acgaaggtgt actcatcatt ggttccggaa gcgcaacaca ccctttggac 540

gaaacccctc attatcatgg tggtgttgct ccttgggctg ctgactttga ttcttggctt 600

gacgtagctc tcactaaagg aaggtttgaa gaagtgaata catatgaaac caaagcacca 660

aactgggaat tggcacatcc attcccggag aatttttatc cattgcatgt tgtcattggg 720

gcggctggtg aaatgtggaa ggctgagctt attcataata gttgggacca tggtacctta 780

tgtcatggct cctacaagtt tacttccacc taa 813

<210> 43
<211> 272
<212> PRT
<213> Chenopodium quinoa

<400> 43

Met Lys Met Ser Gly Glu Asp Asn Ser Met Ile Lys Glu Thr Phe Phe
1               5                   10                  15

Ile Thr His Gly Asn Pro Ile Leu Thr Val Glu Asp Thr His Pro Leu
            20                  25                  30

Arg Pro Phe Phe Glu Thr Trp Arg Glu Lys Ile Phe Thr Met Lys Pro
        35                  40                  45

Lys Ala Ile Leu Val Ile Ser Gly His Trp Glu Thr Asp His Pro Ala
    50                  55                  60

Val Asn Ala Val His Ile Asn Asp Thr Ile Tyr Asp Phe Asp Asp Tyr
65                  70                  75                  80

Pro Gln Ala Met Tyr Lys Leu Lys Tyr Pro Ala Pro Gly Ser Pro Asp
                85                  90                  95

Leu Ala Ser Arg Val Glu Glu Ile Met Lys Lys Ser Gly Phe Asp Thr
            100                 105                 110

Val His Ile Asp Lys Lys Arg Gly Leu Asp His Gly Ala Trp Val Pro
        115                 120                 125

Leu Met Tyr Met Tyr Pro Glu Ala Asp Ile Pro Val Cys Gln Leu Ser
    130                 135                 140

Val Gln Pro Lys Leu Asp Gly Thr Tyr His Tyr Asn Leu Gly Arg Ala
145                 150                 155                 160

Leu Ala Pro Leu Arg Asp Glu Gly Val Leu Ile Ile Gly Ser Gly Ser
                165                 170                 175

Ala Thr His Pro Leu Asp Glu Thr Pro His Tyr His Gly Gly Val Ala
            180                 185                 190

Pro Trp Ala Ala Asp Phe Asp Ser Trp Leu Asp Val Ala Leu Thr Lys
        195                 200                 205

Gly Arg Phe Glu Glu Val Asn Thr Tyr Glu Thr Lys Ala Pro Asn Trp
    210                 215                 220

Glu Leu Ala His Pro Phe Pro Glu His Phe Tyr Pro Leu His Val Val
225                 230                 235                 240

Val Gly Ala Ala Gly Glu Lys Trp Lys Ala Glu Leu Ile His Thr Ser
                245                 250                 255

Trp Asp His Gly Thr Leu Cys His Gly Ser Tyr Lys Phe Thr Ser Thr
            260                 265                 270

<210>  44
<211>  819
<212>  DNA
<213>  Chenopodium quinoa

<400>  44
atgaaaatga gtggagaaga taatagcatg atcaaagaaa ctttcttcat aacccatgga      60

aacccgattt tgaccgtaga agacacacat cccttaaggc cattctttga aacatggaga     120

gaaaaaatct ttacgatgaa gccgaaggcg atacttgtga tctctggtca ctgggaaact     180

gatcatcctg ctgttaatgc tgttcatatc aatgatacta tctacgattt cgatgactat     240

cctcaagcca tgtacaagtt gaagtatcca gctccaggat caccagactt ggctagcaga     300

gtagaagaaa ttatgaaaaa atccgggttc gacacagtgc acattgacaa aaaacgtggg     360

cttgatcatg gtgcatgggt gcctcttatg tacatgtatc cggaggctga cataccggta     420

tgtcagctct ccgtccagcc gaagttggac ggaacgtacc actacaactt gggacgagca     480

ttagcgcctc taagagacga aggtgtactc attattggtt ccggaagtgc aacacaccct     540

ttggacgaaa cccctcatta tcatggtggt gttgctcctt gggctgctga ctttgactca     600

tggcttgacg tagctctcac taaaggaaga tttgaagaag tgaatacata tgaaaccaaa     660

gcaccaaact gggaattggc acatccattc ccggagcatt tttatccatt gcatgtcgtg 720

gttggggcgg ctggtgaaaa gtggaaggct gagcttattc atactagttg ggaccatggt 780

accttatgtc atggctccta caagtttact tccacctaa 819

<210> 45
<211> 276
<212> PRT
<213> Chenopodium quinoa

<400> 45

Met Lys Met Ile Ile Glu Glu Glu Lys Asn Asn Glu Met Asn Phe Asn
1               5                   10                  15

Glu Thr Phe Phe Ile Thr His Gly Asn Pro Ile Leu Thr Val Glu Asp
            20                  25                  30

Thr His Pro Leu Arg Pro Phe Phe Glu Thr Trp Lys Glu Lys Val Phe
        35                  40                  45

Ser Lys Lys Pro Lys Ala Ile Leu Val Ile Ser Gly His Trp Glu Thr
    50                  55                  60

Asp His Pro Ala Val Asn Ala Val His Val Asn Asp Thr Ile Cys Asp
65                  70                  75                  80

Phe Asp Asp Tyr Pro Gln Ala Met Tyr Lys Phe Lys Tyr Pro Ala Pro
                85                  90                  95

Gly Tyr Leu Glu Leu Ala Lys Arg Val Gln Gln Leu Leu Asn Lys Ser
            100                 105                 110

Lys Phe Glu Thr Val His Ile Asp Gln Lys Arg Gly Leu Asp His Gly
        115                 120                 125

Ala Trp Val Pro Leu Met Tyr Met Tyr Pro Glu Ala Asp Ile Pro Val
    130                 135                 140

Cys Gln Leu Ser Val Gln Pro Lys Leu Asp Gly Thr Tyr His Tyr Asn
145                 150                 155                 160

Leu Gly Arg Ala Leu Ala Pro Leu Arg Asp Glu Ser Val Leu Ile Ile
                165                 170                 175

Gly Ser Gly Ser Ala Thr His Pro Leu Asp Glu Thr Pro His Tyr His
            180                 185                 190

Gly Gly Val Ala Pro Trp Ala Ala Glu Phe Asp Ser Trp Leu Asp Thr
        195                 200                 205

Ala Leu Thr Asn Gly Arg Ile Glu Glu Val Asn Thr Tyr Glu Thr Lys
    210                 215                 220

Ala Pro Asn Trp Glu Leu Ala His Pro Phe Pro Glu His Phe Tyr Pro
225                 230                 235                 240

Leu His Val Val Val Gly Ala Ala Gly Glu Lys Trp Lys Ala Glu Leu
                245                 250                 255

Ile His Thr Ser Trp Asp His Gly Thr Leu Cys His Gly Ala Tyr Lys
            260                 265                 270

Phe Thr Ser Ser
        275

<210>  46
<211>  831
<212>  DNA
<213>  Chenopodium quinoa

<400>  46
atgaaaatga ttattgaaga ggaaaaaaat aacgaaatga atttcaatga aaccttcttc      60

ataacccatg gaaacccgat tttgacagta gaagacacac atcccttaag gccattcttt     120

gaaacatgga aagaaaaagt attttcgaag aagccgaagg cgatacttgt gatctctggt     180

cactgggaaa ctgatcatcc tgctgttaat gctgttcatg tcaatgatac tatctgcgat     240

tttgatgact atcctcaagc catgtacaag tttaagtatc cggctcccgg gtatctagaa     300

ttggcaaaaa gggtgcagca actactcaat aaatccaagt tcgaaaccgt gcacattgac     360

caaaaacgtg ggcttgatca tggtgcatgg gtgcctctta tgtacatgta tccggaggcc     420

gacatcccgg tatgtcagct ctccgttcag ccaaaactgg acggaacata ccactacaac     480

ttgggacgag cattagcgcc tctaagagac gaaagtgtac tcatcattgg ttccggaagt     540

gcaacacacc ccttagacga aacccctcat tatcatggtg gtgttgctcc ttgggctgcc     600

gagtttgatt cttggcttga cactgctctc actaatggaa ggattgaaga agtgaataca     660

tatgaaacca aagctccaaa ctgggaatta gcacatccat tcccagaaca tttttatcca     720

ttgcacgttg tcgtcggggc agctggtgaa aagtggaagg ctgagcttat tcataccagt     780

tgggaccatg gtaccctatg tcatggcgca tacaagttca cttccagtta a              831

<210>  47
<211>  267
<212>  PRT
<213>  Mirabilis jalapa

<400>  47

Met Lys Gly Thr Tyr Tyr Ile Asn His Gly Asp Pro Leu Met Tyr Leu
1               5                   10                  15

Lys Lys His Ile Lys Leu Arg Gln Phe Leu Glu Gly Trp Gln Glu Asn
            20                  25                  30

Val Val Ile Glu Lys Pro Lys Ser Ile Leu Ile Ile Ser Ala His Trp
        35                  40                  45

Asp Thr Asn Val Pro Thr Val Asn Phe Val Glu His Cys Asp Thr Ile
    50                  55                  60

His Asp Phe Asp Asp Tyr Pro Asp Pro Leu Tyr Gln Ile Gln Tyr Arg
65                  70                  75                  80

Ala Pro Gly Ala Pro Asn Leu Ala Lys Lys Val Glu Glu Leu Leu Lys
                85                  90                  95

Glu Ser Gly Met Glu Cys Glu Ile Asp Thr Lys Arg Gly Leu Asp His
            100                 105                 110

Ala Ala Trp Phe Pro Leu Met Phe Met Tyr Pro Glu Ala Asn Ile Pro
        115                 120                 125

Ile Cys Glu Leu Ser Val Gln Pro Ser Lys Asp Gly Ile His His Tyr
    130                 135                 140

Asn Val Gly Lys Ala Leu Ser Pro Leu Leu Gln Gln Gly Val Leu Ile
145                 150                 155                 160

Ile Gly Ser Gly Gly Thr Val His Pro Ser Asp Asp Thr Pro His Cys
                165                 170                 175

Pro Asn Gly Val Ala Pro Trp Ala Ile Glu Phe Asp Asn Trp Leu Glu
            180                 185                 190

Asp Ala Leu Leu Ser Gly Arg Tyr Glu Asp Val Asn Asn Phe Lys Lys
        195                 200                 205

Leu Ala Pro Asn Trp Glu Ile Ser His Pro Gly Gln Glu His Leu Tyr
    210                 215                 220

Pro Leu His Val Ala Leu Gly Ala Ala Gly Lys Asn Pro Lys Thr Gln
225                 230                 235                 240

Leu Ile His Arg Ser Trp Ala Ala Asn Gly Val Phe Gly Tyr Ser Thr
                245                 250                 255

Tyr Asn Phe Thr Pro Thr Thr Gln Lys Thr Asp
            260                 265

<210>  48
<211>  804
<212>  DNA
<213>  Mirabilis jalapa

<400>  48
atgaaaggaa catactatat aaatcatggt gatccactaa tgtacttaaa aaaacatata      60

aaactaaggc aattcttaga aggatggcaa gaaaatgttg ttattgaaaa accaaagagt     120

atacttatca tttctgctca ttgggatact aatgtaccta ctgtcaactt tgttgaacat     180

tgtgatacta ttcatgattt tgatgactat cctgatcctt tgtaccagat acaatatcga     240

gcaccgggag caccaaattt agcaaaaaag gtggaagagt tactaaaaga gtcaggaatg     300

gaatgtgaga tagatacaaa gagaggactc gatcatgcag catggtttcc actaatgttt     360

atgtatcctg aagctaatat tcctatttgt gagctctcag tccaaccaag caaagatggg     420

atccaccact ataatgttgg gaaagctctt tctcctcttt tgcaacaagg tgttctcatc     480

attggctctg gtggtactgt tcatccttct gatgatactc ctcattgtcc caatggtgtt     540

gctccttggg ctattgagtt tgataactgg cttgaagatg ctcttcttag tggaaggtat     600

gaagatgtga acaacttcaa gaaattggca ccaaattggg agatatctca tccaggacaa     660

gaacatttgt accctttgca tgtggcatta ggggctgctg gcaaaaatcc aaagacacaa     720

cttattcatc gaagctgggc tgccaatggt gtctttggat attccaccta caacttcact     780

cccaccactc aaaaaactga ttaa                                            804

<210>  49
<211>  48
<212>  PRT
<213>  Chenopodium quinoa

<220>
<221>  MISC_FEATURE
<222>  (19)..(19)
<223>  Xaa stands for Asp or Glu

<220>
<221>  MISC_FEATURE
<222>  (26)..(26)
<223>  Xaa stands for Leu, Val or Thr

<220>
<221>  MISC_FEATURE
<222>  (30)..(30)
<223>  Xaa stands for Lys or Asn

<220>
<221>  MISC_FEATURE
<222>  (33)..(33)
<223>  Xaa stands for Phe or Ile

<400>  49

His Pro Leu Asp Glu Thr Pro His Tyr His Gly Gly Val Ala Pro Trp
1               5                   10                  15

Ala Ala Xaa Phe Asp Ser Trp Leu Asp Xaa Ala Leu Thr Xaa Gly Arg
            20                  25                  30

Xaa Glu Glu Val Asn Thr Tyr Glu Thr Lys Ala Pro Asn Trp Glu Leu
        35                  40                  45

<210>  50
<211>  1515
<212>  DNA
<213>  Chenopodium quinoa

<400>  50
atggaacata caacacttgc attgatactt tcaattttgt ttatttgctt tcatttagtc      60

cgatcatttg ttagtcactc aacaaagtcc aataaacttc cacccgggcc taaaagaatg     120

ccaatttttg gcaatatttt ggatcttggg gagaagcctc atcgatcatt tgcaaatctt     180

gctaagattc acggcccttt ggtgagtttg cagttaggaa gtatcacaac aattatagtg     240

tcttcagctg atgtagccaa agaaatgttt cttaaaaacg accagttact tgctaaccga     300

accattcctg attctgttag ggcaggaaac cacgacaagc tctccatgtc gtggttgccc     360

atatcagcta aatggaggaa tctcagaaag atctccgccg tgcaacttct ctctaatcaa     420

cgacttgatg ctagccaagc ccatagacaa gccaaggtcg aacaacttct tgcatacgtg     480

caagattgtt ctaagaaagg gcaacccgtt gatataggga gggccgcgtt tacaacttct     540

ctaaacttat tatcaaacac atttttctca acggaattgg caagccacga gtcgaataat     600

tcccaagagt ttaagcaact catgtggaac ataatggagg aaattggtag accaaattat     660

gccgattatt tccccattct tggctacgtt gatccgttcg gcataaggcg tcgtttggct     720

gcttactttg ataaactcat agctgttttt caagacataa tttgtgaaag gcaaaaaatt     780

cgatcaacca aagtttcaag tgaaaaacaa acgggtgata ttttagacac acttctcaac     840

ctttatgatg aaaatgagtt gagtatgggt gagattaatc atcttcttgt ggacatcttt     900

gatgccggaa cagatactac agctagcaca ttagagtggg caatggctga gcttgttaaa     960

aatcccggga tgatgataag agtacaaaat gaaatcgaac tagccatcgg caaaggttgc    1020

tccatggtcc aagaagctga tatctcaaaa ctaccttact tgcaagccat catcaaagaa    1080

acattgcgat tgcaccctcc aactgtattt ctcttacctc gaaaggcaga cattgatgta    1140

gagttatatg gttacgtcgt accaaaaaat gcacaagttc tagtcaatct ttgggccatt 1200

ggtcgcgatc caaaggtatg gaaaaatccc gaaatatttt cacctgaaag gtttttaggg 1260

tgtgacatcg attttaaagg acgagatttt gaactcttgc cctttggtgc cggaagaagg 1320

atatgccctg gattaacttt ggcttataga atgttgaact taatgatggc taattttgtt 1380

cattcctttg attggaagct tgaagatgga atgaatccaa aagatttgga catggatgag 1440

aaatttggta tcacattgca aaaggttaag cctctccaag ttattccagt acataggaaa 1500

cactgcatgt cataa 1515

<210> 51
<211> 504
<212> PRT
<213> Chenopodium quinoa

<400> 51

Met Glu His Thr Thr Leu Ala Leu Ile Leu Ser Ile Leu Phe Ile Cys
1               5                   10                  15

Phe His Leu Val Arg Ser Phe Val Ser His Ser Thr Lys Ser Asn Lys
            20                  25                  30

Leu Pro Pro Gly Pro Lys Arg Met Pro Ile Phe Gly Asn Ile Leu Asp
        35                  40                  45

Leu Gly Glu Lys Pro His Arg Ser Phe Ala Asn Leu Ala Lys Ile His
    50                  55                  60

Gly Pro Leu Val Ser Leu Gln Leu Gly Ser Ile Thr Thr Ile Ile Val
65                  70                  75                  80

Ser Ser Ala Asp Val Ala Lys Glu Met Phe Leu Lys Asn Asp Gln Leu
                85                  90                  95

Leu Ala Asn Arg Thr Ile Pro Asp Ser Val Arg Ala Gly Asn His Asp
            100                 105                 110

Lys Leu Ser Met Ser Trp Leu Pro Ile Ser Ala Lys Trp Arg Asn Leu
        115                 120                 125

Arg Lys Ile Ser Ala Val Gln Leu Leu Ser Asn Gln Arg Leu Asp Ala
    130                 135                 140

Ser Gln Ala His Arg Gln Ala Lys Val Glu Gln Leu Leu Ala Tyr Val
145                 150                 155                 160

Gln Asp Cys Ser Lys Lys Gly Gln Pro Val Asp Ile Gly Arg Ala Ala
                165                 170                 175

Phe Thr Thr Ser Leu Asn Leu Leu Ser Asn Thr Phe Phe Ser Thr Glu
            180                 185                 190

Leu Ala Ser His Glu Ser Asn Asn Ser Gln Glu Phe Lys Gln Leu Met
        195                 200                 205

Trp Asn Ile Met Glu Glu Ile Gly Arg Pro Asn Tyr Ala Asp Tyr Phe
    210                 215                 220

Pro Ile Leu Gly Tyr Val Asp Pro Phe Gly Ile Arg Arg Arg Leu Ala
225                 230                 235                 240

Ala Tyr Phe Asp Lys Leu Ile Ala Val Phe Gln Asp Ile Ile Cys Glu
                245                 250                 255

Arg Gln Lys Ile Arg Ser Thr Lys Val Ser Ser Glu Lys Gln Thr Gly
            260                 265                 270

Asp Ile Leu Asp Thr Leu Leu Asn Leu Tyr Asp Glu Asn Glu Leu Ser
        275                 280                 285

Met Gly Glu Ile Asn His Leu Leu Val Asp Ile Phe Asp Ala Gly Thr
    290                 295                 300

Asp Thr Thr Ala Ser Thr Leu Glu Trp Ala Met Ala Glu Leu Val Lys
305                 310                 315                 320

Asn Pro Gly Met Met Ile Arg Val Gln Asn Glu Ile Glu Leu Ala Ile
                325                 330                 335

Gly Lys Gly Cys Ser Met Val Gln Glu Ala Asp Ile Ser Lys Leu Pro
            340                 345                 350

Tyr Leu Gln Ala Ile Ile Lys Glu Thr Leu Arg Leu His Pro Pro Thr
        355                 360                 365

Val Phe Leu Leu Pro Arg Lys Ala Asp Ile Asp Val Glu Leu Tyr Gly
    370                 375                 380

Tyr Val Val Pro Lys Asn Ala Gln Val Leu Val Asn Leu Trp Ala Ile
385                 390                 395                 400

Gly Arg Asp Pro Lys Val Trp Lys Asn Pro Glu Ile Phe Ser Pro Glu
                405                 410                 415

Arg Phe Leu Gly Cys Asp Ile Asp Phe Lys Gly Arg Asp Phe Glu Leu
            420                 425                 430

Leu Pro Phe Gly Ala Gly Arg Arg Ile Cys Pro Gly Leu Thr Leu Ala
        435                 440                 445

Tyr Arg Met Leu Asn Leu Met Met Ala Asn Phe Val His Ser Phe Asp
    450                 455                 460

Trp Lys Leu Glu Asp Gly Met Asn Pro Lys Asp Leu Asp Met Asp Glu
465                 470                 475                 480

Lys Phe Gly Ile Thr Leu Gln Lys Val Lys Pro Leu Gln Val Ile Pro
                485                 490                 495

Val His Arg Lys His Cys Met Ser
            500

<210>  52
<211>  1518
<212>  DNA
<213>  Chenopodium quinoa

<400>  52
atggaacata caatacttgc attgatactt tcaattttgt ttatttgctt tcatttggtt      60

cgatcatttg ttagccactc tacaaagtcc aataaactcc cacccgggcc taaaagaatg     120

ccaatctttg gcaatatttt cgatcttggg gagaagcctc atcgatcatt tgcaaatctt     180

gctaagattc acgggccttt ggtgagctta cagttaggaa gtatcacaac tattgtagtg     240

tcttcagctg atgtagccaa agaaatgttt cttaaaaatg accaggcact tgctaatcga     300

accattcctg attctgttag ggcaggagac catgacaagc tctctatgtc gtggttgccc     360

atatcagcta aatggaggaa tcttagaaag atctccgccg tgcaacttct ctctaatcaa     420

cgacttgatg ctagccaagc ccatagacaa gccaaggtcg aacaacttct tgcatacgtg     480

caagattgtt ctaagaaagg ccaacccgtt gatataggga gggccgcgtt tacaacttct     540

ctaaacttgt tatcaaacac atttttttca acggaattgg caagccatga gtctaataat     600

tcacaagagt ttaagcaact catgtggaac ataatggagg aaattggtaa accaaattac     660

gccgattttt ttcccattct tggctacgtt gatccattcg gaataaggcg tcgtttggct     720

ggttactttg ataaactcat tgctgttttt caagacataa tttgtgaaag acaaaaaatt     780

cgatcaacca aagtttcaag tgaaaaacaa acgggtgata ttttagacac acttctcaac     840

ctctacgatg aaaatgagtt gagtatgggt gagattaatc atcttcttgt ggacatcttt     900

gatgccggaa cagatactac agctagcaca ttagagtggg caatggctga gcttgttaaa     960

aatcctggga tgatgattag agttcaaaat gaaatcgaac tagccattgg caaaggttgc      1020

tccatggtcc aagaatctga tatctcaaaa ctaccttact tgcaagccat cattaaagaa      1080

acattgcgat tgcaccctcc aaccgtattt ctcttacctc gaaaagcaga cgttgatgta      1140

gagttatatg gttacgtcgt acccaaaaat gcacaagttc tagtaaatct ttgggccatt      1200

ggtcgcgatc caaaggtatg gaaaaatcca gaaatatttt cacctgaaag gtttataggg      1260

tgtgacattg attttaaggg acgagatttt gaactcttgc cctttggtgc cggaagaagg      1320

atatgccctg gattaacttt ggcttataga atgttgaact taatgatggc ttattttgtt      1380

cattcctttg attggaaact tgaagatgga atgaatccaa aagacttgga catggatgag      1440

aaatttggta tcactttgca aaaggttaaa cctctacaag ttattccagt acaaaggaaa      1500

caatattgca tgtcataa                                                    1518

<210>  53
<211>  505
<212>  PRT
<213>  Chenopodium quinoa

<400>  53

Met Glu His Thr Ile Leu Ala Leu Ile Leu Ser Ile Leu Phe Ile Cys
1               5                   10                  15

Phe His Leu Val Arg Ser Phe Val Ser His Ser Thr Lys Ser Asn Lys
            20                  25                  30

Leu Pro Pro Gly Pro Lys Arg Met Pro Ile Phe Gly Asn Ile Phe Asp
        35                  40                  45

Leu Gly Glu Lys Pro His Arg Ser Phe Ala Asn Leu Ala Lys Ile His
    50                  55                  60

Gly Pro Leu Val Ser Leu Gln Leu Gly Ser Ile Thr Thr Ile Val Val
65                  70                  75                  80

Ser Ser Ala Asp Val Ala Lys Glu Met Phe Leu Lys Asn Asp Gln Ala
                85                  90                  95

Leu Ala Asn Arg Thr Ile Pro Asp Ser Val Arg Ala Gly Asp His Asp
            100                 105                 110

Lys Leu Ser Met Ser Trp Leu Pro Ile Ser Ala Lys Trp Arg Asn Leu
        115                 120                 125

Arg Lys Ile Ser Ala Val Gln Leu Leu Ser Asn Gln Arg Leu Asp Ala
    130                 135                 140

Ser Gln Ala His Arg Gln Ala Lys Val Glu Gln Leu Leu Ala Tyr Val
145                 150                 155                 160

Gln Asp Cys Ser Lys Lys Gly Gln Pro Val Asp Ile Gly Arg Ala Ala
                165                 170                 175

Phe Thr Thr Ser Leu Asn Leu Leu Ser Asn Thr Phe Phe Ser Thr Glu
            180                 185                 190

Leu Ala Ser His Glu Ser Asn Asn Ser Gln Glu Phe Lys Gln Leu Met
        195                 200                 205

Trp Asn Ile Met Glu Glu Ile Gly Lys Pro Asn Tyr Ala Asp Phe Phe
    210                 215                 220

Pro Ile Leu Gly Tyr Val Asp Pro Phe Gly Ile Arg Arg Arg Leu Ala
225                 230                 235                 240

Gly Tyr Phe Asp Lys Leu Ile Ala Val Phe Gln Asp Ile Ile Cys Glu
                245                 250                 255

Arg Gln Lys Ile Arg Ser Thr Lys Val Ser Ser Glu Lys Gln Thr Gly
            260                 265                 270

Asp Ile Leu Asp Thr Leu Leu Asn Leu Tyr Asp Glu Asn Glu Leu Ser
        275                 280                 285

Met Gly Glu Ile Asn His Leu Leu Val Asp Ile Phe Asp Ala Gly Thr
    290                 295                 300

Asp Thr Thr Ala Ser Thr Leu Glu Trp Ala Met Ala Glu Leu Val Lys
305                 310                 315                 320

Asn Pro Gly Met Met Ile Arg Val Gln Asn Glu Ile Glu Leu Ala Ile
                325                 330                 335

Gly Lys Gly Cys Ser Met Val Gln Glu Ser Asp Ile Ser Lys Leu Pro
            340                 345                 350

Tyr Leu Gln Ala Ile Ile Lys Glu Thr Leu Arg Leu His Pro Pro Thr
        355                 360                 365

Val Phe Leu Leu Pro Arg Lys Ala Asp Val Asp Val Glu Leu Tyr Gly
    370                 375                 380

Tyr Val Val Pro Lys Asn Ala Gln Val Leu Val Asn Leu Trp Ala Ile
385                 390                 395                 400

Gly Arg Asp Pro Lys Val Trp Lys Asn Pro Glu Ile Phe Ser Pro Glu
                405                 410                 415

Arg Phe Ile Gly Cys Asp Ile Asp Phe Lys Gly Arg Asp Phe Glu Leu
            420                 425                 430

Leu Pro Phe Gly Ala Gly Arg Arg Ile Cys Pro Gly Leu Thr Leu Ala
        435                 440                 445

Tyr Arg Met Leu Asn Leu Met Met Ala Tyr Phe Val His Ser Phe Asp
    450                 455                 460

Trp Lys Leu Glu Asp Gly Met Asn Pro Lys Asp Leu Asp Met Asp Glu
465                 470                 475                 480

Lys Phe Gly Ile Thr Leu Gln Lys Val Lys Pro Leu Gln Val Ile Pro
                485                 490                 495

Val Gln Arg Lys Gln Tyr Cys Met Ser
            500                 505

<210>  54
<211>  1503
<212>  DNA
<213>  Chenopodium quinoa

<400>  54
atggatagta caacacttgt tatggttgtt atttctattt tatttgtatt tttatatcat      60

gtcaaatcct tttttatccg ttatttttct aacaggctcc caccagggcc aaaaccaaag     120

ccaatttttg gcaacatttt cgatcttgga gaaaagcctc atcgatcctt tgccaaccta     180

gcaaaaattt ttggcccatt gattagtcta aagctaggca atgtcacaac agttgttgta     240

tcatcctctt atgtggcaga agagatgttt ctaaaaaatg accagtcatt tgcaaatcga     300

accattcctg attctgttcg agctggcaac catgacaaac tctccatgtc atggttgcct     360

atatctccac aatggcggaa tcttaggaag atctctgctg tccaattact ctcaacacaa     420

cgccttgatg ctagccaagc cctaaggcaa gccaaggtat cacaacttca tgcatacgtg     480

caagattgct ctaaaaaatt gcaaccggtt aacataggaa gggcagcatt cactacttcc     540

ctaaacttat tatcaaacac atttttctca atcgaactag ctagccacga gtctagtact     600

tcccaagagt ttaagcaact catgtggaac ataatggagg aaattggaag acctaattat     660

gcagattatt tccccattct tggctacatt gatccatttg gcataagacg tcgtttggct     720

tcttactttg atgaactcat tgttgttttt caaaacatta tttgcgaaag gcaaaacatt     780

cgatcaagtg acgattcaag tgcaaaacat acaaatgatg ttctagacac acttctcaac     840

ctctatgaca agaatgagtt gagtatggat gaaattaatc atcttcttgt tgatattttt 900

aacgcgggaa cagacactac tgcaagtact ttagaatgga caatgacaga gctaattaaa 960

aatccaaaaa gtatgatcat atgtcaaaat gaaattgaac aagctcttgg aaaaggtagt 1020

ttgagcattc aagaatctga tatttcaaaa ttgccttacc tacaagccat catgaaagaa 1080

acgttacgtt tgcaccctcc tacagtgttt ctcctcccac gtaaagcaga cagtgatgta 1140

gagttatgtg gctacgtagt tccaaaacat gcacaagttc ttgttaatct atgggctatt 1200

ggtcgtgatc caaaggtgtg ggaaaaccct gaagtatttt caccggagag gttcttagat 1260

tgtgagatcg atttgaaagg acaagatttt gaacttttac cctttggagc tggtagaaga 1320

atatgccctg gacttaattt ggcatataga atgttgaact tgatgttggc tactcttctt 1380

cacaactaca attggaaact tgaagatggt atgactctta atgatttaga catggatgaa 1440

aagtttggaa ttacattgca gaaggttaag cctcttcaag ttgttccaat cccaaggtat 1500

taa 1503

<210> 55
<211> 500
<212> PRT
<213> Chenopodium quinoa

<400> 55

Met Asp Ser Thr Thr Leu Val Met Val Val Ile Ser Ile Leu Phe Val
1               5                   10                  15

Phe Leu Tyr His Val Lys Ser Phe Phe Ile Arg Tyr Phe Ser Asn Arg
            20                  25                  30

Leu Pro Pro Gly Pro Lys Pro Lys Pro Ile Phe Gly Asn Ile Phe Asp
        35                  40                  45

Leu Gly Glu Lys Pro His Arg Ser Phe Ala Asn Leu Ala Lys Ile Phe
    50                  55                  60

Gly Pro Leu Ile Ser Leu Lys Leu Gly Asn Val Thr Thr Val Val Val
65                  70                  75                  80

Ser Ser Ser Tyr Val Ala Glu Glu Met Phe Leu Lys Asn Asp Gln Ser
                85                  90                  95

Phe Ala Asn Arg Thr Ile Pro Asp Ser Val Arg Ala Gly Asn His Asp
            100                 105                 110

Lys Leu Ser Met Ser Trp Leu Pro Ile Ser Pro Gln Trp Arg Asn Leu
        115                 120                 125

Arg Lys Ile Ser Ala Val Gln Leu Leu Ser Thr Gln Arg Leu Asp Ala
    130                 135                 140

Ser Gln Ala Leu Arg Gln Ala Lys Val Ser Gln Leu His Ala Tyr Val
145                 150                 155                 160

Gln Asp Cys Ser Lys Lys Leu Gln Pro Val Asn Ile Gly Arg Ala Ala
                165                 170                 175

Phe Thr Thr Ser Leu Asn Leu Leu Ser Asn Thr Phe Phe Ser Ile Glu
            180                 185                 190

Leu Ala Ser His Glu Ser Ser Thr Ser Gln Glu Phe Lys Gln Leu Met
        195                 200                 205

Trp Asn Ile Met Glu Glu Ile Gly Arg Pro Asn Tyr Ala Asp Tyr Phe
    210                 215                 220

Pro Ile Leu Gly Tyr Ile Asp Pro Phe Gly Ile Arg Arg Arg Leu Ala
225                 230                 235                 240

Ser Tyr Phe Asp Glu Leu Ile Val Val Phe Gln Asn Ile Ile Cys Glu
                245                 250                 255

Arg Gln Asn Ile Arg Ser Ser Asp Asp Ser Ser Ala Lys His Thr Asn
            260                 265                 270

Asp Val Leu Asp Thr Leu Leu Asn Leu Tyr Asp Lys Asn Glu Leu Ser
        275                 280                 285

Met Asp Glu Ile Asn His Leu Leu Val Asp Ile Phe Asn Ala Gly Thr
    290                 295                 300

Asp Thr Thr Ala Ser Thr Leu Glu Trp Thr Met Thr Glu Leu Ile Lys
305                 310                 315                 320

Asn Pro Lys Ser Met Ile Ile Cys Gln Asn Glu Ile Glu Gln Ala Leu
                325                 330                 335

Gly Lys Gly Ser Leu Ser Ile Gln Glu Ser Asp Ile Ser Lys Leu Pro
            340                 345                 350

Tyr Leu Gln Ala Ile Met Lys Glu Thr Leu Arg Leu His Pro Pro Thr
        355                 360                 365

Val Phe Leu Leu Pro Arg Lys Ala Asp Ser Asp Val Glu Leu Cys Gly

370                 375                 380

Tyr Val Val Pro Lys His Ala Gln Val Leu Val Asn Leu Trp Ala Ile
385                 390                 395                 400

Gly Arg Asp Pro Lys Val Trp Glu Asn Pro Glu Val Phe Ser Pro Glu
                405                 410                 415

Arg Phe Leu Asp Cys Glu Ile Asp Leu Lys Gly Gln Asp Phe Glu Leu
            420                 425                 430

Leu Pro Phe Gly Ala Gly Arg Arg Ile Cys Pro Gly Leu Asn Leu Ala
        435                 440                 445

Tyr Arg Met Leu Asn Leu Met Leu Ala Thr Leu Leu His Asn Tyr Asn
    450                 455                 460

Trp Lys Leu Glu Asp Gly Met Thr Leu Asn Asp Leu Asp Met Asp Glu
465                 470                 475                 480

Lys Phe Gly Ile Thr Leu Gln Lys Val Lys Pro Leu Gln Val Val Pro
                485                 490                 495

Ile Pro Arg Tyr
            500

<210>  56
<211>  1431
<212>  DNA
<213>  Chenopodium quinoa

<400>  56
atgagcaaaa gggttttatc cggtgatgtt tggtggtggt tgacggtggt tttggtggtt      60

gttgttggtg gttatgatgg cgtaaactcg tacccgaaag aggaccttgt tgtaaattta     120

cctggacaac caaaggttga tttcaggcaa tttgctgggt acattgatgt tgatgttaaa     180

gcaggaagaa gcttgtttta ttattttgtt gaagctgaaa ataatactca acatactcct     240

cttactcttt ggcttaatgg aggacctggt tgttcatcaa tgggaggtgg tgcatttaca     300

gaattgggac cattttttcc aaagggtgat ggaagaggtt taagggttaa ttcaaagtca     360

tggaatagag cgtcgaatct actctttgtt gaatcgcctg ctggagtggg atggtcgtat     420

tcaaacaaat cgtctgatta taataccggg gatgtagcct ctgctaagga tatgctcaca     480

tttctggtga attggatggt gaagtttcct gaattcaaat ccagggatct gtatcttact     540

ggggagagtt atgcagggca ctacatacca caactagctg ttgctgtgtt ggatcataat     600

gctcaatcta aggcctcaaa gcttaactta aaaggagttg ctattggaaa tccacttctc     660

aaactcgata gagatgttcc agcaacatat gagtttttct ggtctcatgg aatgatatct 720

gatgaaattg gccttgccat catgaatcaa tgtgattttg aagattatac ctttccaaat 780

cctcataacg aaactgatgc ttgcaacaat gcaatatcaa aagcaaatac cattgttggt 840

gattatgtca atgcatatga tgtcatcctt gatgtctgtt atccatctat cgtagagcaa 900

gaactgcggt tgaaaaaaat ggccactaaa atgagtgttg gagttgatgt ttgtattact 960

tatgaaagaa gattctattt caatcttcct gaagtacaga gagctctcca tgcaaatcgg 1020

acaaatctac cttatgaatg gtctatgtgc agtagcattc tgaattactc tgacactgat 1080

ggtagcttga atatgcttcc tctgcttaaa agaatactgt tgcacgacat tcgtgtttgg 1140

attttcagtg gagaccaaga ttctgttgtg cccctgcttg gctcccgtac ccttgttcgt 1200

gagcttgctc atgacctcaa atacaacgtc accgtcccat atgcaaactg gtttcacaaa 1260

ggacaggtag gaggttggac aaccgaatat ggaaacttac tcacatttgc gactgtgagg 1320

ggcgctgcac atatggtacc ttatgcacaa ccatcaaggg ctctgcatct gttcagttca 1380

tttattcgtg atcgaagact gccaacaaca actaaagaat ccgttgaatg a 1431

<210> 57
<211> 476
<212> PRT
<213> Chenopodium quinoa

<400> 57

Met Ser Lys Arg Val Leu Ser Gly Asp Val Trp Trp Trp Leu Thr Val
1               5                   10                  15

Val Leu Val Val Val Val Gly Gly Tyr Asp Gly Val Asn Ser Tyr Pro
            20                  25                  30

Lys Glu Asp Leu Val Val Asn Leu Pro Gly Gln Pro Lys Val Asp Phe
        35                  40                  45

Arg Gln Phe Ala Gly Tyr Ile Asp Val Asp Val Lys Ala Gly Arg Ser
    50                  55                  60

Leu Phe Tyr Tyr Phe Val Glu Ala Glu Asn Asn Thr Gln His Thr Pro
65                  70                  75                  80

Leu Thr Leu Trp Leu Asn Gly Gly Pro Gly Cys Ser Ser Met Gly Gly
                85                  90                  95

Gly Ala Phe Thr Glu Leu Gly Pro Phe Phe Pro Lys Gly Asp Gly Arg
            100                 105                 110

Gly Leu Arg Val Asn Ser Lys Ser Trp Asn Arg Ala Ser Asn Leu Leu

115                 120                 125

Phe Val Glu Ser Pro Ala Gly Val Gly Trp Ser Tyr Ser Asn Lys Ser
    130                 135                 140

Ser Asp Tyr Asn Thr Gly Asp Val Ala Ser Ala Lys Asp Met Leu Thr
145                 150                 155                 160

Phe Leu Val Asn Trp Met Val Lys Phe Pro Glu Phe Lys Ser Arg Asp
                165                 170                 175

Leu Tyr Leu Thr Gly Glu Ser Tyr Ala Gly His Tyr Ile Pro Gln Leu
            180                 185                 190

Ala Val Ala Val Leu Asp His Asn Ala Gln Ser Lys Ala Ser Lys Leu
        195                 200                 205

Asn Leu Lys Gly Val Ala Ile Gly Asn Pro Leu Leu Lys Leu Asp Arg
    210                 215                 220

Asp Val Pro Ala Thr Tyr Glu Phe Phe Trp Ser His Gly Met Ile Ser
225                 230                 235                 240

Asp Glu Ile Gly Leu Ala Ile Met Asn Gln Cys Asp Phe Glu Asp Tyr
                245                 250                 255

Thr Phe Pro Asn Pro His Asn Glu Thr Asp Ala Cys Asn Asn Ala Ile
            260                 265                 270

Ser Lys Ala Asn Thr Ile Val Gly Asp Tyr Val Asn Ala Tyr Asp Val
        275                 280                 285

Ile Leu Asp Val Cys Tyr Pro Ser Ile Val Glu Gln Glu Leu Arg Leu
    290                 295                 300

Lys Lys Met Ala Thr Lys Met Ser Val Gly Val Asp Val Cys Ile Thr
305                 310                 315                 320

Tyr Glu Arg Arg Phe Tyr Phe Asn Leu Pro Glu Val Gln Arg Ala Leu
                325                 330                 335

His Ala Asn Arg Thr Asn Leu Pro Tyr Glu Trp Ser Met Cys Ser Ser
            340                 345                 350

Ile Leu Asn Tyr Ser Asp Thr Asp Gly Ser Leu Asn Met Leu Pro Leu
        355                 360                 365

Leu Lys Arg Ile Leu Leu His Asp Ile Arg Val Trp Ile Phe Ser Gly
    370                 375                 380

Asp Gln Asp Ser Val Val Pro Leu Leu Gly Ser Arg Thr Leu Val Arg
385                 390                 395                 400

Glu Leu Ala His Asp Leu Lys Tyr Asn Val Thr Val Pro Tyr Ala Asn
                405                 410                 415

Trp Phe His Lys Gly Gln Val Gly Gly Trp Thr Thr Glu Tyr Gly Asn
            420                 425                 430

Leu Leu Thr Phe Ala Thr Val Arg Gly Ala Ala His Met Val Pro Tyr
        435                 440                 445

Ala Gln Pro Ser Arg Ala Leu His Leu Phe Ser Ser Phe Ile Arg Asp
    450                 455                 460

Arg Arg Leu Pro Thr Thr Thr Lys Glu Ser Val Glu
465                 470                 475

<210>  58
<211>  1431
<212>  DNA
<213>  Chenopodium quinoa

<400>  58
atgagcaaaa gggttttatc cggtggtttt tgttggtggt tgacggtggt tttggtggtt      60

gttgttggtg gttttgattg cgtaacggcg tacccgaaag aggatcttgt tgtaaattta     120

cctggacaac caaaggttga tttcaagcaa tttgctgggt acattgatgt tgatgttaaa     180

gcaggaagaa gcttgtttta ttattttgtt gaagctgaaa ataatactca acatactcct     240

cttagtcttt ggcttaatgg aggacctggt tgttcatcaa tgggaggtgg tgcatttaca     300

gaattgggac cattttttcc aaggggtgat ggaagaggtt taagggttaa ttcaaagtca     360

tggaatagag cgtcgaatct actctttgtt gaatcgcctg ctggtgtggg atggtcgtat     420

tcaaacacat cgtctgatta taataccggg gatgcaacct ctgctaagga tatgctcaaa     480

tttctggtga attggatggt caagtttcct gaatttaaat ccagggatct gtatcttact     540

ggggagagtt atgcagggca ctacatacca caactggctg ttgctatatt ggatcataat     600

gctcaatcaa aggcctccaa gctcaacttg aaaggagttg ctattggaaa tccacttctc     660

aaactcgata gagatgttcc tgcaacatac gagttttact ggtctcatgg aatgatctct     720

gatgaaattg gccttgccat catgaatcag tgtgattttg aagattatac ctttccaaat     780

cctcataacg aaactgatgt ttgcaacaat gctatatcaa aagcaaatac cattgttggc     840

gattatgtca acgagtatga tgttatcctt gatgtctgtt atccatctat tgtagagcaa     900

gaactgcggt tgaaaaaaat ggccactaaa atgagtgttg gagttgatgt ttgtatgact 960

tatgaaagac gattctattt caatcttcct gaagtacaga gagctctcca cgcaaatcgg 1020

actaatctac cttatgaatg gtctatgtgc agtagcattc tgaattacac tgacactgat 1080

ggtagcttga atatgcttcc tctgctgaaa aggatactgt tgcacaatat tcgtgtatgg 1140

atcttcagtg gagatcaaga ttctgttgtg ccactgcttg gctcccgtac ccttgttcgt 1200

gagcttgctc atgacctcaa atacaatatc accgtcccct atgcaaactg gtttcacaaa 1260

ggacaggtag gaggttggac tactgaatat ggaaacttac tcacatttgc gactgtgagg 1320

ggcgctgcac atatggtacc ttatgcacaa ccatcaaggg ctctgcacct gttcagttca 1380

tttatacatg atcgaaaact gccaacaaca actaaagaat ccatagaatg a 1431

<210> 59
<211> 476
<212> PRT
<213> Chenopodium quinoa

<400> 59

Met Ser Lys Arg Val Leu Ser Gly Gly Phe Cys Trp Trp Leu Thr Val
1               5                   10                  15

Val Leu Val Val Val Val Gly Gly Phe Asp Cys Val Thr Ala Tyr Pro
            20                  25                  30

Lys Glu Asp Leu Val Val Asn Leu Pro Gly Gln Pro Lys Val Asp Phe
        35                  40                  45

Lys Gln Phe Ala Gly Tyr Ile Asp Val Asp Val Lys Ala Gly Arg Ser
    50                  55                  60

Leu Phe Tyr Tyr Phe Val Glu Ala Glu Asn Asn Thr Gln His Thr Pro
65                  70                  75                  80

Leu Ser Leu Trp Leu Asn Gly Gly Pro Gly Cys Ser Ser Met Gly Gly
                85                  90                  95

Gly Ala Phe Thr Glu Leu Gly Pro Phe Phe Pro Arg Gly Asp Gly Arg
            100                 105                 110

Gly Leu Arg Val Asn Ser Lys Ser Trp Asn Arg Ala Ser Asn Leu Leu
        115                 120                 125

Phe Val Glu Ser Pro Ala Gly Val Gly Trp Ser Tyr Ser Asn Thr Ser
    130                 135                 140

Ser Asp Tyr Asn Thr Gly Asp Ala Thr Ser Ala Lys Asp Met Leu Lys
145                 150                 155                 160

Phe Leu Val Asn Trp Met Val Lys Phe Pro Glu Phe Lys Ser Arg Asp
                165                 170                 175

Leu Tyr Leu Thr Gly Glu Ser Tyr Ala Gly His Tyr Ile Pro Gln Leu
            180                 185                 190

Ala Val Ala Ile Leu Asp His Asn Ala Gln Ser Lys Ala Ser Lys Leu
        195                 200                 205

Asn Leu Lys Gly Val Ala Ile Gly Asn Pro Leu Leu Lys Leu Asp Arg
    210                 215                 220

Asp Val Pro Ala Thr Tyr Glu Phe Tyr Trp Ser His Gly Met Ile Ser
225                 230                 235                 240

Asp Glu Ile Gly Leu Ala Ile Met Asn Gln Cys Asp Phe Glu Asp Tyr
                245                 250                 255

Thr Phe Pro Asn Pro His Asn Glu Thr Asp Val Cys Asn Asn Ala Ile
            260                 265                 270

Ser Lys Ala Asn Thr Ile Val Gly Asp Tyr Val Asn Glu Tyr Asp Val
        275                 280                 285

Ile Leu Asp Val Cys Tyr Pro Ser Ile Val Glu Gln Glu Leu Arg Leu
    290                 295                 300

Lys Lys Met Ala Thr Lys Met Ser Val Gly Val Asp Val Cys Met Thr
305                 310                 315                 320

Tyr Glu Arg Arg Phe Tyr Phe Asn Leu Pro Glu Val Gln Arg Ala Leu
                325                 330                 335

His Ala Asn Arg Thr Asn Leu Pro Tyr Glu Trp Ser Met Cys Ser Ser
            340                 345                 350

Ile Leu Asn Tyr Thr Asp Thr Asp Gly Ser Leu Asn Met Leu Pro Leu
        355                 360                 365

Leu Lys Arg Ile Leu Leu His Asn Ile Arg Val Trp Ile Phe Ser Gly
    370                 375                 380

Asp Gln Asp Ser Val Val Pro Leu Leu Gly Ser Arg Thr Leu Val Arg
385                 390                 395                 400

Glu Leu Ala His Asp Leu Lys Tyr Asn Ile Thr Val Pro Tyr Ala Asn
                405                 410                 415

Trp Phe His Lys Gly Gln Val Gly Gly Trp Thr Thr Glu Tyr Gly Asn
            420                 425                 430

Leu Leu Thr Phe Ala Thr Val Arg Gly Ala Ala His Met Val Pro Tyr
        435                 440                 445

Ala Gln Pro Ser Arg Ala Leu His Leu Phe Ser Ser Phe Ile His Asp
    450                 455                 460

Arg Lys Leu Pro Thr Thr Thr Lys Glu Ser Ile Glu
465                 470                 475

<210>  60
<211>  1494
<212>  DNA
<213>  Chenopodium quinoa

<400>  60
atggaaaagt tgatcatttt cttgacagtc ctcttcttcc tctcttcctt cacctctgcc      60

aaaatttcgg ttttcaacga atttcaggct gataatccca ttgaagcgtt caatttattc     120

ccaaaaagac ccgttaacat aatcaatagc tctgataatg gcgatctttc tcacagcagc     180

tcaaaaatcg tcgaaaagag gttcaaattt cccaatttct acgatcctag tggtgcaact     240

gttgaagaac tgggccatca tgctggttat tatcagattc aacactctca taatgctagg     300

atgttctact ttttctttga atcacgtaac agaaaagaag accctgttgt tatctggtta     360

actggagggc ctggttgcag cagtgagttg gctctctttt atgaaaatgg gccatttaaa     420

attgcaaaca acttgtccct tgtatggaat gaatatggct gggaccaggc tgccaacctc     480

atctttgttg accagcccat tgggactgga ttcagttact ccactgatag acatgacata     540

cgacatgatg aaagtggggt tagcaatgat ttgtatgact ttttacaggc attctttacc     600

aagcacccaa gtttcgcaaa aaatgacttc tacatcactg gagaatcata tgctggccac     660

tacattcctg cctttgctgc ttgtgttcat agcaagaata aagctaagaa aggaattcat     720

atcaacctca agggatttgc tattggcaat gggctgactg atcctgcagt gcagtatgaa     780

gcatatcctg attatgcatt agagatgggg ttgatcaaga aatcatccta caatgtgatc     840

actaagatgg ttcctctgtg tgaaacagca gtaaaactct gtggtacaaa gggtacagtc     900

tcctgcatga cttcatacct tttatgcaac actatattta caagcattag actgctaatt     960

ggtgatacta attattacga tataagaaaa aagtgtgagg gacgcttgtg ctatgacttc    1020

tcaaacatgg aaaagttctt aaatcaggaa tctgttagga gtgctctggg tgttggagac    1080

attgaatttc agtcttgtag caccacggta tatcaggcct tgcttgttga ttggatgaga 1140

aatcttgagg ttggcatccc agctcttctt gaggatggaa ttcagttgct tgtatatgct 1200

ggagaatacg atctcatctg caattggctt ggcaactcaa gatgggtcca tgctatggaa 1260

tggagtgcgc agcaagattt tgtagcatca cctgagattc ctttcgaagt tgatggctct 1320

gaggcaggaa tactgaagag ccttggtcct ttgagtttcc tcaaggttca tgatgctggt 1380

cacatggttc ctatggatca gccgaaggca gccttagaaa tgttgagacg atggactaaa 1440

ggctcacttt tgctatctac ttctacgcct caaacttttg ttgcttccca gtga 1494

<210> 61
<211> 497
<212> PRT
<213> Chenopodium quinoa

<400> 61

Met Glu Lys Leu Ile Ile Phe Leu Thr Val Leu Phe Phe Leu Ser Ser
1               5                   10                  15

Phe Thr Ser Ala Lys Ile Ser Val Phe Asn Glu Phe Gln Ala Asp Asn
            20                  25                  30

Pro Ile Glu Ala Phe Asn Leu Phe Pro Lys Arg Pro Val Asn Ile Ile
        35                  40                  45

Asn Ser Ser Asp Asn Gly Asp Leu Ser His Ser Ser Ser Lys Ile Val
    50                  55                  60

Glu Lys Arg Phe Lys Phe Pro Asn Phe Tyr Asp Pro Ser Gly Ala Thr
65                  70                  75                  80

Val Glu Glu Leu Gly His His Ala Gly Tyr Tyr Gln Ile Gln His Ser
                85                  90                  95

His Asn Ala Arg Met Phe Tyr Phe Phe Phe Glu Ser Arg Asn Arg Lys
            100                 105                 110

Glu Asp Pro Val Val Ile Trp Leu Thr Gly Gly Pro Gly Cys Ser Ser
        115                 120                 125

Glu Leu Ala Leu Phe Tyr Glu Asn Gly Pro Phe Lys Ile Ala Asn Asn
    130                 135                 140

Leu Ser Leu Val Trp Asn Glu Tyr Gly Trp Asp Gln Ala Ala Asn Leu
145                 150                 155                 160

Ile Phe Val Asp Gln Pro Ile Gly Thr Gly Phe Ser Tyr Ser Thr Asp

                165                 170                 175

Arg His Asp Ile Arg His Asp Glu Ser Gly Val Ser Asn Asp Leu Tyr
            180                 185                 190

Asp Phe Leu Gln Ala Phe Phe Thr Lys His Pro Ser Phe Ala Lys Asn
        195                 200                 205

Asp Phe Tyr Ile Thr Gly Glu Ser Tyr Ala Gly His Tyr Ile Pro Ala
    210                 215                 220

Phe Ala Ala Cys Val His Ser Lys Asn Lys Ala Lys Lys Gly Ile His
225                 230                 235                 240

Ile Asn Leu Lys Gly Phe Ala Ile Gly Asn Gly Leu Thr Asp Pro Ala
                245                 250                 255

Val Gln Tyr Glu Ala Tyr Pro Asp Tyr Ala Leu Glu Met Gly Leu Ile
            260                 265                 270

Lys Lys Ser Ser Tyr Asn Val Ile Thr Lys Met Val Pro Leu Cys Glu
        275                 280                 285

Thr Ala Val Lys Leu Cys Gly Thr Lys Gly Thr Val Ser Cys Met Thr
    290                 295                 300

Ser Tyr Leu Leu Cys Asn Thr Ile Phe Thr Ser Ile Arg Leu Leu Ile
305                 310                 315                 320

Gly Asp Thr Asn Tyr Tyr Asp Ile Arg Lys Lys Cys Glu Gly Arg Leu
                325                 330                 335

Cys Tyr Asp Phe Ser Asn Met Glu Lys Phe Leu Asn Gln Glu Ser Val
            340                 345                 350

Arg Ser Ala Leu Gly Val Gly Asp Ile Glu Phe Gln Ser Cys Ser Thr
        355                 360                 365

Thr Val Tyr Gln Ala Leu Leu Val Asp Trp Met Arg Asn Leu Glu Val
    370                 375                 380

Gly Ile Pro Ala Leu Leu Glu Asp Gly Ile Gln Leu Leu Val Tyr Ala
385                 390                 395                 400

Gly Glu Tyr Asp Leu Ile Cys Asn Trp Leu Gly Asn Ser Arg Trp Val
                405                 410                 415

His Ala Met Glu Trp Ser Ala Gln Gln Asp Phe Val Ala Ser Pro Glu
            420                 425                 430

Ile Pro Phe Glu Val Asp Gly Ser Glu Ala Gly Ile Leu Lys Ser Leu
        435                 440                 445

Gly Pro Leu Ser Phe Leu Lys Val His Asp Ala Gly His Met Val Pro
    450                 455                 460

Met Asp Gln Pro Lys Ala Ala Leu Glu Met Leu Arg Arg Trp Thr Lys
465                 470                 475                 480

Gly Ser Leu Leu Leu Ser Thr Ser Thr Pro Gln Thr Phe Val Ala Ser
                485                 490                 495

Gln

<210>  62
<211>  1554
<212>  DNA
<213>  Chenopodium quinoa

<400>  62
atgggtttta agttttttca taaatttagt tattttgttg ttgttttttg tctattgaat      60

ttacaatcat tgtgttttct aaccaatgct aaaccaaacc ataaacaaca agaacatttt     120

aagttgtcat ctaccacaac atttccaaaa cttcaagcag agagacttat aaggcaactt     180

aatttattcc caaaacaccc ttataaccac cacgacgacg tcgaaagcgg gttatcggtt     240

tcggaatcgg gtttggtaga gaaaaggttt aagatatccg aattgggtgg tgacccgggt     300

actagtgttg aggatttggg tcatcatgca gggtattatc gtcttcctca ttccaaagat     360

gccaggatgt tctacttctt ttttgaatca cgaaatcgca aggatgatcc cgtcgtgatt     420

tggttgacag gaggaccagg gtgcagcagt gagctagctc tattctatga gaatggacct     480

tatcaaattg ccaacaatat gtctttggtt tggaatgatt atggttggga taaggcttcc     540

aatctcatct acgtcgacca gccaatagga acaggtttta gttacacttc caattctggt     600

gacatccgac acgatgagaa tggtgttagc aatgacttgt atgacttcct gcaggctttc     660

ttcaaggcgc atcctcagta tgccaagaat gacttctata tcactggaga atcctacgct     720

gggcattaca tccctgcctt tgctgctaga gttcacaacg gaaacaaggc taaagaagga     780

ctccccataa atctcaaggg atttgcaatt ggtaacggcc tcacccatcc agaaatccaa     840

tacaaagcat acccagactt tgctttggat aatggaatca tcaaacaggc agagcacaaa     900

agcatcagcg aaatggttcc tgcttgtgaa caagcaatca agcagtgtgg tactgatggt     960

aaagattctt gcttgaatgc gtactttgct tgcaccaaca tcttcaacaa aatcatggac    1020

tatgcaggag acacaaatta ctatgacatc aggaagaagt gtgagaccag cttgtgttat 1080

gacttttcga atatggagac attactgaat cagaaatcag taagaaaagc ccttggtgtt 1140

ggggacattg acttcgtatc atgtagtacc gaggtgtatg atgccatgca aatggactgg 1200

atgaggaatc tcgaagttgg tatccctgcc cttttggagg atggaatcaa gctgctggtt 1260

tatgctgggg agtatgatct gatatgcaac tggcttggga actctaggtg ggttcatgcc 1320

gtggaatggt ctggacagaa agatttacaa gcagctccta ctgtatcctt cactgttgat 1380

aacaaagaag caggacaaat gaaaagccat ggttcccttg ctttcctcaa ggtgcatgat 1440

gctggacaca tggtcccaat ggaccagcct aaggctgctc tagagatgct caagaggtgg 1500

actcaaggta ctctggcttc agcagatata acagagaaga tttcacctgt gtga 1554

<210> 63
<211> 517
<212> PRT
<213> Chenopodium quinoa

<400> 63

Met Gly Phe Lys Phe Phe His Lys Phe Ser Tyr Phe Val Val Val Phe
1               5                   10                  15

Cys Leu Leu Asn Leu Gln Ser Leu Cys Phe Leu Thr Asn Ala Lys Pro
            20                  25                  30

Asn His Lys Gln Gln Glu His Phe Lys Leu Ser Ser Thr Thr Thr Phe
        35                  40                  45

Pro Lys Leu Gln Ala Glu Arg Leu Ile Arg Gln Leu Asn Leu Phe Pro
    50                  55                  60

Lys His Pro Tyr Asn His His Asp Asp Val Glu Ser Gly Leu Ser Val
65                  70                  75                  80

Ser Glu Ser Gly Leu Val Glu Lys Arg Phe Lys Ile Ser Glu Leu Gly
                85                  90                  95

Gly Asp Pro Gly Thr Ser Val Glu Asp Leu Gly His His Ala Gly Tyr
            100                 105                 110

Tyr Arg Leu Pro His Ser Lys Asp Ala Arg Met Phe Tyr Phe Phe Phe
        115                 120                 125

Glu Ser Arg Asn Arg Lys Asp Asp Pro Val Val Ile Trp Leu Thr Gly
    130                 135                 140

Gly Pro Gly Cys Ser Ser Glu Leu Ala Leu Phe Tyr Glu Asn Gly Pro
145                 150                 155                 160

Tyr Gln Ile Ala Asn Asn Met Ser Leu Val Trp Asn Asp Tyr Gly Trp
                165                 170                 175

Asp Lys Ala Ser Asn Leu Ile Tyr Val Asp Gln Pro Ile Gly Thr Gly
            180                 185                 190

Phe Ser Tyr Thr Ser Asn Ser Gly Asp Ile Arg His Asp Glu Asn Gly
        195                 200                 205

Val Ser Asn Asp Leu Tyr Asp Phe Leu Gln Ala Phe Phe Lys Ala His
    210                 215                 220

Pro Gln Tyr Ala Lys Asn Asp Phe Tyr Ile Thr Gly Glu Ser Tyr Ala
225                 230                 235                 240

Gly His Tyr Ile Pro Ala Phe Ala Ala Arg Val His Asn Gly Asn Lys
                245                 250                 255

Ala Lys Glu Gly Leu Pro Ile Asn Leu Lys Gly Phe Ala Ile Gly Asn
            260                 265                 270

Gly Leu Thr His Pro Glu Ile Gln Tyr Lys Ala Tyr Pro Asp Phe Ala
        275                 280                 285

Leu Asp Asn Gly Ile Ile Lys Gln Ala Glu His Lys Ser Ile Ser Glu
    290                 295                 300

Met Val Pro Ala Cys Glu Gln Ala Ile Lys Gln Cys Gly Thr Asp Gly
305                 310                 315                 320

Lys Asp Ser Cys Leu Asn Ala Tyr Phe Ala Cys Thr Asn Ile Phe Asn
                325                 330                 335

Lys Ile Met Asp Tyr Ala Gly Asp Thr Asn Tyr Tyr Asp Ile Arg Lys
            340                 345                 350

Lys Cys Glu Thr Ser Leu Cys Tyr Asp Phe Ser Asn Met Glu Thr Leu
        355                 360                 365

Leu Asn Gln Lys Ser Val Arg Lys Ala Leu Gly Val Gly Asp Ile Asp
    370                 375                 380

Phe Val Ser Cys Ser Thr Glu Val Tyr Asp Ala Met Gln Met Asp Trp
385                 390                 395                 400

Met Arg Asn Leu Glu Val Gly Ile Pro Ala Leu Leu Glu Asp Gly Ile
                405                 410                 415

Lys Leu Leu Val Tyr Ala Gly Glu Tyr Asp Leu Ile Cys Asn Trp Leu
            420                 425                 430

Gly Asn Ser Arg Trp Val His Ala Val Glu Trp Ser Gly Gln Lys Asp
        435                 440                 445

Phe Thr Ala Ala Pro Thr Val Ser Phe Thr Val Asp Asn Lys Glu Ala
    450                 455                 460

Gly Gln Met Lys Ser His Gly Ser Leu Ala Phe Leu Lys Val His Asp
465                 470                 475                 480

Ala Gly His Met Val Pro Met Asp Gln Pro Lys Ala Ala Leu Glu Met
                485                 490                 495

Leu Lys Arg Trp Thr Gln Gly Thr Leu Ala Ser Ala Asp Ile Thr Glu
            500                 505                 510

Lys Ile Ser Pro Val
        515

<210>  64
<211>  1341
<212>  DNA
<213>  Chenopodium quinoa

<400>  64
   atgaaaacat tcatcctctc attatctatc cttcttcttc tattccattt tactcctact      60
   attagttcat catccacccc atttacaccc gaatcgcttc ctactaaatc cggctactta     120
   gtcgtaaact cgacttctaa gtcgtcgatt tactacactt attatgaagc acagcaacct     180
   gtttcaacac ttgaaaaaac tccacttctt gtatggctcc aaggaggtcc tggttgttcc     240
   tctatggttg ggaacttcta tgagctcggc ccgtggcggg ttaaccataa gattgagtta     300
   gaaagaaata aaggcgcatg gaatcgaata ttcgggcttt tgttcttgga taatccaatt     360
   gggacagggt ttagcattgc tgctacacca gaggagattc caaggatcaa acatggtgta     420
   gctaaacatc tttttattgc tattaaatca tttcttgcat tagataaagc tgtatttgga     480
   aatcgacccg tttatattac tggtgaaagt tatgcaggaa aatatgctcc tgcaataggg     540
   tattacatag taaggagaaa tagtgagcta ccagaaactg agagggtaaa ttttggtgga     600
   attgcaattg gaaacgggtt aactgacccg ataacccagg tgacaaccca tgggttgaat     660
   gcttatttta gtgggttggt gaatgaaaag cagagattgc agttagagga aggtcaaagg     720
   gcggcggtaa agttggttat ggagaggaag tggactgagg caactgatgc aagaagtaga 780
   gtattgaatt tgctccaaga gatgacaggg ttggctactt tgtatgatat cagaaggaaa 840
   ataccctatg agtcgtactt ggtagagcgg tttttgagca gttctgaggt caagacggcg 900
   ttgggggtga ataaatccat ggtttgggag gactgcagta atatagtagg cgagatttta 960
   catgaagatg taatgaagag tgtgaagtac atggtggaaa tgttgttgca gaagaccaag 1020
   gttctgttat atcaggggca gtgtgacctg agagatgggg tggtttccgt agaggcttgg 1080
   ctgaagaagt tgaagtggga aggggttcgg aagtttgaag atgcagagag gaaagtgtgg 1140
   aaagtagaag aggaggttgc cgggtatgtg cagaaatggg ggagtttgac tcatgctgta 1200
   gtgttgggag caggacattt tgtgcccact gatcaggctg ttcattcgca ggtcatgatc 1260
   gaggattgga tccttgaaaa aggaattttc gcaagagatg aaattgagat tgaatcacga 1320
   tgtccaaaga attttctttg a 1341

<210> 65
<211> 446
<212> PRT
<213> Chenopodium quinoa

<400> 65

Met Lys Thr Phe Ile Leu Ser Leu Ser Ile Leu Leu Leu Leu Phe His
1               5                   10                  15

Phe Thr Pro Thr Ile Ser Ser Ser Ser Thr Pro Phe Thr Pro Glu Ser
            20                  25                  30

Leu Pro Thr Lys Ser Gly Tyr Leu Val Val Asn Ser Thr Ser Lys Ser
        35                  40                  45

Ser Ile Tyr Tyr Thr Tyr Tyr Glu Ala Gln Gln Pro Val Ser Thr Leu
    50                  55                  60

Glu Lys Thr Pro Leu Leu Val Trp Leu Gln Gly Gly Pro Gly Cys Ser
65                  70                  75                  80

Ser Met Val Gly Asn Phe Tyr Glu Leu Gly Pro Trp Arg Val Asn His
                85                  90                  95

Lys Ile Glu Leu Glu Arg Asn Lys Gly Ala Trp Asn Arg Ile Phe Gly
            100                 105                 110

Leu Leu Phe Leu Asp Asn Pro Ile Gly Thr Gly Phe Ser Ile Ala Ala
        115                 120                 125

Thr Pro Glu Glu Ile Pro Arg Asp Gln His Gly Val Ala Lys His Leu

130                 135                 140

Phe Ile Ala Ile Lys Ser Phe Leu Ala Leu Asp Lys Ala Val Phe Gly
145                 150                 155                 160

Asn Arg Pro Val Tyr Ile Thr Gly Glu Ser Tyr Ala Gly Lys Tyr Ala
                165                 170                 175

Pro Ala Ile Gly Tyr Tyr Ile Val Arg Arg Asn Ser Glu Leu Pro Glu
            180                 185                 190

Thr Glu Arg Val Asn Phe Gly Gly Ile Ala Ile Gly Asn Gly Leu Thr
        195                 200                 205

Asp Pro Ile Thr Gln Val Thr Thr His Gly Leu Asn Ala Tyr Phe Ser
    210                 215                 220

Gly Leu Val Asn Glu Lys Gln Arg Leu Gln Leu Glu Glu Gly Gln Arg
225                 230                 235                 240

Ala Ala Val Lys Leu Val Met Glu Arg Lys Trp Thr Glu Ala Thr Asp
                245                 250                 255

Ala Arg Ser Arg Val Leu Asn Leu Leu Gln Glu Met Thr Gly Leu Ala
            260                 265                 270

Thr Leu Tyr Asp Ile Arg Arg Lys Ile Pro Tyr Glu Ser Tyr Leu Val
        275                 280                 285

Glu Arg Phe Leu Ser Ser Ser Glu Val Lys Thr Ala Leu Gly Val Asn
    290                 295                 300

Lys Ser Met Val Trp Glu Asp Cys Ser Asn Ile Val Gly Glu Ile Leu
305                 310                 315                 320

His Glu Asp Val Met Lys Ser Val Lys Tyr Met Val Glu Met Leu Leu
                325                 330                 335

Gln Lys Thr Lys Val Leu Leu Tyr Gln Gly Gln Cys Asp Leu Arg Asp
            340                 345                 350

Gly Val Val Ser Val Glu Ala Trp Leu Lys Lys Leu Lys Trp Glu Gly
        355                 360                 365

Val Arg Lys Phe Glu Asp Ala Glu Arg Lys Val Trp Lys Val Glu Glu
    370                 375                 380

Glu Val Ala Gly Tyr Val Gln Lys Trp Gly Ser Leu Thr His Ala Val
385                 390                 395                 400

Val Leu Gly Ala Gly His Phe Val Pro Thr Asp Gln Ala Val His Ser
                405                 410                 415

Gln Val Met Ile Glu Asp Trp Ile Leu Glu Lys Gly Ile Phe Ala Arg
            420                 425                 430

Asp Glu Ile Glu Ile Glu Ser Arg Cys Pro Lys Asn Phe Leu
        435                 440                 445

<210>  66
<211>  1500
<212>  DNA
<213>  Chenopodium quinoa

<400>  66
atggctttct cctcttctac accatcatca ttaacatttt cttgtaaata tttttgttta      60

tctatactcc taacatggct tacaacccat gttatgatag tttccgcttc ggtatcgacc     120

cttgggctaa ccccagaggt ggtggctaag cagaacgccg accgcgtcgt cgacttgcca     180

gggcagccgg tagttgggtt taagcattat gctggttatg tgaatgtgaa tgctagtcat     240

ggtagagaac tgttttattg gttttttgaa gctgatcata atcctcatct taaacctgtg     300

gttctctggc ttaatggagg tcctggatgc tcttcggttg gttatggtga agctgaagag     360

ctaggtccct tcttccctaa gaagggaagc aaaccgtcgt tgaaacacaa caaattctca     420

tggaataaag ctgcaaatct gttgtttttg gagtctcctg ttggtgttgg tttttcttac     480

accaatacta gcagtgattt tgatgaacta ggtgacactt ttacagcaaa ggattcttac     540

atattccttg tcaactggtt caagagattt ccgcaattta agtcccatga attctattta     600

gcaggggaaa gttatgcagg ccactatgtt ccacagctcg cagagtacat ctttgacagt     660

aaaaaacatg cttgcaagga ggattttatc aatctgaaag gtttcttgat tgggaacgcg     720

ttgttggatg atgaaacaga tcaaacagga atgatcgact atgcctggga tcatgctgtt     780

atatcagata aagtcttcag agatgtgaag agaaagtgca acttcaaagt tgagaacgta     840

tcagatgatt gcacaagtgc acttaatgag tactttgaag tttacgacct gatagatatg     900

tacagcttgt acgcgcctat ctgcacaaaa aattacactg atgttaacag cagcaacagc     960

aggaggcaac tttctgccat caaaggcatt gcacctcgcg tgttctccaa atttgataaa    1020

tggcacaggc ggcctgctgg atacgaccct tgtttagatc attacacaga cgtgtatcta    1080

aataggccag atgttcaagc agcacttcat gccaatacct cgaaaattcc ttacgcttgg    1140

accttctgca gcgacaacat tacattttgg gctgatgcac ctgcctcaat acttcctgtt    1200

attaggaagc ttatagatgg tgggcttcgg atatgggttt tcagtggaga tactgatggg    1260

aggattcctg tgagttcgac gaggttaaca ctcagaaagc ttggcttgaa gatcaaacaa     1320

gattggacac cctggtatag caaagatcag gttggtggat ggaccattgc ctatgatggg     1380

ctgatgtttg ttacagttcg aggcgcgggt caccaagttc caacttacaa gccaagggaa     1440

gcccttctga tgctcaggca tttccttgcc aacaaaacaa tgccgtctaa agccttttga     1500

<210>  67
<211>  499
<212>  PRT
<213>  Chenopodium quinoa

<400>  67

Met Ala Phe Ser Ser Ser Thr Pro Ser Ser Leu Thr Phe Ser Cys Lys
1               5                   10                  15

Tyr Phe Cys Leu Ser Ile Leu Leu Thr Trp Leu Thr Thr His Val Met
            20                  25                  30

Ile Val Ser Ala Ser Val Ser Thr Leu Gly Leu Thr Pro Glu Val Val
        35                  40                  45

Ala Lys Gln Asn Ala Asp Arg Val Val Asp Leu Pro Gly Gln Pro Val
    50                  55                  60

Val Gly Phe Lys His Tyr Ala Gly Tyr Val Asn Val Asn Ala Ser His
65                  70                  75                  80

Gly Arg Glu Leu Phe Tyr Trp Phe Phe Glu Ala Asp His Asn Pro His
                85                  90                  95

Leu Lys Pro Val Val Leu Trp Leu Asn Gly Gly Pro Gly Cys Ser Ser
            100                 105                 110

Val Gly Tyr Gly Glu Ala Glu Glu Leu Gly Pro Phe Phe Pro Lys Lys
        115                 120                 125

Gly Ser Lys Pro Ser Leu Lys His Asn Lys Phe Ser Trp Asn Lys Ala
    130                 135                 140

Ala Asn Leu Leu Phe Leu Glu Ser Pro Val Gly Val Gly Phe Ser Tyr
145                 150                 155                 160

Thr Asn Thr Ser Ser Asp Phe Asp Glu Leu Gly Asp Thr Phe Thr Ala
                165                 170                 175

Lys Asp Ser Tyr Ile Phe Leu Val Asn Trp Phe Lys Arg Phe Pro Gln
            180                 185                 190

Phe Lys Ser His Glu Phe Tyr Leu Ala Gly Glu Ser Tyr Ala Gly His
        195                 200                 205

Tyr Val Pro Gln Leu Ala Glu Tyr Ile Phe Asp Ser Lys Lys His Ala
    210                 215                 220

Cys Lys Glu Asp Phe Ile Asn Leu Lys Gly Phe Leu Ile Gly Asn Ala
225                 230                 235                 240

Leu Leu Asp Asp Glu Thr Asp Gln Thr Gly Met Ile Asp Tyr Ala Trp
                245                 250                 255

Asp His Ala Val Ile Ser Asp Lys Val Phe Arg Asp Val Lys Arg Lys
            260                 265                 270

Cys Asn Phe Lys Val Glu Asn Val Ser Asp Asp Cys Thr Ser Ala Leu
        275                 280                 285

Asn Glu Tyr Phe Glu Val Tyr Asp Leu Ile Asp Met Tyr Ser Leu Tyr
    290                 295                 300

Ala Pro Ile Cys Thr Lys Asn Tyr Thr Asp Val Asn Ser Ser Asn Ser
305                 310                 315                 320

Arg Arg Gln Leu Ser Ala Ile Lys Gly Ile Ala Pro Arg Val Phe Ser
                325                 330                 335

Lys Phe Asp Lys Trp His Arg Arg Pro Ala Gly Tyr Asp Pro Cys Leu
            340                 345                 350

Asp His Tyr Thr Asp Val Tyr Leu Asn Arg Pro Asp Val Gln Ala Ala
        355                 360                 365

Leu His Ala Asn Thr Ser Lys Ile Pro Tyr Ala Trp Thr Phe Cys Ser
    370                 375                 380

Asp Asn Ile Thr Phe Trp Ala Asp Ala Pro Ala Ser Ile Leu Pro Val
385                 390                 395                 400

Ile Arg Lys Leu Ile Asp Gly Gly Leu Arg Ile Trp Val Phe Ser Gly
                405                 410                 415

Asp Thr Asp Gly Arg Ile Pro Val Ser Ser Thr Arg Leu Thr Leu Arg
            420                 425                 430

Lys Leu Gly Leu Lys Ile Lys Gln Asp Trp Thr Pro Trp Tyr Ser Lys

435                 440                 445

Asp Gln Val Gly Gly Trp Thr Ile Ala Tyr Asp Gly Leu Met Phe Val
    450                 455                 460

Thr Val Arg Gly Ala Gly His Gln Val Pro Thr Tyr Lys Pro Arg Glu
465                 470                 475                 480

Ala Leu Leu Met Leu Arg His Phe Leu Ala Asn Lys Thr Met Pro Ser
                485                 490                 495

Lys Ala Phe

<210>  68
<211>  1515
<212>  DNA
<213>  Chenopodium quinoa

<400>  68
atgggtttta agttttttca taaatttagt tattttgttg ttgttttttg tctattgaat      60

ttacaatcat tgtgttttct aaccaatgct aaaccaaacc ataaacaaca agaacatttt     120

aagttgtcat ctaccacaac atttccaaaa cttcaagcag agagacttat aaggcaactt     180

aatttattcc caaaacaccc ttataaccac cacgacgacg tcgaaagcgg gttatcggtt     240

tcggaatcgg gtttggtaga gaaaaggttt aagatatccg aattgggtgg tgacccgggt     300

actagtgttg aggatttggg tcatcatgca gggtattatc gtcttcctca ttccaaagat     360

gccaggatgt tctacttctt ttttgaatca cgaaatcgca aggatgatcc cgtcgtgatt     420

tggttgacag gaggaccagg gtgcagcagt gagctagctc tattctatga gaatggacct     480

tatcaaattg ccaacaatat gtctttggtt tggaatgatt atggttggga taaggcttcc     540

aatctcatct acgtcgacca gccaatagga acaggtttta gttacacttc caattctggt     600

gacatccgac acgatgagaa tggtgttagc aatgacttgt atgacttcct gcaggctttc     660

ttcaaggcgc atcctcagta tgccaagaat gacttctata tcactggaga atcctacgct     720

gggcattaca tccctgcctt tgctgctaga gttcacaacg gaaacaaggc taaagaagga     780

ctccccataa atctcaaggg atttgcaatt ggtaacggcc tcacccatcc agaaatccaa     840

tacaaagcat acccagactt tgctttggat aatggaatca tcaaacaggc agagcacaaa     900

agcatcagcg aaatggttcc tgcttgtgaa caagcaatca agcagtgtgg tactgatggt     960

aaagattctt gcttgaatgc gtactttgct tgcaccaaca tcttcaacaa aatcatggac    1020

tatgcaggag acacaaatta ctatgacatc aggaagaagt gtgagaccag cttgtgttat    1080

gacttttcga atatggagac attactgaat cagaaatcag taagaaaagc ccttggtgtt    1140

ggggacattg acttcgtatc atgtagtacc gaggtgtatg atgccatgca aatggactgg 1200

atgaggaatc tcgaagttgg tatccctgcc cttttggagg atggaatcaa gctgctggtt 1260

tatgctgggg agtatgatct gatatgcaac tggcttggga actctaggtg ggttcatgcc 1320

gtggaatggt ctggacagaa agattttaca gcagctccta ctgtatcctt cactgttgat 1380

aacaaagaag caggacaaat gaaaagccat ggttcccttg ctttcctcaa ggtgcatgat 1440

gctggacaca tggtcccaat ggaccagcct aaggctgctc tagagatgct caagaggtgg 1500

actcaaggat cataa 1515

<210> 69
<211> 504
<212> PRT
<213> Chenopodium quinoa

<400> 69

Met Gly Phe Lys Phe Phe His Lys Phe Ser Tyr Phe Val Val Val Phe
1               5                   10                  15

Cys Leu Leu Asn Leu Gln Ser Leu Cys Phe Leu Thr Asn Ala Lys Pro
            20                  25                  30

Asn His Lys Gln Gln Glu His Phe Lys Leu Ser Ser Thr Thr Thr Phe
        35                  40                  45

Pro Lys Leu Gln Ala Glu Arg Leu Ile Arg Gln Leu Asn Leu Phe Pro
    50                  55                  60

Lys His Pro Tyr Asn His His Asp Asp Val Glu Ser Gly Leu Ser Val
65                  70                  75                  80

Ser Glu Ser Gly Leu Val Glu Lys Arg Phe Lys Ile Ser Glu Leu Gly
                85                  90                  95

Gly Asp Pro Gly Thr Ser Val Glu Asp Leu Gly His His Ala Gly Tyr
            100                 105                 110

Tyr Arg Leu Pro His Ser Lys Asp Ala Arg Met Phe Tyr Phe Phe Phe
        115                 120                 125

Glu Ser Arg Asn Arg Lys Asp Asp Pro Val Val Ile Trp Leu Thr Gly
    130                 135                 140

Gly Pro Gly Cys Ser Ser Glu Leu Ala Leu Phe Tyr Glu Asn Gly Pro
145                 150                 155                 160

Tyr Gln Ile Ala Asn Asn Met Ser Leu Val Trp Asn Asp Tyr Gly Trp

                165                 170                 175

Asp Lys Ala Ser Asn Leu Ile Tyr Val Asp Gln Pro Ile Gly Thr Gly
            180                 185                 190

Phe Ser Tyr Thr Ser Asn Ser Gly Asp Ile Arg His Asp Glu Asn Gly
        195                 200                 205

Val Ser Asn Asp Leu Tyr Asp Phe Leu Gln Ala Phe Phe Lys Ala His
    210                 215                 220

Pro Gln Tyr Ala Lys Asn Asp Phe Tyr Ile Thr Gly Glu Ser Tyr Ala
225                 230                 235                 240

Gly His Tyr Ile Pro Ala Phe Ala Ala Arg Val His Asn Gly Asn Lys
                245                 250                 255

Ala Lys Glu Gly Leu Pro Ile Asn Leu Lys Gly Phe Ala Ile Gly Asn
            260                 265                 270

Gly Leu Thr His Pro Glu Ile Gln Tyr Lys Ala Tyr Pro Asp Phe Ala
        275                 280                 285

Leu Asp Asn Gly Ile Ile Lys Gln Ala Glu His Lys Ser Ile Ser Glu
    290                 295                 300

Met Val Pro Ala Cys Glu Gln Ala Ile Lys Gln Cys Gly Thr Asp Gly
305                 310                 315                 320

Lys Asp Ser Cys Leu Asn Ala Tyr Phe Ala Cys Thr Asn Ile Phe Asn
                325                 330                 335

Lys Ile Met Asp Tyr Ala Gly Asp Thr Asn Tyr Tyr Asp Ile Arg Lys
            340                 345                 350

Lys Cys Glu Thr Ser Leu Cys Tyr Asp Phe Ser Asn Met Glu Thr Leu
        355                 360                 365

Leu Asn Gln Lys Ser Val Arg Lys Ala Leu Gly Val Gly Asp Ile Asp
    370                 375                 380

Phe Val Ser Cys Ser Thr Glu Val Tyr Asp Ala Met Gln Met Asp Trp
385                 390                 395                 400

Met Arg Asn Leu Glu Val Gly Ile Pro Ala Leu Leu Glu Asp Gly Ile
                405                 410                 415

Lys Leu Leu Val Tyr Ala Gly Glu Tyr Asp Leu Ile Cys Asn Trp Leu
            420                 425                 430

Gly Asn Ser Arg Trp Val His Ala Val Glu Trp Ser Gly Gln Lys Asp
        435                 440                 445

Phe Thr Ala Ala Pro Thr Val Ser Phe Thr Val Asp Asn Lys Glu Ala
    450                 455                 460

Gly Gln Met Lys Ser His Gly Ser Leu Ala Phe Leu Lys Val His Asp
465                 470                 475                 480

Ala Gly His Met Val Pro Met Asp Gln Pro Lys Ala Ala Leu Glu Met
            485                 490                 495

Leu Lys Arg Trp Thr Gln Gly Ser
            500

## Description

### Technical Field

The present invention relates to a method of synthesizing celosianin II, a method of synthesizing a betaxanthin, an amyioid-β polymerization inhibitor or a therapeutic or preventive agent for Alzheimer's, an amyloid peptide aggregation inhibitor, and an HIV-1 protease activity inhibitor.

The present application claims priority of Japanese Patent Application No. 2020-119563 and priority of Japanese Patent Application No. 2019-229647, which are incorporated herein by reference.

### Background Art

### (Betalains)

Betalains, which are a group of plant pigments, are produced in plants of the order Caryophyllales and some fungi. The betalain pigments are broadly classified into two groups, i.e., betacyanins, which range from red to violet in color, and betaxanthins, which range from yellow to orange in color. In plants, the betalain pigments are known to be involved in resistance to environmental stresses (Non Patent Literatures 1 and 2). The betalain pigments are used for food additives because of their vivid colors.

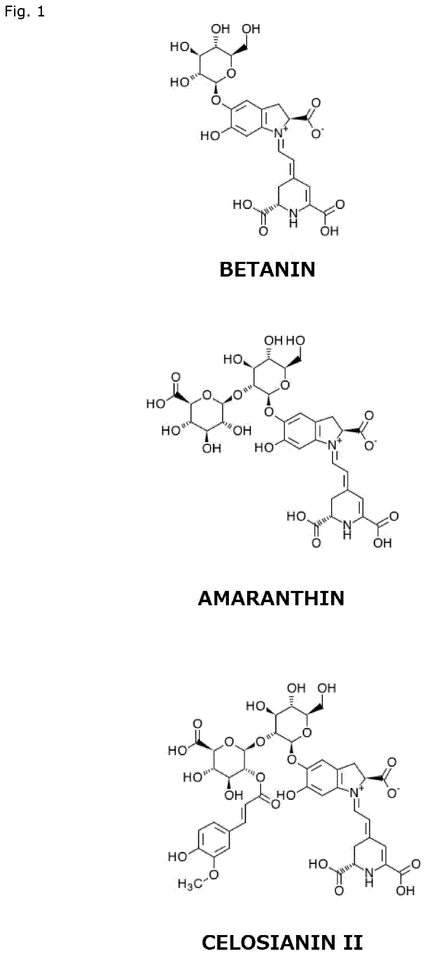

In addition, the betalain pigments have high antioxidant activity, and hence are expected to be utilized as pharmaceuticals and supplements. Previous studies have reported that the betalain pigments have an anti-inflammatory action, an anticancer action, and physiological actions, such as suppression of oxidation of LDL cholesterol and inhibition of HIV-1 protease activity (Non Patent Literatures 3 to 9 and FIG. 1).

The betalain pigments, though having various physiological actions, are difficult to produce because the production of the betalain pigments is mainly performed through extraction from betalain pigment-producing plants. In recent years, it has been reported that betalains have been successfully produced by introducing betalain biosynthesis genes into non-betalain-producing plants (Non Patent Literature 2). The inventors of the present invention have also succeeded in synthesizing amaranthin, which is a betalain pigment (Non Patent Literature 4). However, of the betalain pigments, of which many kinds exist, betalain pigments that can be artificially synthesized are limited, and physiological activities of few individual betalain pigments have been revealed.

### (Quinoa)

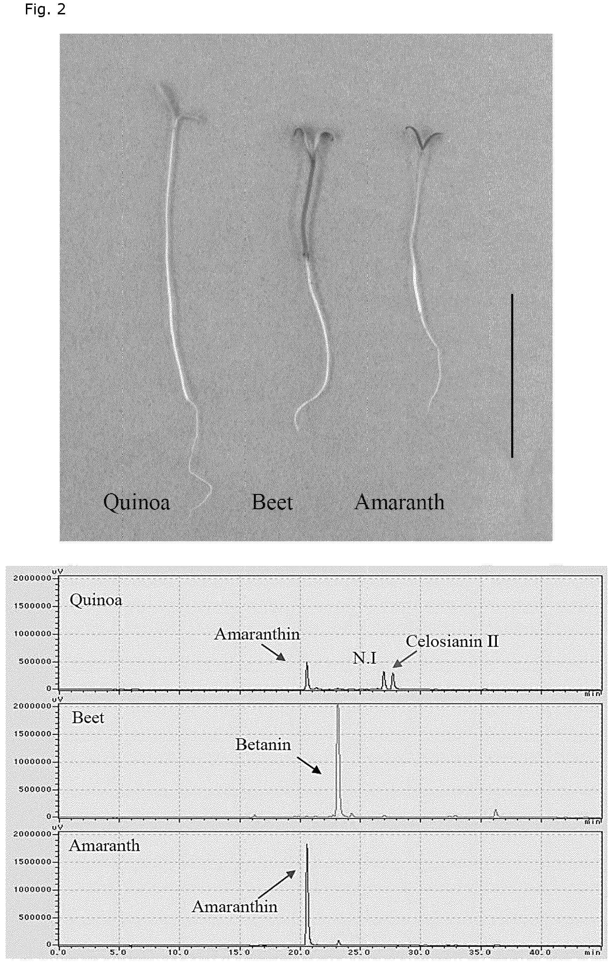

Quinoa (Chenopodium quinoa) is a plant belonging to the genus Chenopodium of the subfamily Chenopodioideae of the family Amaranthaceae, and has rich nutrients and excellent environmental stress tolerance. It is known that three kinds of betalains (betanin, amaranthin, and celosianin II) are synthesized and accumulated in hypocotyls of quinoa (FIG. 1 and FIG. 2). The inventors of the present invention have succeeded in synthesizing betanin and amaranthin in tobacco BYII cells (Non Patent Literature 4).

With regard to celosianin II, no synthesis gene has been identified, and an artificial production system in a non-betalain-producing plant has not been constructed. Further, characteristics and physiological activity of celosianin II have not been reported and are unknown (FIG. 1).

In Patent Literature 1, there is a disclosure of a "method of producing a betacyanin, including a step (conversion step) of converting a raw material into a betacyanin in an aqueous medium in the presence of a microorganism having enzyme activity of hydroxylating the 3-position of the phenol ring of tyrosine, DOPA 4,5-dioxygenase activity, L-DOPA oxidase activity, and enzyme activity of glycosylating a phenolic hydroxy group, or a treated product thereof."

However, a celosianin II synthetase gene and a gene required for betaxanthin synthesis of the present invention are not disclosed or suggested therein.

### Citation List

### Patent Literature

[PTL 1] JP 2016-182044 A

### Non-Patent Literature

[NPL 1] Jain, G., Schwinn, K.E., and Gould, K.S. (2015) Betalain induction byl-DOPA application confers photoprotection to saline-exposed leaves of Disphymaaustrale. New Phytol. 207, 1075-1083.
[NPL 2] Polturak, G., Grossman, N., Vela-Corcia, D., Dong, Y., Nudel, A., Pliner, M., Levy, M., Rogachev, I. and Aharoni, A. (2017) Engineered gray mold resistance, antioxidant capacity, and pigmentation in betalain-producing crops and ornamentals. Proc. Natl. Acad. Sci. U. S. A. 114, 9062-9067.
[NPL 3] Kapadia, G.J., Tokuda, H., Konoshima, T. and Nishino, H. (1996) Chemoprevention of lung and skin cancer by Beta vulgaris (beet) root extract. Cancer Lett. 100, 211-214.
[NPL 4] Imamura, T. et al. (2019) Isolation of amaranthin synthetase from Chenopodium quinoa and construction of an amaranthin production system using suspension-cultured tobacco BY-2 cells. Plant Biotechnol J 17, 969-981.
[NPL 5] Lechner, J.F., Wang, L.S., Rocha, C.M., Larue, B., Henry, C., McIntyre, C.M., Riedl, K.M., Schwartz, S.J. and Stoner, G.D. (2010) Drinking water with red beetroot food color antagonizes esophageal carcinogenesis in N-nitrosomethylbenzylamine-treated rats. J. Med. Food 13, 733-739.
[NPL 6] Martinez, R.M., Longhi-Balbinot, D.T., Zarpelon, A.C., Staurengo-Ferrari, L., Baracat, M.M., Georgetti, S.R., Sassonia, R.C., Verri, W.A., Jr. and Casagrande, R. (2015) Anti-inflammatory activity of betalain-rich dye of Beta vulgaris: effect on edema, leukocyte recruitment, superoxide anion and cytokine production. Arch. Pharm. Res. 38, 494-504.
[NPL 7] Rodriguez, E.B., Vidallon, M.L., Mendoza, D.J. and Reyes, C.T. (2016) Health-promoting bioactivities of betalains from red dragon fruit (Hylocereus polyrhizus (Weber) Britton and Rose) peels as affected by carbohydrate encapsulation. J. Sci. Food Agric. 96, 4679-4689.
[NPL 8] Tesoriere, L., Butera, D., D'Arpa, D., DiGaudio, F., Allegra, M., Gentile, C., and Livrea, M.A. (2003) Increased resistance to oxidation of betalainenriched human low density lipoproteins. Free Radic. Res. 37, 689-696.
[NPL 9] Tesoriere, L., Allegra, M., Butera, D. and Livrea, M.A. (2004) Absorption, excretion, and distribution of dietary antioxidant betalains in LDLs: potential health effects of betalains in humans. Am. J. Clin. Nutr. 80, 941-945.

### Summary of invention

### Technical Problem

An object of the present invention is to provide a method of synthesizing celosianin II, a method of synthesizing a betaxanthin, an amyioid-β polymerization inhibitor or a therapeutic or preventive agent for Alzheimer's, an amyloid peptide aggregation inhibitor, and an HIV-1 protease activity inhibitor.

### Solution to Problem

In order to achieve the above-mentioned object, the inventors of the present invention have isolated a gene having a celosianin II synthesis ability from quinoa, and have constructed a method of synthesizing celosianin II of the present invention.

Further, in order to achieve the above-mentioned object, the inventors of the present invention have isolated a gene required for betaxanthin synthesis from quinoa, and have constructed a method of synthesizing a betaxanthin of the present invention.

Besides, the inventors have recognized that celosianin II or the like serves as an active ingredient of each of an amyioid-β polymerization inhibitor or a therapeutic or preventive agent for Alzheimer's, an amyloid peptide aggregation inhibitor, and an HIV-1 protease activity inhibitor.

That is, the present invention is as described below.
1. A method of synthesizing celosianin II, including:
   culturing a host that has introduced therein the following gene encoding a celosianin II synthetase, a gene encoding an enzyme having activity of hydroxylating a 3-position of a phenol ring of tyrosine, a gene encoding an enzyme having L-DOPA oxidase activity and/or a gene encoding an enzyme having DOPA 4,5-dioxygenase activity, a gene encoding an enzyme having activity of glycosylating a phenolic hydroxy group, and a gene encoding an enzyme having activity of adding glucuronic acid, and that has an ability to produce tyrosine or 3-hydroxy-L-tyrosine (L-DOPA), and extracting celosianin II from the host after the culturing; or
   culturing a host that has introduced therein the following gene encoding a celosianin II synthetase, and that has enzyme activity of having activity of hydroxylating a 3-position of a phenol ring of tyrosine, L-DOPA oxidase activity and/or DOPA 4,5-dioxygenase activity, enzyme activity of having activity of glycosylating a phenolic hydroxy group, enzyme activity of having activity of adding glucuronic acid, and an ability to produce tyrosine or 3-hydroxy-L-tyrosine, and extracting celosianin II from the host after the culturing,
   wherein the gene encoding a celosianin II synthetase is any one or more selected from the following:
      (1) a gene encoding a polypeptide formed of an amino acid sequence set forth in SEQ ID NO: 57, 59, 61, 63, 65, 67, 69, 2, 4, 6, 8, 10, 12, 14, or 16;
      (2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
      (3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
      (4) a gene formed of DNA formed of a base sequence set forth in SEQ ID NO: 56, 58, 60, 62, 64, 66, 68, 1, 3, 5, 7, 9, 11, 13, or 15;
      (5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
      (6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
      (7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
      (8) a gene formed of DNA formed of a degenerate isomer of the base sequence set forth in SEQ ID NO: 56, 58, 60, 62, 64, 66, 68, 1, 3, 5, 7, 9, 11, 13, or 15.
2. A method of synthesizing celosianin II, including culturing a host that has introduced therein the following gene encoding a celosianin II synthetase and that has an ability to produce amaranthin, and extracting celosianin II from the host after the culturing,
   wherein the gene encoding a celosianin II synthetase is any one or more selected from the following:
   (1) a gene encoding a polypeptide formed of an amino acid sequence set forth in SEQ ID NO: 57, 59, 61, 63, 65, 67, 69, 2, 4, 6, 8, 10, 12, 14, or 16;
   (2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
   (3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
   (4) a gene formed of DNA formed of a base sequence set forth in SEQ ID NO: 56, 58, 60, 62, 64, 66, 68, 1, 3, 5, 7, 9, 11, 13, or 15;
   (5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
   (6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
   (7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
   (8) a gene formed of DNA formed of a degenerate isomer of the base sequence set forth in SEQ ID NO: 56, 58, 60, 62, 64, 66, 68, 1, 3, 5, 7, 9, 11, 13, or 15.
3. A method of synthesizing celosianin II, including:
   culturing a host that has introduced therein the following gene encoding a celosianin II synthetase and a gene encoding an enzyme having activity of adding glucuronic acid, and that has an ability to produce betanin, and extracting celosianin II from the host after the culturing; or
   culturing a host that has introduced therein the following gene encoding a celosianin II synthetase, and that has enzyme activity of having activity of adding glucuronic acid and an ability to produce betanin, and extracting celosianin II from the host after the culturing,
   wherein the gene encoding a celosianin II synthetase is any one or more selected from the following:
      (1) a gene encoding a polypeptide formed of an amino acid sequence set forth in SEQ ID NO: 57, 59, 61, 63, 65, 67, 69, 2, 4, 6, 8, 10, 12, 14, or 16;
      (2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
      (3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
      (4) a gene formed of DNA formed of a base sequence set forth in SEQ ID NO: 56, 58, 60, 62, 64, 66, 68, 1, 3, 5, 7, 9, 11, 13, or 15;
      (5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
      (6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
      (7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
      (8) a gene formed of DNA formed of a degenerate isomer of the base sequence set forth in SEQ ID NO: 56, 58, 60, 62, 64, 66, 68, 1, 3, 5, 7, 9, 11, 13, or 15.
4. A method of synthesizing celosianin II, including:
   culturing a host that has introduced therein the following gene encoding a celosianin II synthetase, a gene encoding an enzyme having activity of glycosylating a phenolic hydroxy group, and a gene encoding an enzyme having activity of adding glucuronic acid, and that has an ability to produce betanidin, and extracting celosianin II from the host after the culturing; or
   culturing a host that has introduced therein the following gene encoding a celosianin II synthetase, and that has enzyme activity of having activity of glycosylating a phenolic hydroxy group, enzyme activity of having activity of adding glucuronic acid, and an ability to produce betanidin, and extracting celosianin II from the host after the culturing,
   wherein the gene encoding a celosianin II synthetase is any one or more selected from the following:
      (1) a gene encoding a polypeptide formed of an amino acid sequence set forth in SEQ ID NO: 57, 59, 61, 63, 65, 67, 69, 2, 4, 6, 8, 10, 12, 14, or 16;
      (2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
      (3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
      (4) a gene formed of DNA formed of a base sequence set forth in SEQ ID NO: 56, 58, 60, 62, 64, 66, 68, 1, 3, 5, 7, 9, 11, 13, or 15;
      (5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
      (6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
      (7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
      (8) a gene formed of DNA formed of a degenerate isomer of the base sequence set forth in SEQ ID NO: 56, 58, 60, 62, 64, 66, 68, 1, 3, 5, 7, 9, 11, 13, or 15.
5. The method of synthesizing celosianin II according to any one of the above-mentioned items 1 to 4, wherein the gene encoding a celosianin II synthetase is any one or more selected from genes formed of DNAs formed of the base sequences set forth in SEQ ID NOS: 56, 58, 60, 62, 64, 66, 68, 1, 3, 5, 7, 9, 11, 13, and 15.
6. A celosianin II synthesis composition, including a gene shown in any one of the following items or a vector carrying the gene:
   (1) a gene encoding a polypeptide formed of an amino acid sequence set forth in SEQ ID NO: 57, 59, 61, 63, 65, 67, 69, 2, 4, 6, 8, 10, 12, 14, or 16;
   (2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
   (3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
   (4) a gene formed of DNA formed of a base sequence set forth in SEQ ID NO: 56, 58, 60, 62, 64, 66, 68, 1, 3, 5, 7, 9, 11, 13, or 15;
   (5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
   (6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15; and
   (7) a gene formed of DNA having 90% or more homology to DNA formed of the base sequence set forth in SEQ ID NO: 56, 58, 60, 62, 64, 66, 68, 1, 3, 5, 7, 9, 11, 13, or 15.
7. A celosianin II synthesis composition, including a peptide represented by any one of the following amino acid sequences:
   (1) an amino acid sequence set forth in SEQ ID NO: 57, 59, 61, 63, 65, 67, 69, 2, 4, 6, 8, 10, 12, 14, or 16;
   (2) an amino acid sequence that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that forms a polypeptide having a substantially equivalent ability to synthesize celosianin II to that of the amino acid sequence set forth in SEQ ID NO: 57, 59, 61, 63, 65, 67, 69, 2, 4, 6, 8, 10, 12, 14, or 16; and
   (3) an amino acid sequence that has 90% or more homology to the amino acid sequence set forth in SEQ ID NO: 57, 59, 61, 63, 65, 67, 69, 2, 4, 6, 8, 10, 12, 14, or 16, and that forms a polypeptide having a substantially equivalent ability to synthesize celosianin II to that of the amino acid sequence set forth in SEQ ID NO: 57, 59, 61, 63, 65, 67, 69, 2, 4, 6, 8, 10, 12, 14, or 16.
8. A celosianin II-producing host having introduced therein the synthesis composition of the above-mentioned item 6 or 7.
9. A method of synthesizing celosianin II, including the following (1) or (2):
   (1) a step of bringing amaranthin into contact with the celosianin II synthesis composition of the above-mentioned item 7; or
   (2)
      (a) a step of bringing betanin into contact with an enzyme having activity of adding glucuronic acid, and
      (b) a step of bringing amaranthin obtained in the step (a) into contact with the celosianin II synthesis composition of the above-mentioned item 7.
10. A celosianin II-producing host,
   the host having introduced therein the following gene encoding a celosianin II synthetase, a gene encoding an enzyme having activity of hydroxylating a 3-position of a phenol ring of tyrosine, a gene encoding an enzyme having L-DOPA oxidase activity and/or a gene encoding an enzyme having DOPA 4,5-dioxygenase activity, a gene encoding an enzyme having activity of glycosylating a phenolic hydroxy group, and a gene encoding an enzyme having activity of adding glucuronic acid, and having an ability to produce tyrosine or L-DOPA, or
   the host having introduced therein the following gene encoding a celosianin II synthetase, and having enzyme activity of having activity of hydroxylating a 3-position of a phenol ring of tyrosine, L-DOPA oxidase activity and/or DOPA 4,5-dioxygenase activity, enzyme activity of having activity of glycosylating a phenolic hydroxy group, enzyme activity of having activity of adding glucuronic acid, and an ability to produce tyrosine or 3-hydroxy-L-tyrosine,
   wherein the gene encoding a celosianin II synthetase is any one or more selected from the following:
      (1) a gene encoding a polypeptide formed of an amino acid sequence set forth in SEQ ID NO: 57, 59, 61, 63, 65, 67, 69, 2, 4, 6, 8, 10, 12, 14, or 16;
      (2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, or 12;
      (3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
      (4) a gene formed of DNA formed of a base sequence set forth in SEQ ID NO: 56, 58, 60, 62, 64, 66, 68, 1, 3, 5, 7, 9, 11, 13, or 15;
      (5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
      (6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
      (7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
      (8) a gene formed of DNA formed of a degenerate isomer of the base sequence set forth in SEQ ID NO: 56, 58, 60, 62, 64, 66, 68, 1, 3, 5, 7, 9, 11, 13, or 15.
11. A method of synthesizing a betaxanthin, including:
   culturing, with addition of an amine, a host that has introduced therein a gene encoding an enzyme having enzyme activity of hydroxylating a 3-position of a phenol ring of tyrosine but being free of L-DOPA oxidase activity, and a gene encoding an enzyme having DOPA 4,5-dioxygenase activity, and that has an ability to produce tyrosine or 3-hydroxy-L-tyrosine, and extracting a betaxanthin from the host after the culturing; or
   culturing, with addition of an amine, a host that has introduced therein a gene encoding an enzyme having enzyme activity of hydroxylating a 3-position of a phenol ring of tyrosine but being free of L-DOPA oxidase activity, and that has enzyme activity of having DOPA 4,5-dioxygenase activity, and an ability to produce tyrosine or 3-hydroxy-L-tyrosine, and extracting a betaxanthin from the host after the culturing; or
   culturing a host that has introduced therein a gene encoding an enzyme having enzyme activity of hydroxylating a 3-position of a phenol ring of tyrosine but being free of L-DOPA oxidase activity, a gene encoding an enzyme having DOPA 4,5-dioxygenase activity, and an amine synthesis gene, and that has an ability to produce tyrosine or 3-hydroxy-L-tyrosine, and extracting a betaxanthin from the host after the culturing; or
   culturing a host that has introduced therein a gene encoding an enzyme having enzyme activity of hydroxylating a 3-position of a phenol ring of tyrosine but being free of L-DOPA oxidase activity, and that has enzyme activity of having DOPA 4,5-dioxygenase activity, enzyme activity of having amine synthesis activity, and an ability to produce tyrosine or 3-hydroxy-L-tyrosine, and extracting a betaxanthin from the host after the culturing,
   wherein the gene encoding an enzyme having enzyme activity of hydroxylating a 3-position of a phenol ring of tyrosine but being free of L-DOPA oxidase activity is any one or more selected from the following:
      (1) a gene encoding a polypeptide formed of an amino acid sequence set forth in SEQ ID NO: 51, 53, or 55;
      (2) a gene encoding a polypeptide that has 1 to 20 amino acids substituted, deleted, inserted, and/or added in the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
      (3) a gene encoding a polypeptide that has 90% or more homology to the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
      (4) a gene formed of DNA formed of a base sequence set forth in SEQ ID NO: 50, 52, or 54;
      (5) a gene formed of DNA that hybridizes with DNA formed of a base sequence complementary to DNA formed of the base sequence set forth in SEQ ID NO: 50, 52, or 54 under stringent conditions, and that encodes a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity;
      (6) a gene formed of DNA having a 1- to 50-base sequence substituted, deleted, inserted, and/or added in DNA formed of the base sequence set forth in SEQ ID NO: 50, 52, or 54;
      (7) a gene formed of DNA having 90% or more homology to DNA formed of the base sequence set forth in SEQ ID NO: 50, 52, or 54; and
      (8) a gene formed of DNA formed of a degenerate isomer of the base sequence set forth in SEQ ID NO: 50, 52, or 54.
12. The method of synthesizing a betaxanthin according to the above-mentioned item 11, wherein the gene encoding an enzyme having enzyme activity of hydroxylating a 3-position of a phenol ring of tyrosine but being free of L-DOPA oxidase activity is any one or more selected from SEQ ID NOS: 50, 52, and 54.
13. A betalamic acid synthesis composition for betaxanthin synthesis, including a gene shown in any one of the following items or a vector carrying the gene:
   (1) a gene encoding a polypeptide formed of an amino acid sequence set forth in SEQ ID NO: 51, 53, or 55;
   (2) a gene encoding a polypeptide that has 1 to 20 amino acids substituted, deleted, inserted, and/or added in the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
   (3) a gene encoding a polypeptide that has 90% or more homology to the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
   (4) a gene formed of DNA formed of a base sequence set forth in SEQ ID NO: 50, 52, or 54;
   (5) a gene formed of DNA that hybridizes with DNA formed of a base sequence complementary to DNA formed of the base sequence set forth in SEQ ID NO: 50, 52, or 54 under stringent conditions, and that encodes a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity;
   (6) a gene formed of DNA having a 1- to 50-base sequence substituted, deleted, inserted, and/or added in DNA formed of the base sequence set forth in SEQ ID NO: 50, 52, or 54;
   (7) a gene formed of DNA having 90% or more homology to DNA formed of the base sequence set forth in SEQ ID NO: 50, 52, or 54; and
   (8) a gene formed of DNA formed of a degenerate isomer of the base sequence set forth in SEQ ID NO: 50, 52, or 53.
14. A betalamic acid synthesis composition for betaxanthin synthesis, including a peptide represented by any one of the following amino acid sequences:
   (1) an amino acid sequence set forth in SEQ ID NO: 51, 53, or 55;
   (2) an amino acid sequence that has 1 to 20 amino acids substituted, deleted, inserted, and/or added in the amino acid sequence set forth in SEQ ID NO: 51, 53, or 55, and that forms a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity; and
   (3) an amino acid sequence that has 90% or more homology to the amino acid sequence set forth in SEQ ID NO: 51, 53, or 55, and that forms a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity.
15. A betaxanthin-producing host having introduced therein the synthesis composition of the above-mentioned item 13 or 14.
16. A betaxanthin-producing host,
   the host having introduced therein a gene encoding an enzyme having enzyme activity of hydroxylating a 3-position of a phenol ring of tyrosine but being free of L-DOPA oxidase activity, and a gene encoding an enzyme having DOPA 4,5-dioxygenase activity, and having an ability to produce tyrosine or 3-hydroxy-L-tyrosine, or
   the host having introduced therein a gene encoding an enzyme having enzyme activity of hydroxylating a 3-position of a phenol ring of tyrosine but being free of L-DOPA oxidase activity, and having enzyme activity of having DOPA 4,5-dioxygenase activity, and an ability to produce tyrosine or 3-hydroxy-L-tyrosine,
   wherein the gene encoding an enzyme having enzyme activity of hydroxylating a 3-position of a phenol ring of tyrosine but being free of L-DOPA oxidase activity is any one or more selected from the following:
      (1) a gene encoding a polypeptide formed of an amino acid sequence set forth in SEQ ID NO: 51, 53, or 55;
      (2) a gene encoding a polypeptide that has 1 to 20 amino acids substituted, deleted, inserted, and/or added in the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
      (3) a gene encoding a polypeptide that has 90% or more homology to the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
      (4) a gene formed of DNA formed of a base sequence set forth in SEQ ID NO: 50, 52, or 54;
      (5) a gene formed of DNA that hybridizes with DNA formed of a base sequence complementary to DNA formed of the base sequence set forth in SEQ ID NO: 50, 52, or 54 under stringent conditions, and that encodes a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity;
      (6) a gene formed of DNA having a 1- to 50-base sequence substituted, deleted, inserted, and/or added in DNA formed of the base sequence set forth in SEQ ID NO: 50, 52, or 54;
      (7) a gene formed of DNA having 90% or more homology to DNA formed of the base sequence set forth in SEQ ID NO: 50, 52, or 54; and
      (8) a gene formed of DNA formed of a degenerate isomer of the base sequence set forth in SEQ ID NO: 50, 52, or 54.
17. An amyioid-β polymerization inhibitor or a therapeutic or preventive agent for Alzheimer's, including any one of the following:
   (1) celosianin II;
   (2) celosianin II obtained by the method of synthesizing celosianin II of any one of the above-mentioned items 1 to 5 and 9;
   (3) celosianin II obtained from the celosianin II-producing host of the above-mentioned item 8 or 10;
   (4) amaranthin;
   (5) betanin;
   (6) ferulic acid;
   (7) a betaxanthin;
   (8) a betaxanthin obtained by the method of synthesizing a betaxanthin of the above-mentioned item 11 or 12; and
   (9) a betaxanthin obtained from the betaxanthin-producing host of the above-mentioned item 16.
18. An amyloid peptide aggregation inhibitor, including any one of the following:
   (1) celosianin II;
   (2) celosianin II obtained by the method of synthesizing celosianin II of any one of the above-mentioned items 1 to 5 and 9;
   (3) celosianin II obtained from the celosianin II-producing host of the above-mentioned item 8 or 10;
   (4) amaranthin;
   (5) betanin;
   (6) ferulic acid; and
   (7) a betaxanthin.
19. A preventive and/or therapeutic agent for a neurodegenerative disease, Alzheimer's disease, systemic amyloidosis, Parkinson's disease, Huntington's disease, a prion disease, multiple sclerosis, or amyotrophic lateral sclerosis, the preventive and/or therapeutic agent including the amyloid peptide aggregation inhibitor of the above-mentioned item 18 as an active ingredient.
20. An HIV-1 protease activity inhibitor, including any one of the following:
   (1) celosianin II;
   (2) celosianin II obtained by the method of synthesizing celosianin II of any one of the above-mentioned items 1 to 5 and 9; and
   (3) celosianin II obtained from the celosianin II-producing host of the above-mentioned item 8 or 10.

### Advantageous Effects of Invention

According to the present invention, the method of synthesizing celosianin II, the method of synthesizing a betaxanthin, the amyioid-β polymerization inhibitor or the therapeutic or preventive agent for Alzheimer's, the amyloid peptide aggregation inhibitor, and the HIV-1 protease activity inhibitor can be provided.

### Brief Description of Drawings

FIG. 1 is an illustration of the structures of betalain pigments.
FIG. 2 shows the accumulation of betalain pigments in hypocotyls.
FIG. 3 shows the results of evaluation of the stabilities of betalain pigments.
FIG. 4 shows the results of evaluation of inhibitory activity on HIV-1 protease activity.
FIG. 5 shows the results of evaluation of the polymerization inhibitory activities of betalain pigments on amyloid-β.
FIG. 6 is an overview of the results of a search for a celosianin II synthetase gene.
FIG. 7 is a scheme for a biosynthesis pathway of betalain pigments.
FIG. 8 is a schematic diagram of celosianin II biosynthesis.
FIG. 9 is a scheme for a biosynthesis pathway of a betaxanthin.
FIG. 10 shows the results of activity evaluation in tobacco BY2 cells.
FIG. 11 shows the results of evaluation of the polymerization inhibitory activity of a betaxanthin on amyloid-β.
FIGS. 12 show the results of observation of amyloid peptide aggregation inhibition using an electron microscope. A and B: 25 µM Aβ40+water, C and D: 25 µM Aβ40+50 µM betanin, E: 25 µM Aβ40+50 µM amaranthin, F: 25 µM Aβ40+50 µM celosianin II, G: 25 µM Aβ40+50 µM ferulic acid, H: 25 µM Aβ40+50 µM betaxanthin. Scale bars: 200 µm.
FIG. 13A shows the ratio of nematodes that are not paralyzed in a group of nematodes fed with dextrin (control), and FIG. 13B shows the ratio of nematodes that are not paralyzed in a group of nematodes fed with betanin. Vertical axis: ratio of nematodes that are not paralyzed, horizontal axis: number of days of adulthood. FIG. 13C shows nematodes grown with dextrin-mixed food, and FIG. 13D shows normal nematodes.
FIG. 14 shows results for an amaranth plant body caused to express XM_021915966 in celosianin II synthesis activity evaluation. The gene XM_021915966 was transiently expressed, and pigments were analyzed by HPLC. Infected plant body: amaranth (which does not accumulate celosianin II). In the amaranth plant body caused to express XM_021915966, the accumulation of celosianin II was able to be newly found.
FIG. 15 shows results for quinoa caused to transiently express XM_021915966 in celosianin II synthesis activity evaluation. The candidate gene was transiently expressed, and pigments were analyzed by HPLC. Infected plant body: quinoa. In the quinoa caused to transiently express XM_021915966, the accumulation of celosianin II was increased about 2-fold.

### Description of Embodiments

The present invention relates to a method of synthesizing celosianin II, a celosianin II synthesis composition, and a celosianin II-producing host.

The present invention relates to a method of synthesizing a betaxanthin, a betalamic acid synthesis composition for betaxanthin synthesis, and a betaxanthin-producing host.

### (Betalain Pigment)

Betalain pigments in the present invention are classified into betaxanthins and betacyanins on the basis of their structural features. The betaxanthins exhibit yellow colors and the betacyanins exhibit red-violet colors, and hence the betaxanthins and the betacyanins have heretofore been utilized as natural colorants. The term "betacyanins" collectively refers to a group of compounds in each of which a sugar is glycosidically bonded to a phenolic hydroxy group of betanidin.

### (Celosianin II Synthesis System)

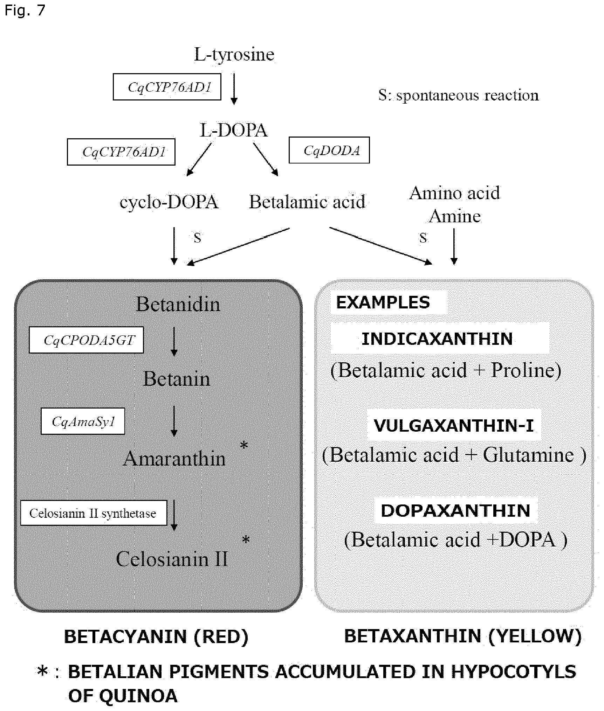

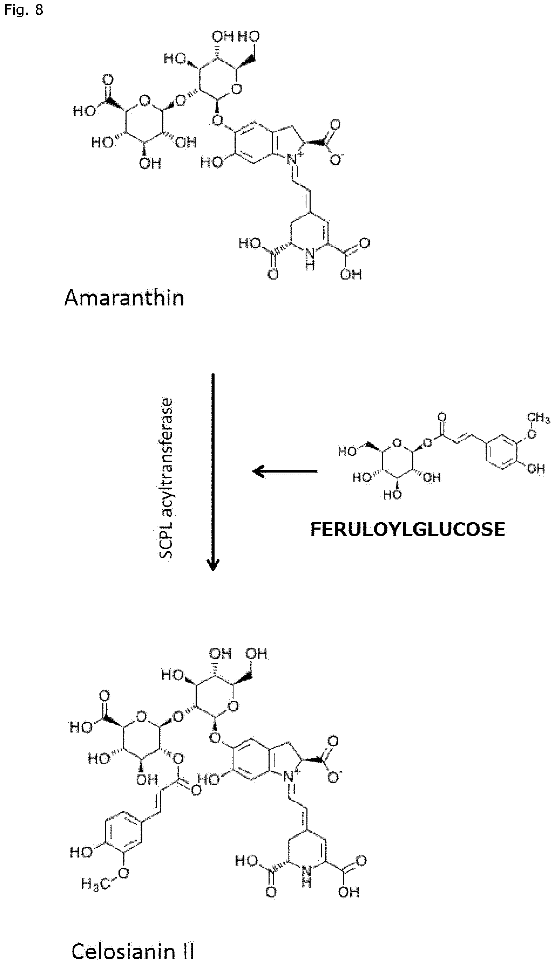

Schemes for betalain pigment biosynthetic pathways in the present invention are illustrated in FIG. 7 and FIG. 8.

As apparent from the illustrations in FIG. 7 and FIG. 8, when a host having an ability to produce tyrosine (L-tyrosine) or 3-hydroxy-L-tyrosine (L-DOPA), and an amino acid or an amine (e.g., putrescine, spermidine, or spermine) (including a host capable of incorporating these compounds from outside) has the following enzyme activities, celosianin II can be synthesized.

Enzyme (e.g., CqCYP76AD1) activity of hydroxylating the 3-position of the phenol ring of tyrosine, L-DOPA oxidase (e.g., CqCYP76AD1) activity and/or DOPA 4,5-dioxygenase (e.g., CqDODA-1) activity, the activity of an enzyme having activity of glycosylating a phenolic hydroxy group {e.g., an enzyme having betanidin 5-O-glucosyltransferase (5GT) activity (e.g., Cyclo-DOPA 5-O-glucosyltransferase, CqCDOPA5GT)}, enzyme (e.g., CqAmaSyl1) activity of having activity of adding glucuronic acid, and celosianin II synthetase activity of the present invention.

CqCYP76AD1 has both of the following properties: enzyme activity of hydroxylating the 3-position of the phenol ring of tyrosine; and L-DOPA oxidase activity.

According to the results of Examples to be described below, a plant body that is a host having introduced therein a gene group of a gene encoding an enzyme (CqCYP76AD1-1) having activity of hydroxylating the 3-position of the phenol ring of tyrosine, a gene encoding an enzyme having L-DOPA oxidase activity and/or a gene encoding an enzyme (CqDODA-1) having DOPA 4,5-dioxygenase activity, a gene encoding an enzyme (CqCDOPA5GT) having activity of glycosylating a phenolic hydroxy group, a gene encoding an enzyme (CqAmaSy1) having activity of adding glucuronic acid, and a gene encoding the celosianin II synthetase of the present invention has an ability to synthesize celosianin II, which is a betalain pigment.

As apparent from the illustrations in FIG. 7 and FIG. 8, the present invention also encompasses the following celosianin II synthesis method.
(1) Amaranthin is brought into contact with the celosianin II synthetase of the present invention. As required, a metal ion is added.
(2) Betanin is brought into contact with an enzyme having activity of adding glucuronic acid to provide amaranthin, which is then brought into contact with the celosianin II synthetase of the present invention. As required, a metal ion is added.
(3) Betanidin is brought into contact with an enzyme having activity of glycosylating a phenolic hydroxy group to provide betanin, which is then brought into contact with an enzyme having activity of adding glucuronic acid to provide amaranthin, which is then brought into contact with the celosianin II synthetase of the present invention. As required, a metal ion is added.

### (Enzyme for Hydroxylating 3-Position of Phenol Ring of Tyrosine)

The enzyme for hydroxylating the 3-position of the phenol ring of tyrosine in the present invention has activity of allowing a hydroxy group to be added to the 3-position of the phenol ring that tyrosine has, and the enzyme may be derived from any species as long as the enzyme has the activity.

Examples of the enzyme for hydroxylating the 3-position of the phenol ring of tyrosine include tyrosinase, cytochromes P450 (in particular, CYP76AD1, CYP76AD2, CYP76AD3, and the like), and catechol oxidase. Of those, CqCYP76AD1 (base sequence: SEQ ID NO: 17, amino acid sequence: SEQ ID NO: 18, accession number: XP_021769302) is preferred.

### (Enzyme Having L-DOPA Oxidase Activity)

The enzyme having L-DOPA oxidase activity in the present invention has activity of converting L-DOPA into cyclo-DOPA, and may be derived from any species as long as the enzyme has the activity.

Examples of the enzyme having L-DOPA oxidase activity include tyrosinase and cytochromes P450, including a subfamily α of a CYP76AD group, such as CYP76AD1, CYP76AD2, and CYP76AD3. Of those, CqCYP76AD1 (base sequence: SEQ ID NO: 17, amino acid sequence: SEQ ID NO: 18) is preferred.

### (Enzyme having Activity of Glycosylating Phenolic Hydroxy Group)

The enzyme having activity of glycosylating a phenolic hydroxy group in the present invention has an ability to synthesize betanin through enzyme activity of having activity of glycosylating a phenolic hydroxy group, i.e., activity of glycosylating a phenolic hydroxy group present at the 5-position or the 6-position of a cyclo-DOPA skeleton, and may be derived from any species as long as the enzyme has the activity.

The enzyme having activity of glycosylating a phenolic hydroxy group encompasses a betanidin-to-betanin synthetase.

Examples of the enzyme having activity of glycosylating a phenolic hydroxy group include cyclo-DOPA 5-O-glucosyltransferase (CDOPA5GT), betanidin 5-O-glucosyltransferase (B5GT), and betanidin 6-O-glucosyltransferase. Of those, CqCDOPA5GT (base sequence: SEQ ID NO: 19, amino acid sequence: SEQ ID NO: 20, accession number: XP_021748306) is preferred.

### (Betanidin-to-Gomphrenin-I Synthetase)

The betanidin-to-gomphrenin-I synthetase in the present invention is not particularly limited as long as gomphrenin-I can be synthesized from betanidin, and examples thereof may include betanidin 6-O-glucosyltransferase (6GT (B6GT): SEQ ID NOS: 21 and 22) and cyclo-DOPA 6-O-glucosyltransferase (CDOPA6GT) (see: Substrate specificity and sequence analysis define a polyphyletic origin of betanidin 5- and 6-O-glucosyltransferase from *Dorotheanthus bellidiformis*. planta 214, 492-495).

### (Enzyme having Activity of adding Glucuronic Acid)

The enzyme having activity of adding glucuronic acid in the present invention has an ability to synthesize amaranthin through enzyme activity of having activity of adding glucuronic acid, and may be derived from any species as long as the enzyme has the activity.

The enzyme having activity of adding glucuronic acid encompasses, for example, a betanin-to-amaranthin synthetase, and an enzyme having activity of synthesizing amaranthin by bonding glucuronic acid to betanin (e.g., activity of synthesizing UDP-glucoronate (betanin beta-D-glucuronosyltransferase)).

Examples of the gene encoding the enzyme having activity of adding glucuronic acid (amaranthin synthetase gene) may include the following:
(1) a gene encoding a polypeptide formed of an amino acid sequence set forth in SEQ ID NO: 24, 26, 28, 30, 32, or 34;
(2) a gene encoding a polypeptide that has 1 to 20 amino acids substituted, deleted, inserted, and/or added in the amino acid sequence set forth in SEQ ID NO: 24, 26, 28, 30, 32, or 34, and that has a substantially equivalent ability to synthesize amaranthin to that of the amino acid sequence set forth in SEQ ID NO: 24, 26, 28, 30, 32, or 34;
(3) a gene encoding a polypeptide that has 90% or more homology to the amino acid sequence set forth in SEQ ID NO: 24, 26, 28, 30, 32, or 34, and that has a substantially equivalent ability to synthesize amaranthin to that of the amino acid sequence set forth in SEQ ID NO: 24, 26, 28, 30, 32, or 34;
(4) a gene formed of DNA formed of a base sequence set forth in SEQ ID NO: 23, 25, 27, 29, 31, or 33;
(5) a gene formed of DNA that hybridizes with DNA formed of a base sequence complementary to DNA formed of the base sequence set forth in SEQ ID NO: 23, 25, 27, 29, 31, or 33 under stringent conditions, and that encodes a polypeptide having an ability to synthesize amaranthin;
(6) a gene formed of DNA having a 1- to 50-base sequence substituted, deleted, inserted, and/or added in DNA formed of the base sequence set forth in SEQ ID NO: 23, 25, 27, 29, 31, or 33;
(7) a gene formed of DNA having 90% or more homology to DNA formed of the base sequence set forth in SEQ ID NO: 23, 25, 27, 29, 31, or 33; and
(8) a gene formed of DNA formed of a degenerate isomer of the base sequence set forth in SEQ ID NO: 23, 25, 27, 29, 31, or 33.

The gene of the above-mentioned item (2) is a gene encoding a polypeptide having introduced therein such a mutation as not to cause the loss of the ability to synthesize amaranthin. Such mutation encompasses an artificial mutation as well as a naturally occurring mutation. As means for causing the artificial mutation, there may be given, for example, a site-directed mutagenesis method (Nucleic Acids Res. 10, 6487-6500, 1982). The number of mutated amino acids is generally 20 or less, preferably 10 or less, more preferably 5 or less, most preferably 3. Whether or not the polypeptide having introduced therein the mutation retains the ability to synthesize amaranthin can be found by, for example, introducing a gene encoding the polypeptide having introduced therein the mutation into a plant body or the like, and checking the ability to synthesize amaranthin in the plant body.

With regard to the gene of the above-mentioned item (3), the "substantially equivalent ability to synthesize amaranthin to that of the amino acid sequence set forth in SEQ ID NO: 24, 26, 28, 30, 32, or 34" may be stronger or weaker in degree of the action as compared to the substantially equivalent ability to synthesize amaranthin to that of the amino acid sequence set forth in SEQ ID NO: 24, 26, 28, 30, 32, or 34. The degree of the action may be, for example, about 50%, about 60%, about 70%, about 80%, about 90%, about 100%, about 110%, about 120%, about 130%, about 140%, or about 150% as compared to the ability of the amino acid sequence set forth in SEQ ID NO: 24, 26, 28, 30, 32, or 34 to synthesize amaranthin.

In addition, the identity may be calculated using the Basic Local Alignment Search Tool at the National Center for Biological Information (BLAST) or the like (using, for example, default, namely initially set, parameters).

The gene of the above-mentioned item (5) is a gene obtained by utilizing hybridization between DNAs. The term "stringent conditions" in this gene refers to conditions under which only specific hybridization occurs and non-specific hybridization does not occur. Such conditions are generally conditions such as hybridization in a buffer containing 5xSSC and 1% SDS at 37°C and washing treatment with a buffer containing 1×SSC and 0.1% SDS at 37°C, preferably conditions such as hybridization in a buffer containing 5xSSC and 1% SDS at 42°C and washing treatment with a buffer containing 0.5xSSC and 0.1% SDS at 42°C, more preferably conditions such as hybridization in a buffer containing 5xSSC and 1% SDS at 65°C and washing treatment with a buffer containing 0.2xSSC and 0.1% SDS at 65°C. Whether or not DNA obtained by utilizing hybridization encodes a polypeptide having activity can be found by, for example, introducing the DNA into a plant body or the like, and checking the ability of the plant body to synthesize amaranthin. The DNA obtained by hybridization generally has high identity to the gene of the above-mentioned item (4) (SEQ ID NO: 23, 25, 27, 29, 31, or 33). The "high identity" refers to 90% or more identity, preferably 95% or more identity, more preferably 98% or more identity.

The gene of the above-mentioned item (6) is a gene formed of DNA having a 1- to 50-base sequence, preferably a 1- to 30-base sequence, more preferably a 1- to 20-base sequence, most preferably a 1- to 10-base sequence, even most preferably a 1- to 5-base sequence substituted, deleted, inserted, and/or added in DNA formed of the base sequence set forth in SEQ ID NO: 23, 25, 27, 29, 31, or 33.

The gene of the above-mentioned item (7) is a gene formed of DNA having 90% or more, preferably 93% or more, more preferably 95% or more, most preferably 98% or more identity to DNA formed of the base sequence set forth in SEQ ID NO: 23, 25, 27, 29, 31, or 33.

The enzyme having enzyme activity of having activity of adding glucuronic acid in the present invention has any one or more amino acid sequences selected from the following:
(1) an amino acid sequence set forth in SEQ ID NO: 24, 26, 28, 30, 32, or 34;
(2) an amino acid sequence that has 1 to 20 amino acids substituted, deleted, inserted, and/or added in the amino acid sequence set forth in SEQ ID NO: 24, 26, 28, 30, 32, or 34, and that forms a polypeptide having a substantially equivalent ability to synthesize amaranthin to that of the amino acid sequence set forth in SEQ ID NO: 57, 59, 61, 63, 65, 67, 69, 2, 4, 6, 8, 10, 12, 14, or 16; and
(3) an amino acid sequence that has 90% or more homology to the amino acid sequence set forth in SEQ ID NO: 24, 26, 28, 30, 32, or 34, and that forms a polypeptide having a substantially equivalent ability to synthesize amaranthin to that of the amino acid sequence set forth in SEQ ID NO: 24, 26, 28, 30, 32, or 34.

In the introduction of a mutation into a peptide, for example, a substitution between homologous amino acids (e.g., polar amino acids, non-polar amino acids, hydrophobic amino acids, hydrophilic amino acids, positively charged amino acids, negatively charged amino acids, and aromatic amino acids) is easily conceivable from the viewpoint of preventing basic properties (e.g., physical properties, function, physiological activity, or immunological activity) of the peptide from being changed.

### (Enzyme Having DOPA 4,5-Dioxygenase Activity)

The enzyme having DOPA 4,5-dioxygenase activity in the present invention has an ability to convert L-DOPA into 4,5-seco-DOPA through activity of catalyzing extradiol cleavage of L-3,4-dihydroxyphenylalanine. Further, 4,5-seco-DOPA converts into betalamic acid through a spontaneous reaction.

The gene encoding the enzyme having DOPA 4,5-dioxygenase activity in the present invention is any one or more selected from the following:
(1) a gene encoding a polypeptide formed of an amino acid sequence set forth in SEQ ID NO: 35 (accession number: XP_021769303), 37 (accession number: XP_021769301), 39, 41, 43, 45, or 47;
(2) a gene encoding a polypeptide that has 1 to 20 amino acids substituted, deleted, inserted, and/or added in the amino acid sequence set forth in SEQ ID NO: 35, 37, 39, 41, 43, 45, or 47, and that has a substantially equivalent ability to convert L-DOPA into 4,5-seco-DOPA to that of the amino acid sequence set forth in SEQ ID NO: 35, 37, 39, 41, 43, or 45;
(3) a gene encoding a polypeptide that has 90% or more homology to the amino acid sequence set forth in SEQ ID NO: 35, 37, 39, 41, 43, 45, or 47, and that has a substantially equivalent ability to convert L-DOPA into 4,5-seco-DOPA to that of the amino acid sequence set forth in SEQ ID NO: 35, 37, 39, 41, 43, 45, or 47;
(4) a gene formed of DNA formed of a base sequence set forth in SEQ ID NO: 36, 38, 40, 42, 44, 46, or 48;
(5) a gene formed of DNA that hybridizes with DNA formed of a base sequence complementary to DNA formed of the base sequence set forth in SEQ ID NO: 36, 38, 40, 42, 44, 46, or 48 under stringent conditions, and that encodes a polypeptide having an ability to convert L-DOPA into 4,5-seco-DOPA;
(6) a gene formed of DNA having a 1- to 50-base sequence substituted, deleted, inserted, and/or added in DNA formed of the base sequence set forth in SEQ ID NO: 36, 38, 40, 42, 44, 46, or 48;
(7) a gene formed of DNA having 90% or more homology to DNA formed of the base sequence set forth in SEQ ID NO: 36, 38, 40, 42, 44, 46, or 48;
(8) the gene of any one or more of the above-mentioned items (1) to (7) including a gene encoding a polypeptide formed of an amino acid sequence set forth in SEQ ID NO: 33; and
(9) a gene formed of DNA formed of a degenerate isomer of the base sequence set forth in SEQ ID NO: 36, 38, 40, 42, 44, 46, or 48.

The gene of the above-mentioned item (2) is a gene encoding a polypeptide having introduced therein such a mutation as not to cause the loss of the ability to convert L-DOPA into 4,5-seco-DOPA. Such mutation may be introduced by the method described above.

With regard to the gene of the above-mentioned item (3), the "substantially equivalent ability to convert L-DOPA into 4,5-seco-DOPA to that of the amino acid sequence set forth in SEQ ID NO: 35, 37, 39, 41, 43, 45, or 47" may be stronger or weaker in degree of the action as compared to the ability of the amino acid sequence set forth in SEQ ID NO: 35, 37, 39, 41, 43, 45, or 47 to convert L-DOPA into 4,5-seco-DOPA. The degree of the action may be, for example, about 50%, about 60%, about 70%, about 80%, about 90%, about 100%, about 110%, about 120%, about 130%, about 140%, or about 150% as compared to the ability of the amino acid sequence set forth in SEQ ID NO: 35, 37, 39, 41, 43, 45, or 47 to convert L-DOPA into 4,5-seco-DOPA.

The gene of the above-mentioned item (5) is a gene obtained by utilizing hybridization between DNAs. DNA obtained by hybridization generally has high identity to the gene of the above-mentioned item (4) (SEQ ID NO: 18, 20, 22, 24, 26, 28, or 48). The "high identity" refers to 90% or more identity, preferably 95% or more identity, more preferably 98% or more identity.

The gene of the above-mentioned item (6) is a gene formed of DNA having a 1- to 50-base sequence, preferably a 1- to 30-base sequence, more preferably a 1- to 20-base sequence, most preferably a 1- to 10-base sequence, even most preferably a 1- to 5-base sequence substituted, deleted, inserted, and/or added in DNA formed of the base sequence set forth in SEQ ID NO: 36, 38, 40, 42, 44, 46, or 48.

The gene of the above-mentioned item (7) is a gene formed of DNA having 90% or more, preferably 93% or more, more preferably 95% or more, most preferably 98% or more identity to DNA formed of the base sequence set forth in SEQ ID NO: 36, 38, 40, 42, 44, 46, or 48.

The enzyme having DOPA 4,5-dioxygenase activity in the present invention is any one or more selected from the following:
(1) an amino acid sequence set forth in SEQ ID NO: 35, 37, 39, 41, 43, 45, or 47;
(2) an amino acid sequence that has 1 to 20 amino acids substituted, deleted, inserted, and/or added in the amino acid sequence set forth in SEQ ID NO: 35, 37, 39, 41, 43, 45, or 47, and that has a substantially equivalent ability to convert L-DOPA into 4,5-seco-DOPA to that of the amino acid sequence set forth in SEQ ID NO: 35, 37, 39, 41, 43, 45, or 47;
(3) an amino acid sequence that has 90% or more homology to the amino acid sequence set forth in SEQ ID NO: 35, 37, 39, 41, 43, 45, or 47, and that has a substantially equivalent ability to convert L-DOPA into 4,5-seco-DOPA to that of an amino acid sequence set forth in SEQ ID NO: 57, 59, 61, 63, 65, 67, 69, or 2; and
(4) the amino acid sequence of any one or more of the above-mentioned items (1) to (3) including an amino acid sequence set forth in SEQ ID NO: 33.

In the introduction of a mutation into a peptide, for example, a substitution between homologous amino acids (e.g., polar amino acids, non-polar amino acids, hydrophobic amino acids, hydrophilic amino acids, positively charged amino acids, negatively charged amino acids, and aromatic amino acids) is easily conceivable from the viewpoint of preventing basic properties (e.g., physical properties, function, physiological activity, or immunological activity) of the peptide from being changed.

In particular, an amino acid sequence set forth in SEQ ID NO: 49 contains the sequence of an active site. When a mutation is introduced into this sequence, the ability to convert L-DOPA into 4,5-seco-DOPA is highly liable to be lost, and hence it is preferred that the mutation be introduced at an amino acid outside those domains.

### (Celosianin II Synthetase)

The celosianin II synthetase in the present invention is not particularly limited as long as the synthetase has an ability to synthesize celosianin II through activity of synthesizing celosianin II from amaranthin. The celosianin II synthetase has activity of adding an aromatic carboxylic acid other than ferulic acid (e.g., caffeic acid or coumaric acid) to amaranthin as well as synthesizing celosianin II by adding ferulic acid to amaranthin. Further, the celosianin II synthetase has activity of adding an aromatic carboxylic acid to a betacyanin other than amaranthin (e.g., betanin or gomphrenin) as well.

Examples of the gene encoding the celosianin II synthetase (celosianin II synthetase gene) may include the following:
(1) a gene encoding a polypeptide formed of an amino acid sequence set forth in SEQ ID NO: 57, 59, 61, 63, 65, 67, 69, 2, 4, 6, 8, 10, 12, 14, or 16;
(2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(4) a gene formed of DNA formed of a base sequence set forth in SEQ ID NO: 56, 58, 60, 62, 64, 66, 68, 1, 3, 5, 7, 9, 11, 13, or 15;
(5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
(6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
(7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
(8) a gene formed of DNA formed of a degenerate isomer of the base sequence set forth in SEQ ID NO: 56, 58, 60, 62, 64, 66, 68, 1, 3, 5, 7, 9, 11, 13, or 15.

The gene of the above-mentioned item (2) is a gene encoding a polypeptide having introduced therein such a mutation as not to cause the loss of the ability to synthesize celosianin II. Such mutation encompasses an artificial mutation as well as a naturally occurring mutation. As means for causing the artificial mutation, there may be given, for example, a site-directed mutagenesis method (Nucleic Acids Res. 10, 6487-6500, 1982). The number of mutated amino acids is generally 20 or less, preferably 10 or less, more preferably 5 or less, most preferably 3. Whether or not the polypeptide having introduced therein the mutation retains the ability to synthesize celosianin II can be found by, for example, introducing a gene encoding the polypeptide having introduced therein the mutation into a plant body or the like, and checking the ability to synthesize celosianin II in the plant body.

With regard to the gene of the above-mentioned item (3), the "substantially equivalent ability to synthesize celosianin II to that of the amino acid sequence set forth in SEQ ID NO: 57, 59, 61, 63, 65, 67, 69, 2, 4, 6, 8, 10, 12, 14, or 16" may be stronger or weaker in degree of the action as compared to the substantially equivalent ability to synthesize celosianin II to that of the amino acid sequence set forth in SEQ ID NO: 57, 59, 61, 63, 65, 67, 69, 2, 4, 6, 8, 10, 12, 14, or 16. The degree of the action may be, for example, about 50%, about 60%, about 70%, about 80%, about 90%, about 100%, about 110%, about 120%, about 130%, about 140%, or about 150% as compared to the ability of the amino acid sequence set forth in SEQ ID NO: 57, 59, 61, 63, 65, 67, 69, 2, 4, 6, 8, 10, 12, 14, or 16 to synthesize celosianin II.

In addition, the identity may be calculated using the Basic Local Alignment Search Tool at the National Center for Biological Information (BLAST) or the like (using, for example, default, namely initially set, parameters).

The gene of the above-mentioned item (5) is a gene obtained by utilizing hybridization between DNAs. The term "stringent conditions" in this gene refers to conditions under which only specific hybridization occurs and non-specific hybridization does not occur. Such conditions are generally conditions such as hybridization in a buffer containing 5xSSC and 1% SDS at 37°C and washing treatment with a buffer containing 1×SSC and 0.1% SDS at 37°C, preferably conditions such as hybridization in a buffer containing 5xSSC and 1% SDS at 42°C and washing treatment with a buffer containing 0.5xSSC and 0.1% SDS at 42°C, more preferably conditions such as hybridization in a buffer containing 5xSSC and 1% SDS at 65°C and washing treatment with a buffer containing 0.2xSSC and 0.1% SDS at 65°C. Whether or not DNA obtained by utilizing hybridization encodes a polypeptide having activity can be found by, for example, introducing the DNA into a plant body or the like, and checking the ability of the plant body to synthesize celosianin II. The DNA obtained by hybridization generally has high identity to the gene of the above-mentioned item (4) (SEQ ID NO: 56, 58, 60, 62, 64, 66, 68, 1, 3, 5, 7, 9, 11, 13, or 15). The "high identity" refers to 90% or more identity, preferably 95% or more identity, more preferably 98% or more identity.

The gene of the above-mentioned item (6) is a gene formed of DNA having a 1- to 50-base sequence, preferably a 1- to 30-base sequence, more preferably a 1- to 20-base sequence, most preferably a 1- to 10-base sequence, even most preferably a 1- to 5-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15.

The gene of the above-mentioned item (7) is a gene formed of DNA having 90% or more, preferably 93% or more, more preferably 95% or more, most preferably 98% or more identity to DNA formed of the base sequence set forth in SEQ ID NO: 56, 58, 60, 62, 64, 66, 68, 1, 3, 5, 7, 9, 11, 13, or 15.

The enzyme having celosianin II synthetase activity in the present invention has any one or more amino acid sequences selected from the following:
(1) an amino acid sequence set forth in SEQ ID NO: 57, 59, 61, 63, 65, 67, 69, 2, 4, 6, 8, 10, 12, 14, or 16;
(2) an amino acid sequence that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that forms a polypeptide having a substantially equivalent ability to synthesize celosianin II to that of the amino acid sequence set forth in SEQ ID NO: 57, 59, 61, 63, 65, 67, 69, 2, 4, 6, 8, 10, 12, 14, or 16; and
(3) an amino acid sequence that has 90% or more homology to the amino acid sequence set forth in SEQ ID NO: 57, 59, 61, 63, 65, 67, 69, 2, 4, 6, 8, 10, 12, 14, or 16, and that forms a polypeptide having a substantially equivalent ability to synthesize celosianin II to that of the amino acid sequence set forth in SEQ ID NO: 57, 59, 61, 63, 65, 67, 69, 2, 4, 6, 8, 10, 12, 14, or 16.

In the introduction of a mutation into a peptide, for example, a substitution between homologous amino acids (e.g., polar amino acids, non-polar amino acids, hydrophobic amino acids, hydrophilic amino acids, positively charged amino acids, negatively charged amino acids, and aromatic amino acids) is easily conceivable from the viewpoint of preventing basic properties (e.g., physical properties, function, physiological activity, or immunological activity) of the peptide from being changed.

### (Method of Synthesizing Celosianin II)

A method of synthesizing celosianin II of the present invention may be exemplified by the following:
(1) culturing a host that has introduced therein a gene encoding a celosianin II synthetase, a gene encoding an enzyme having activity of hydroxylating a 3-position of a phenol ring of tyrosine, a gene encoding an enzyme having L-DOPA oxidase activity and/or a gene encoding an enzyme having DOPA 4,5-dioxygenase activity, a gene encoding an enzyme having activity of glycosylating a phenolic hydroxy group, and a gene encoding an enzyme having activity of adding glucuronic acid, and that has an ability to produce tyrosine or L-DOPA, and extracting celosianin II from the host after the culturing; or
(2) culturing a host that has introduced therein a gene encoding a celosianin II synthetase, and that has enzyme activity of having activity of hydroxylating a 3-position of a phenol ring of tyrosine, L-DOPA oxidase activity and/or DOPA 4,5-dioxygenase activity, enzyme activity of having activity of glycosylating a phenolic hydroxy group, enzyme activity of having activity of adding glucuronic acid, and an ability to produce tyrosine or 3-hydroxy-L-tyrosine, and extracting celosianin II from the host after the culturing.

### (Host)

The host to be used in the method of synthesizing celosianin II or a betaxanthin of the present invention is not particularly limited as long as the host has an ability to produce tyrosine or 3-hydroxy-L-tyrosine. For example, there may be used a synthesis system known per se, such as a recombinant *Escherichia coli* protein synthesis system, an insect protein synthesis system, a yeast protein synthesis system, a plant cell protein synthesis system, a cell-free protein synthesis system, a plant protein synthesis system, or animal cultured cells.

A method known per se may be used as a method of introducing each gene into the host to be used in the method of synthesizing celosianin II or a betaxanthin of the present invention. For example, the gene may be introduced into the host using a vector carrying the gene.

As the vector, a viral vector known per se, in particular, a plant virus vector (e.g., a vector derived from a virus belonging to the genus *Tobamovirus,* a tobacco mosaic virus vector, or a tomato mosaic virus vector) may be utilized.

In addition, an *Agrobacterium* method involving using a Ti plasmid may be utilized.

### (Method of Introducing Each Gene into Host)

In the method of synthesizing celosianin II or a betaxanthin of the present invention, with regard to the method of introducing each gene into the host, the gene may be introduced into a plant body by a method known per se. For example, each gene may be introduced into the host by applying a solution containing a plant virus vector carrying each gene to a leaf, a stalk, a root, an ear, or the like of the plant body. Other examples of the method may include a particle gun method and an *Agrobacterium* method.

### (Method of Introducing Each Protein into Host)

In the method of synthesizing celosianin II or a betaxanthin of the present invention, with regard to a method of introducing each protein into the host (in particular, a plant body), the protein may be introduced into the plant body by a method known per se. For example, each protein may be introduced into the host by applying a solution containing each protein to a leaf, a stalk, a root, an ear, or the like of the plant body. Other examples of the method may include a particle gun method and an *Agrobacterium* method.

### (Celosianin II-producing Host)

A celosianin II-producing host of the present invention has, for example, any one or more of the following configurations and features:
(1) the host has introduced therein a gene encoding a celosianin II synthetase, a gene encoding an enzyme having activity of hydroxylating a 3-position of a phenol ring of tyrosine, a gene encoding an enzyme having L-DOPA oxidase activity and/or a gene encoding an enzyme having DOPA 4,5-dioxygenase activity, a gene encoding an enzyme having activity of glycosylating a phenolic hydroxy group, and a gene encoding an enzyme having activity of adding glucuronic acid, and has an ability to produce tyrosine or 3-hydroxy-L-tyrosine;
(2) the host has introduced therein a gene encoding a celosianin II synthetase, and has enzyme activity of having activity of hydroxylating a 3-position of a phenol ring of tyrosine, L-DOPA oxidase activity and/or DOPA 4,5-dioxygenase activity, enzyme activity of having activity of glycosylating a phenolic hydroxy group, enzyme activity of having activity of adding glucuronic acid, and an ability to produce tyrosine or 3-hydroxy-L-tyrosine;
(3) the host has introduced therein a gene encoding a celosianin II synthetase, and has an ability to produce amaranthin;
(4) the host has introduced therein a gene encoding a celosianin II synthetase and a gene encoding an enzyme having activity of adding glucuronic acid, and has an ability to produce betanin;
(5) the host has introduced therein a gene encoding a celosianin II synthetase, and has enzyme activity of having activity of adding glucuronic acid and an ability to produce betanin;
(6) the host has introduced therein a gene encoding a celosianin II synthetase, a gene encoding an enzyme having activity of glycosylating a phenolic hydroxy group, and a gene encoding an enzyme having activity of adding glucuronic acid, and has an ability to produce betanidin;
(7) the host has introduced therein a gene encoding a celosianin II synthetase, and has enzyme activity of having activity of glycosylating a phenolic hydroxy group, enzyme activity of having activity of adding glucuronic acid, and an ability to produce betanidin; and
(8) the host has introduced therein a celosianin II synthesis composition.

### (Celosianin II Synthesis Composition)

A celosianin II synthesis composition (celosianin II synthesis agent or celosianin II synthetase agent) of the present invention contains a gene shown in any one of the following items or a vector carrying the gene:
(1) a gene encoding a polypeptide formed of an amino acid sequence set forth in SEQ ID NO: 57, 59, 61, 63, 65, 67, 69, 2, 4, 6, 8, 10, 12, 14, or 16;
(2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(4) a gene formed of DNA formed of a base sequence set forth in SEQ ID NO: 56, 58, 60, 62, 64, 66, 68, 1, 3, 5, 7, 9, 11, 13, or 15;
(5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
(6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
(7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
(8) a gene formed of DNA formed of a degenerate isomer of the base sequence set forth in SEQ ID NO: 56, 58, 60, 62, 64, 66, 68, 1, 3, 5, 7, 9, 11, 13, or 15.

Alternatively, the celosianin II synthesis composition of the present invention has a peptide represented by an amino acid sequence of any one of the following items (1) to (3):
(1) an amino acid sequence set forth in SEQ ID NO: 57, 59, 61, 63, 65, 67, 69, 2, 4, 6, 8, 10, 12, 14, or 16;
(2) an amino acid sequence that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that forms a polypeptide having a substantially equivalent ability to synthesize celosianin II to that of the amino acid sequence set forth in SEQ ID NO: 57, 59, 61, 63, 65, 67, 69, 2, 4, 6, 8, 10, 12, 14, or 16; and
(3) an amino acid sequence that has 90% or more homology to the amino acid sequence set forth in SEQ ID NO: 57, 59, 61, 63, 65, 67, 69, 2, 4, 6, 8, 10, 12, 14, or 16, and that forms a polypeptide having a substantially equivalent ability to synthesize celosianin II to that of the amino acid sequence set forth in SEQ ID NO: 57, 59, 61, 63, 65, 67, 69, 2, 4, 6, 8, 10, 12, 14, or 16.

### (Amyloid-β Polymerization Inhibitor or Therapeutic or Preventive Agent for Alzheimer's)

An amyioid-β polymerization inhibitor or a therapeutic or preventive agent for Alzheimer's of the present invention contains any one or more of the following:
(1) celosianin II;
(2) celosianin II obtained by the method of synthesizing celosianin II described herein;
(3) celosianin II obtained from the celosianin II-producing host described herein;
(4) amaranthin;
(5) betanin;
(6) ferulic acid;
(7) a betaxanthin;
(8) a betaxanthin obtained by the method of synthesizing a betaxanthin described herein; and
(9) a betaxanthin obtained from the betaxanthin-producing host described herein.

### (HIV-1 Protease Activity Inhibitor)

An HIV-1 protease activity inhibitor of the present invention contains any one or more of the following:
(1) celosianin II;
(2) celosianin II obtained by the method of synthesizing celosianin II described herein; and
(3) celosianin II obtained from the celosianin II-producing host described herein.

### (Amyloid Peptide Aggregation Inhibitor)

An amyloid peptide aggregation inhibitor of the present invention contains any one or more of the following:
(1) celosianin II;
(2) celosianin II obtained by the method of synthesizing celosianin II described herein;
(3) celosianin II obtained from the celosianin II-producing host described herein;
(4) amaranthin;
(5) betanin;
(6) ferulic acid; and
(7) a betaxanthin.

It has been able to be recognized through Examples to be described later that the amyloid peptide aggregation inhibitor of the present invention inhibits the formation of aggregates of an amyloid peptide, thereby being effective for the prevention and/or treatment of a neurodegenerative disease, Alzheimer's disease, systemic amyloidosis, Parkinson's disease, Huntington's disease, a prion disease, multiple sclerosis, and amyotrophic lateral sclerosis.

### (Betaxanthin Synthesis System)

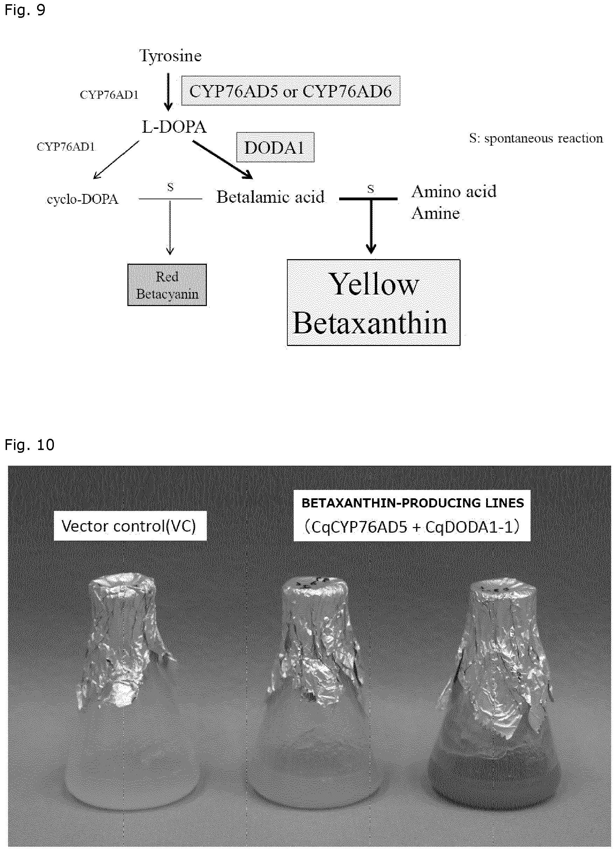

Schemes for betaxanthin biosynthetic pathways in the present invention are illustrated in FIG. 7 and FIG. 9.

As apparent from the illustrations in FIG. 7 and FIG. 9, when a host having an ability to produce tyrosine or 3-hydroxy-L-tyrosine, and an amino acid or an amine (including a host capable of incorporating these compounds from outside) has the following enzyme activities, a betaxanthin can be synthesized.

Enzyme (e.g., CqCYP76AD5 or CqCYP76AD6) activity of having enzyme activity of hydroxylating the 3-position of the phenol ring of tyrosine but being free of L-DOPA oxidase activity and DOPA 4,5-dioxygenase (e.g., CqDODA-1) activity.

According to the results of Examples to be described below, a plant body that is a host having introduced therein a gene group of a gene encoding an enzyme (CqCYP76AD5 or CqCYP76AD6) having enzyme activity of hydroxylating the 3-position of the phenol ring of tyrosine but being free of L-DOPA oxidase activity and a gene encoding an enzyme (CqDODA-1) having DOPA 4,5-dioxygenase activity has an ability to synthesis a betaxanthin.

Alternatively, a betaxanthin may be synthesized as follows: betalamic acid synthesized by a host having the above-mentioned enzyme activity, having an ability to produce tyrosine or 3-hydroxy-L-tyrosine, and being free of an ability to produce an amine is subjected to a spontaneous reaction with an amino acid or an amine as required.

As apparent from the illustration in FIG. 9, the present invention also encompasses the following methods of synthesizing a betaxanthin.
(1) Tyrosine or 3-hydroxy-L-tyrosine is brought into contact with an enzyme having enzyme activity of hydroxylating the 3-position of the phenol ring of tyrosine but being free of L-DOPA oxidase activity to provide L-DOPA, which is then brought into contact with an enzyme having DOPA 4,5-dioxygenase activity to provide betalamic acid, which is then brought into contact with an amine.
(2) A host that has introduced therein a gene group of a gene encoding an enzyme having enzyme activity of hydroxylating the 3-position of the phenol ring of tyrosine but being free of L-DOPA oxidase activity and a gene encoding an enzyme having DOPA 4,5-dioxygenase activity, and that has an ability to produce tyrosine or 3-hydroxy-L-tyrosine and an ability to produce an amino acid and/or an amine is cultured, and a betaxanthin is extracted from the host after the culture.
(3) A host that has introduced therein a gene group of a gene encoding an enzyme having enzyme activity of hydroxylating the 3-position of the phenol ring of tyrosine but being free of L-DOPA oxidase activity and a gene encoding an enzyme having DOPA 4,5-dioxygenase activity, and that has an ability to produce tyrosine or 3-hydroxy-L-tyrosine is cultured, and betalamic acid is extracted from the host after the culture and brought into contact with an amine.

With regard to the "culture" in the method of synthesizing a betaxanthin described above in each of (2) and (3), for example, the host may be cultured in the presence of an exogenous amine corresponding to the target betaxanthin.

In addition, the method of synthesizing a betaxanthin described above in each of (2) and (3) may further include a step of purifying the target betaxanthin from the extracted pigment, or a step of separating the target betaxanthin and a secondary betaxanthin, which is derived from an endogenous amine and/or amino acid, from the extracted pigment.

### (Amine)

The amine of the present invention may be a naturally occurring amine or a non-naturally occurring amine. Examples thereof include putrescine, spermidine, spermine, methylamine, ethanolamine, piperidine, histamine, dopamine, phenethylamine, tyramine, 3-methoxytyramine, γ-aminobutyric acid, noradrenaline, serotonin, muscimol, ethylamine, piperazine, morpholine, ethylenediamine, diethylenetriamine, triethylenetetramine, tetraethylenepentamine, pentaethylenehexamine, hexamethylenediamine, 1,3-butadien-1-amine, 1,3,5-hexatrien-1-amine, aniline, 4-biphenylamine, amantadine, p-aminobenzoic acid (PABA), anthranilic acid (Ant.), 6-aminoindole (6AI), and o-dianisidine (oDA).

### (Betaxanthin)

The betaxanthin of the present invention is not particularly limited, and examples thereof include indicaxanthin, vulgaxanthin I, dopaxanthin, a betaxanthin obtained by a condensation reaction between threonine and betalamic acid, putrescine-betaxanthin, spermidine-betaxanthin, spermine-betaxanthin, methylamine-betaxanthin, ethanolamine-betaxanthin, piperidine-betaxanthin, histamine-betaxanthin, dopamine-betaxanthin, phenethylamine-betaxanthin, tyramine-betaxanthin, 3-methoxytyramine-betaxanthin, γ-aminobutyric acidbetaxanthin, noradrenaline-betaxanthin, serotonin-betaxanthin, muscimolbetaxanthin, ethylamine-betaxanthin, piperazine-betaxanthin, morpholinebetaxanthin, ethylenediamine-betaxanthin, diethylenetriamine-betaxanthin, triethylenetetramine-betaxanthin, tetraethylenepentamine-betaxanthin, pentaethylene hexamine-betaxanthin, hexamethylenediamine-betaxanthin, 1,3-butadien-1-amine-betaxanthin, 1,3,5-hexatrien-1-amine-betaxanthin, anilinebetaxanthin, 4-biphenylamine-betaxanthin, and amantadine-betaxanthin.

### (Enzyme having Enzyme Activity of Hydroxylating 3-Position of Phenol Ring of Tyrosine but being Free of L-DOPA Oxidase Activity)

The enzyme having enzyme activity of hydroxylating the 3-position of the phenol ring of tyrosine but being free of L-DOPA oxidase activity in the present invention may be derived from any species as long as the enzyme has activity of allowing a hydroxy group to be added to the 3-position of the phenol ring that tyrosine has, and is free of L-DOPA oxidase activity.

Examples of the enzyme having enzyme activity of hydroxylating the 3-position of the phenol ring of tyrosine but being free of L-DOPA oxidase activity include cytochromes P450 (in particular, a subfamily β of a CYP76AD group, such as CYP76AD5 and CYP76AD6). Of those, CqCYP76AD5 {CqCYP76AD5a has a base sequence set forth in SEQ ID NO: 50 (accession number: XM_021920495) and an amino acid sequence set forth in SEQ ID NO: 51 (accession number: XP_021776187), and CqCYP76AD5b has a base sequence set forth in SEQ ID NO: 52 (accession number: XM_021861483) and an amino acid sequence set forth in SEQ ID NO: 53 (accession number: XP_021717175)} or CqCYP76AD6 {having a base sequence set forth in SEQ ID NO: 54 (accession number: XM_021861500) and an amino acid sequence set forth in SEQ ID NO: 55 (accession number: XP_021717192)} is preferred.

Examples of the gene encoding the enzyme having enzyme activity of hydroxylating the 3-position of the phenol ring of tyrosine but being free of L-DOPA oxidase activity may include the following:
(1) a gene encoding a polypeptide formed of an amino acid sequence set forth in SEQ ID NO: 51, 53, or 55;
(2) a gene encoding a polypeptide that has 1 to 20 amino acids substituted, deleted, inserted, and/or added in the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
(3) a gene encoding a polypeptide that has 90% or more homology to the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
(4) a gene formed of DNA formed of a base sequence set forth in SEQ ID NO: 50, 52, or 54;
(5) a gene formed of DNA that hybridizes with DNA formed of a base sequence complementary to DNA formed of the base sequence set forth in SEQ ID NO: 50, 52, or 54 under stringent conditions, and that encodes a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity;
(6) a gene formed of DNA having a 1- to 50-base sequence substituted, deleted, inserted, and/or added in DNA formed of the base sequence set forth in SEQ ID NO: 50, 52, or 54;
(7) a gene formed of DNA having 90% or more homology to DNA formed of the base sequence set forth in SEQ ID NO: 50, 52, or 54; and
(8) a gene formed of DNA formed of a degenerate isomer of the base sequence set forth in SEQ ID NO: 50, 52, or 54.

The gene of the above-mentioned item (2) is a gene encoding a polypeptide having introduced therein such a mutation as not to cause the loss of the ability to hydroxylate a 3-position of a phenol ring of tyrosine. Such mutation encompasses an artificial mutation as well as a naturally occurring mutation. As means for causing the artificial mutation, there may be given, for example, a site-directed mutagenesis method (Nucleic Acids Res. 10, 6487-6500, 1982). The number of mutated amino acids is generally 20 or less, preferably 10 or less, more preferably 5 or less, most preferably 3. Whether or not the polypeptide having introduced therein the mutation retains the ability to synthesize amaranthin can be found by, for example, introducing a gene encoding the polypeptide having introduced therein the mutation into a plant body or the like, and checking the ability to synthesize amaranthin in the plant body.

With regard to the gene of the above-mentioned item (3), the "substantially equivalent ability to hydroxylate a 3-position of a phenol ring of tyrosine to that of the amino acid sequence set forth in SEQ ID NO: 51, 53, or 55" may be stronger or weaker in degree of the action as compared to the substantially equivalent ability to synthesize amaranthin to that of the amino acid sequence set forth in SEQ ID NO: 51, 53, or 55. The degree of the action may be, for example, about 50%, about 60%, about 70%, about 80%, about 90%, about 100%, about 110%, about 120%, about 130%, about 140%, or about 150% as compared to the ability of the amino acid sequence set forth in SEQ ID NO: 51, 53, or 55 to synthesize amaranthin.

In addition, the identity may be calculated using the Basic Local Alignment Search Tool at the National Center for Biological Information (BLAST) or the like (using, for example, default, namely initially set, parameters).

The gene of the above-mentioned item (5) is a gene obtained by utilizing hybridization between DNAs. The term "stringent conditions" in this gene refers to conditions under which only specific hybridization occurs and non-specific hybridization does not occur. Such conditions are generally conditions such as hybridization in a buffer containing 5xSSC and 1% SDS at 37°C and washing treatment with a buffer containing 1×SSC and 0.1% SDS at 37°C, preferably conditions such as hybridization in a buffer containing 5xSSC and 1% SDS at 42°C and washing treatment with a buffer containing 0.5xSSC and 0.1% SDS at 42°C, more preferably conditions such as hybridization in a buffer containing 5xSSC and 1% SDS at 65°C and washing treatment with a buffer containing 0.2xSSC and 0.1% SDS at 65°C. Whether or not DNA obtained by utilizing hybridization encodes a polypeptide having activity can be found by, for example, introducing the DNA into a plant body or the like, and checking the ability of the plant body to synthesize amaranthin. The DNA obtained by hybridization generally has high identity to the gene of the above-mentioned item (4) (SEQ ID NO: 50, 52, or 54). The "high identity" refers to 90% or more identity, preferably 95% or more identity, more preferably 98% or more identity.

The gene of the above-mentioned item (6) is a gene formed of DNA having a 1- to 50-base sequence, preferably a 1- to 30-base sequence, more preferably a 1- to 20-base sequence, most preferably a 1- to 10-base sequence, even most preferably a 1- to 5-base sequence substituted, deleted, inserted, and/or added in DNA formed of the base sequence set forth in SEQ ID NO: 50, 52, or 54.

The gene of the above-mentioned item (7) is a gene formed of DNA having 90% or more, preferably 93% or more, more preferably 95% or more, most preferably 98% or more identity to DNA formed of the base sequence set forth in SEQ ID NO: 50, 52, or 54.

The enzyme having enzyme activity of hydroxylating the 3-position of the phenol ring of tyrosine but being free of L-DOPA oxidase activity in the present invention has any one or more amino acid sequences selected from the following:
(1) an amino acid sequence set forth in SEQ ID NO: 51, 53, or 55;
(2) an amino acid sequence that has 1 to 20 amino acids substituted, deleted, inserted, and/or added in the amino acid sequence set forth in SEQ ID NO: 51, 53, or 55, and that forms a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity; and
(3) an amino acid sequence that has 90% or more homology to the amino acid sequence set forth in SEQ ID NO: 51, 53, or 55, and that forms a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity.

In the introduction of a mutation into a peptide, for example, a substitution between homologous amino acids (e.g., polar amino acids, non-polar amino acids, hydrophobic amino acids, hydrophilic amino acids, positively charged amino acids, negatively charged amino acids, and aromatic amino acids) is easily conceivable from the viewpoint of preventing basic properties (e.g., physical properties, function, physiological activity, or immunological activity) of the peptide from being changed.

### (Method of Synthesizing Betaxanthin)

A method of synthesizing a betaxanthin of the present invention may be exemplified by the following.
(1) A host that has introduced therein a gene group of a gene encoding an enzyme having enzyme activity of hydroxylating the 3-position of the phenol ring of tyrosine but being free of L-DOPA oxidase activity and a gene encoding an enzyme having DOPA 4,5-dioxygenase activity, and that has an ability to produce tyrosine or 3-hydroxy-L-tyrosine and an ability to produce an amino acid and/or an amine is cultured, and a betaxanthin is extracted from the host after the culture.
(2) A host that has introduced therein a gene group of a gene encoding an enzyme having enzyme activity of hydroxylating the 3-position of the phenol ring of tyrosine but being free of L-DOPA oxidase activity and a gene encoding an enzyme having DOPA 4,5-dioxygenase activity, and that has an ability to produce tyrosine or 3-hydroxy-L-tyrosine is cultured, and betalamic acid is extracted from the host after the culture and brought into contact with an amine.

### (Betaxanthin-producing Host)

A betaxanthin-producing host of the present invention has, for example, any one or more of the following configurations and features:
(1) the host has introduced therein a gene encoding an enzyme having enzyme activity of hydroxylating the 3-position of the phenol ring of tyrosine but being free of L-DOPA oxidase activity and a gene encoding an enzyme having DOPA 4,5-dioxygenase activity, and has an ability to produce tyrosine or 3-hydroxy-L-tyrosine and an ability to produce an amino acid and/or an amine; and
(2) the host has an ability to produce an amino acid and/or an amine, and has introduced therein a betalamic acid synthesis composition for betaxanthin synthesis.

### (Betalamic acid-producing Host)

A betalamic acid-producing host of the present invention has, for example, any one or more of the following configurations and features:
(1) the host has introduced therein a gene encoding an enzyme having enzyme activity of hydroxylating the 3-position of the phenol ring of tyrosine but being free of L-DOPA oxidase activity and a gene encoding an enzyme having DOPA 4,5-dioxygenase activity, and has an ability to produce tyrosine or 3-hydroxy-L-tyrosine; and
(2) the host has introduced therein a betalamic acid synthesis composition for betaxanthin synthesis.

### (Betalamic Acid Synthesis Composition for Betaxanthin Synthesis)

A betalamic acid synthesis composition for betaxanthin synthesis (betalamic acid synthesis agent for betaxanthin synthesis, or betalamic acid synthetase agent for betaxanthin synthesis) of the present invention contains a gene shown in any one of the following items or a vector carrying the gene:
(1) a gene encoding a polypeptide formed of an amino acid sequence set forth in SEQ ID NO: 51, 53, or 55;
(2) a gene encoding a polypeptide that has 1 to 20 amino acids substituted, deleted, inserted, and/or added in the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
(3) a gene encoding a polypeptide that has 90% or more homology to the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
(4) a gene formed of DNA formed of a base sequence set forth in SEQ ID NO: 50, 52, or 54;
(5) a gene formed of DNA that hybridizes with DNA formed of a base sequence complementary to DNA formed of the base sequence set forth in SEQ ID NO: 50, 52, or 54 under stringent conditions, and that encodes a polypeptide having an ability to hydroxylate a 3-position of a phenol ring of tyrosine and being free of L-DOPA oxidase activity;
(6) a gene formed of DNA having a 1- to 50-base sequence substituted, deleted, inserted, and/or added in DNA formed of the base sequence set forth in SEQ ID NO: 50, 52, or 54;
(7) a gene formed of DNA having 90% or more homology to DNA formed of the base sequence set forth in SEQ ID NO: 50, 52, or 54; and
(8) a gene formed of DNA formed of a degenerate isomer of the base sequence set forth in SEQ ID NO: 50, 52, or 54.

Alternatively, the betalamic acid synthesis composition for betaxanthin synthesis of the present invention has a peptide represented by an amino acid sequence of any one of the following items (1) to (3):
(1) an amino acid sequence set forth in SEQ ID NO: 51, 53, or 55;
(2) an amino acid sequence that has 1 to 20 amino acids substituted, deleted, inserted, and/or added in the amino acid sequence set forth in SEQ ID NO: 51, 53, or 55, and that forms a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity; and
(3) an amino acid sequence that has 90% or more homology to the amino acid sequence set forth in SEQ ID NO: 51, 53, or 55, and that forms a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity.

### Examples

The present invention is hereinafter described in detail by way of specific examples. However, the present invention is not limited to these Examples.

### Example 1

### <Stability of Betalain Pigment>

An aqueous solution of each of celosianin II, amaranthin, and betanin was evaluated for its stability at 37°C.

### [Betalain Pigment Extraction and HPLC Analysis]

For amaranthin and betanin extraction, amaranthin-synthesizing tobacco BYII cells and betanin-synthesizing tobacco BYII cells generated in accordance with a previously reported method were used (Non Patent Literature 4). For celosianin II extraction, a wild-type quinoa individual was used. Each plant sample having celosianin II, amaranthin, or betanin accumulated in the plant body was disrupted, and water was added, followed by centrifugation (20,000 g, 10 minutes or 15 minutes). The centrifuged supernatant was collected, and an equal amount of acetonitrile was added, followed by centrifugation (20,000 g, 10 minutes or 15 minutes). The supernatant obtained by the centrifugal operation was concentrated with a centrifugal concentrator (CC-105, Tomy Seiko). The resultant concentrate was subjected to HPLC analysis as a pigment extract.

The HPLC analysis was performed by: using a reversed-phase column (Shim-pack GWS C18 column, 5 µm; 200 mmx4.6 mm i.d.; Shimadzu GLC); using 0.05% TFA in water as solvent A and 0.05% TFA in acetonitrile as solvent B; setting a flow rate to 0.5 ml/min; setting an analysis program to such a condition that the concentration of acetonitrile was linearly changed from 0% at an analysis time of 0 minutes to reach 45% over 45 minutes; and setting an analytical wavelength to 536 nm allowing betacyanin to be detected.

The purified betalain pigments were subjected to measurement with a UV-2450 spectrophotometer (Shimadzu), followed by the calculation of the concentrations of solutions using the molar extinction coefficient of amaranthin and betanin (ε=54,000 M⁻¹cm⁻¹ at 536 nm) (Gandia-Herrero, F., Escribano, J. and Garcia-Carmona, F. (2010) Structural implications on color, fluorescence, and antiradical activity in betalains. Planta 232, 449-460.; Schwartz, S.J. and Von Elbe, J.H. (1980) Quantitative determination of individual betacyanin pigments by high-performance liquid chromatography. J. Agric. Food Chem. 28, 540-543.).

### [With regard to Stability of Betalain Pigment]

For each of betanin, amaranthin, and celosianin II, a 1 mM aqueous solution was prepared, and left to stand still in an incubator at 37°C. Absorbances at 536 nm on days 0, 1, 2, 3, and 4 were measured, and for the resultant values, relative amounts with respect to the absorbance on day 0 were calculated.

### [Results]

According to the results of the measurement of the residual amounts of the betalain pigments with the absorbances at 536 nm serving as indicators, celosianin was more abundant than amaranthin and betanin. Further, in the case of 4 days of treatment at 37°C, the red colors of betanin and amaranthin disappeared, but celosianin II still exhibited a red color.

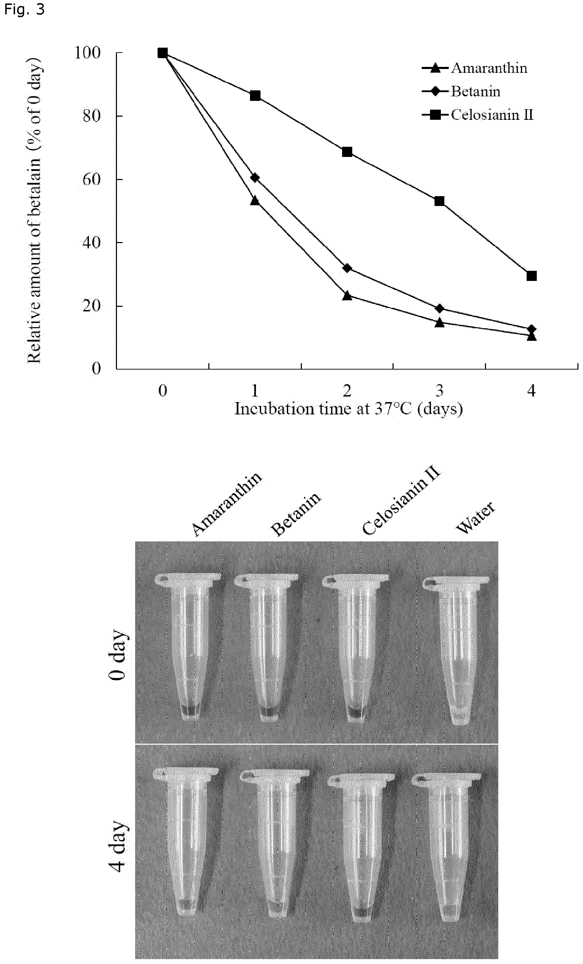

Thus, it was revealed that celosianin II was stable as compared under the condition of 37°C as compared to amaranthin and betanin (FIG. 3).

### Example 2

### <Effect of Celosianin II on HIV-1 Protease Activity>

Whether celosianin II inhibited HIV-1 protease activity was tested.

### [Evaluation of HIV-1 Protease Inhibitory Activity of Betalain Pigment]

A recombinant HIV-1 protease (ab84117) from Abcam was used as an HIV-1 protease, and an HIV-1 protease substrate (Lys-Ala-Arg-Val-Nle-p-nitro-Phe-Glu-Ala-Nle amide) from Sigma-Aldrich was used as a substrate peptide for the HIV-1 protease. HIV-1 protease activity evaluation was performed with reference to a previously reported method (Boso, G., Orvell, C. and Somia, N.V. (2015) The nature of the N-terminal amino acid residue of HIV-1 RNase H is critical for the stability of reverse transcriptase in viral particles. J. Virol. 89, 1286-1297.). Amaranthin, betanin, or celosianin II was added to 16 pmol of the HIV-1 protease and 4 nmol of the HIV-1 protease substrate, a buffer (25 mM NaCl, 25 mM Na₂HPO₄, 1 mM dithiothreitol, pH 4.7) was added so that the amount of a reaction liquid was 30 µl, and a reaction was performed at 25°C for 2 hours. With regard to the addition amount of amaranthin, betanin, or celosianin II, amaranthin, betanin, and celosianin II were each added at the ratio of a 100-fold amount with respect to the amount of the HIV-1 protease. For celosianin II, the ratios of 25- and 50-fold amounts were further evaluated. An HIV-1 inhibitor saquinavir was used as a positive control.

After the reaction, the reaction liquid was measured for the amount of the residual substrate peptide through use of HPLC, and a residual ratio with respect to the substrate before the reaction was calculated. For the HPLC, LC-20AD from Shimadzu was utilized, and a Shim-pack GWS C18 column (5 µm; 200 mmx4.6 mm i.d.; Shimadzu GLC) was used as an analytical column. For an analytical solvent system, 0.05% TFA in water was used as solvent A and 0.05% TFA in acetonitrile was used as solvent B. An HPLC program was set to a linear gradient from 0% of solvent B at 0 minutes to 50% of solvent B at 50 minutes, and the HPLC was performed at a flow rate of 0.5 mL/min and 25°C. Analysis was performed with an analytical wavelength being set to 260 nm allowing the HIV-1 protease substrate peptide to be detected.

### [Results]

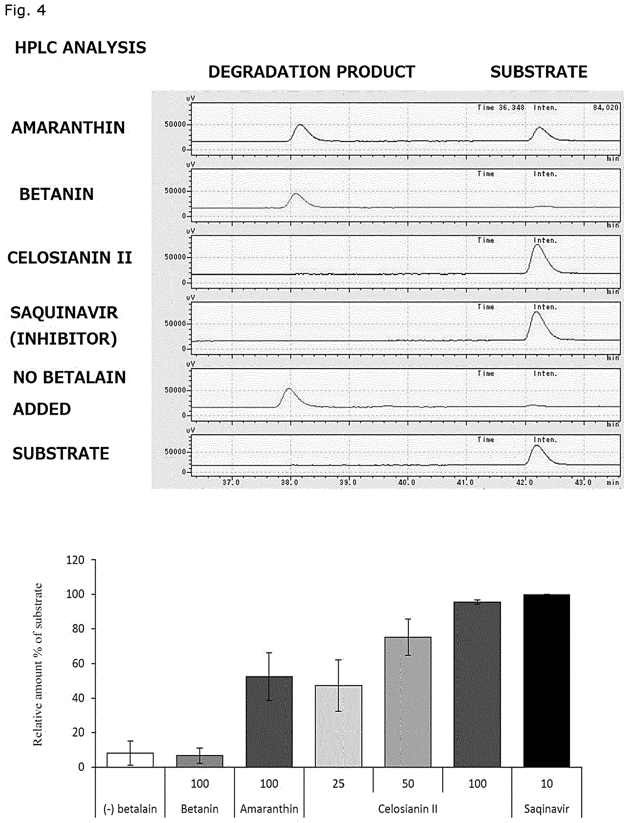

It was revealed that, when added in a 25-fold amount with respect to the HIV-1 protease, celosianin II had HIV-1 protease inhibitory activity comparable to that in the case where a 100-fold amount of amaranthin was added (FIG. 4). That is, it was recognized that celosianin II had an HIV-1 protease activity inhibitory effect 4 times as high as that of amaranthin. Further, the inhibitory activity of celosianin II on the HIV-1 protease was amount-dependent (FIG. 4).

### Example 3

### [Effect of Betalain Pigment on Amyloid-β Polymerization]

With regard to the polymerization of amyloid-β, there is no report about a betalain pigment. The inventors of the present invention evaluated whether celosianin II, amaranthin, and betanin pigments, and ferulic acid, a constituent component of celosianin II, inhibited the polymerization of amyioid-β by a ThT assay method.

### [Evaluation of Amyloid-β Polymerization Inhibitory Activity in Thioflavin T (ThT) Assay]

ThT assay involved evaluating polymerization using SensoLyte Thioflavin T β-Amyloid (1-40) Aggregation Kit (ANASPEC) in accordance with an accompanying protocol.

As an apparatus used for measurement, Varioskan LUX (Thermo Fisher Scientific) was used, and fluorescence derived from the polymerization of amyioid-β was measured for 4 hours at intervals of 5 minutes. A sample was stirred for 15 seconds before each measurement and subjected to the measurement. An excitation wavelength of 440 nm and a detection wavelength of 484 nm were used.

The sample used for the ThT assay was prepared to have a final concentration of 50 µM before being used for the experiment. Tannic acid (Ono, K., Hasegawa, K., Naiki, H. & Yamada, M. (2004) Anti-amyloidogenic activity of tannic acid and its activity to destabilize Alzheimer's beta-amyloid fibrils in vitro. Biochim Biophys Acta 1690, 193-202.) was used as a control of an inhibitor.

### [Results]

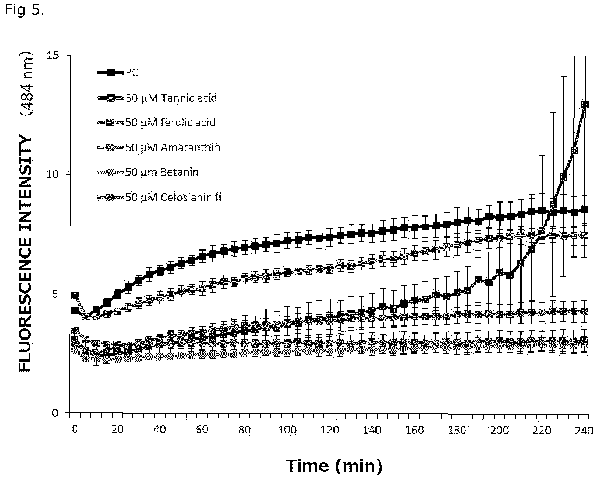

Celosianin II and betanin each showed polymerization inhibitory activity comparable to or higher than that of the existing inhibitor (tannic acid) (FIG. 5). In addition, amaranthin possessed inhibitory activity, though its effect was low as compared to celosianin II and betanin (FIG. 5). Meanwhile, in the case of only ferulic acid, a constituent component of celosianin II, the polymerization inhibitory effect was weaker than those of celosianin II, the betanin pigment, and amaranthin (FIG. 5).

Thus, it was recognized that the betalain pigments each had activity of inhibiting amyloid polymerization.

### Example 4

### <Search for Celosianin II Synthetase Gene>

In order to isolate a celosianin II-synthesizing gene from a quinoa genome, a celosianin II synthetase gene was identified on the basis of an intracellular localization prediction and gene expression analysis. Celosianin II is a substance in which ferulic acid is bonded to glucuronic acid present in the molecule of amaranthin (FIG. 1). In a flavonoid, another plant pigment, enzymes that add ferulic acid analogs, such as sinapinic acid, p-coumaric acid, and gallic acid, to the flavonoid have already been isolated (Bontpart, T., Cheynier, V., Ageorges, A. & Terrier, N. (2015) BAHD or SCPL acyltransferase? What a dilemma for acylation in the world of plant phenolic compounds. New Phytol 208, 695-707.). It is known that the enzymes (acyltransferases) that add those molecules are proteins belonging to the Serine CarboxyPeptidase Like (SCPL) group, and are localized in vacuoles (Bontpart, T., Cheynier, V., Ageorges, A. & Terrier, N. (2015) BAHD or SCPL acyltransferase? What a dilemma for acylation in the world of plant phenolic compounds. New Phytol 208, 695-707.). In view of this, in order to isolate a celosianin II-synthesizing gene from quinoa, first, 87 SCPL group proteins present in the quinoa genome were analyzed using an intracellular localization prediction program (WoLF PSORT).

### [Vacuolar Localization Prediction]

SCPL group proteins registered at NCBI were analyzed using the intracellular localization prediction program (WoLF PSORT, https://wolfpsort.hgc.jp/). On the basis of the results of the analysis, proteins each having a vacuolar localization score of 4 or more were selected as vacuole-localized proteins.

### [Comprehensive Expression Analysis in Quinoa Hypocotyls]

RNA was extracted from hypocotyls of quinoa (*Chenopodium quinoa*) on day 5 of germination through use of an RNeasy Plant Mini kit (Qiagen). A library for next-generation sequencing was constructed from 1 µg of the extracted RNA through use of NEBNext Poly(A) mRNA Magnetic Isolation Module (NEB). The constructed library was sequenced using MiSeq (Illumina). The resultant next-generation sequencing data was aligned to quinoa genomic data through use of HISAT2 (Kim, D., Langmead, B. & Salzberg, S.L. (2015) HISAT: a fast spliced aligner with low memory requirements. Nat Methods 12, 357-360.). A gene prediction was performed using String Tie (Pertea, M. et al. (2015) String Tie enables improved reconstruction of a transcriptome from RNA-seq reads. Nat Biotechnol 33, 290-295.), and an expression amount was determined using feature Counts (Liao, Y., Smyth, G.K. & Shi, W. (2014) feature Counts: an efficient general purpose program for assigning sequence reads to genomic features. Bioinformatics 30, 923-930.).

### [Results]

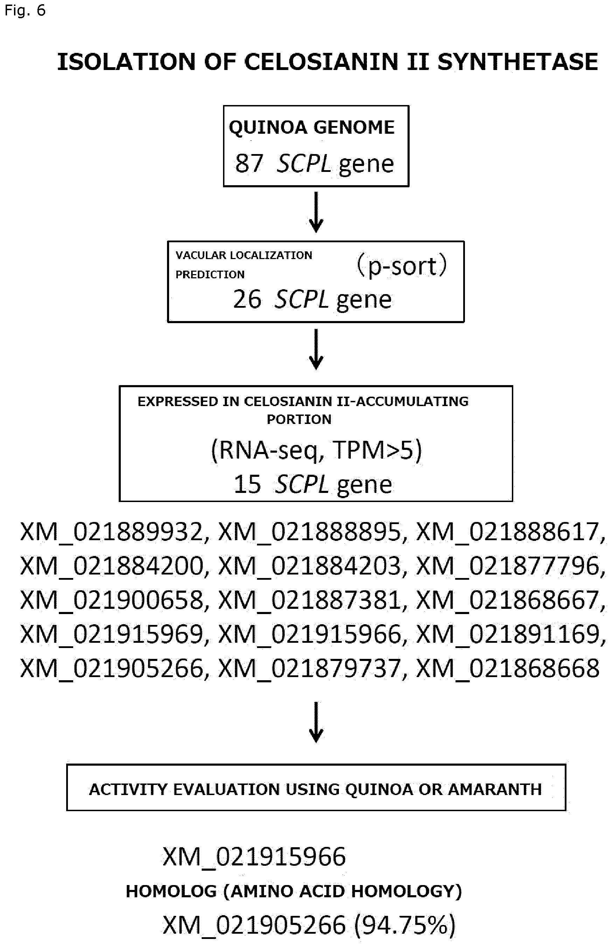

According to the results of the vacuolar localization prediction, there were 26 proteins predicted to be localized in vacuoles (FIG. 6).

Celosianin II is accumulated in hypocotyls, and hence it is predicted that a celosianin II-synthesizing gene is also expressed in hypocotyls. In view of this, as the next selection, the 26 genes predicted to be localized in vacuoles were examined for expression in hypocotyls. For the selection, RNA-seq was performed for hypocotyls of quinoa. On the basis of the resultant RNA-seq data, genes expressed in the hypocotyls were selected (Transcripts Per Kilobase Million (TPM)>5). As a result, it was revealed that 15 genes were expressed in the hypocotyls (FIG. 6).

### Example 5

### <Evaluation of Celosianin II Synthesis Activity of Celosianin II-synthesizing Gene>

For the 15 selected candidate genes, the intensities of activity of synthesizing celosianin II were compared using an evaluation system using quinoa and amaranth.

### [Generation of Plasmid for Plant Expression]

For the expression of betalain pigment biosynthesis genes used in this experiment, a pCAMBIA1301 modification vector (Imamura, T., Takagi, H., Miyazato, A., Ohki, S., Mizukoshi, H. and Mori, M. (2018) Isolation and characterization of the betalain biosynthesis gene involved in hypocotyl pigmentation of the allotetraploid Chenopodiumquinoa. Biochem. Biophys. Res. Commun. 496, 280-286.) was used. For the betalain pigment biosynthesis genes, a fragment in which restriction enzyme sites were added to the 5' and 3' ends of each gene was synthesized by PCR. Then, the resultant PCR fragment was cleaved at the added sites and inserted into the plant modification vector to construct a plant expression vector.

### [Agrobacterium Transformation Method]

The constructed plasmid was introduced from *Escherichia coli* harboring the plasmid into an *Agrobacterium* using a triparental mating method (Wise, A.A., Liu, Z. and Binns, A.N. (2006) Three methods for the introduction of foreign DNA into Agrobacterium. Methods Mol. Biol. 343, 43-53.) to generate a transformed *Agrobacterium.*

### [Evaluation of Celosianin Synthesis Activity using Transient Plant Expression System]

Whether a selected candidate gene possessed an ability to synthesize celosianin II was evaluated. For the experiment, quinoa and amaranth (which does not synthesize celosianin II), which is an Amaranthaceae plant like quinoa and performs synthesis up to amaranthin, a precursor of celosianin II, were used. Those plant bodies were each infected with the transformed *Agrobacterium* to transiently express the candidate gene in the plant body, and the synthesis of celosianin II in the infected plant body was evaluated.

A plasmid for expressing the selected candidate gene in a plant is constructed and introduced into an *Agrobacterium.* The resultant transformed *Agrobacterium* was subjected to shaking culture at 120 rpm in 3 mL of LB medium at 26°C for 1 day. 3 ml of the culture liquid in which the *Agrobacterium* was sufficiently proliferated was diluted with sterilized water to 20 mL, and 20 µL of 10 mg/mL acetosyringone and 10 µL of Tween 20 (surfactant) were added to prepare an infected bacterial liquid. A plant tissue of quinoa or amaranth was placed in the infected bacterial liquid, and a pressure reduction operation was performed using a desiccator for from 10 minutes to 15 minutes to allow the bacterium to infiltrate the plant tissue. The infiltrated plant tissue was transferred to an MS plate and subjected to static culture in a dark place at 23°C for from 3 days to 4 days. A betalain pigment was extracted from the cultured plant tissue, and the ability of the candidate gene to synthesize celosianin II was evaluated by HPLC analysis.

### [Results]

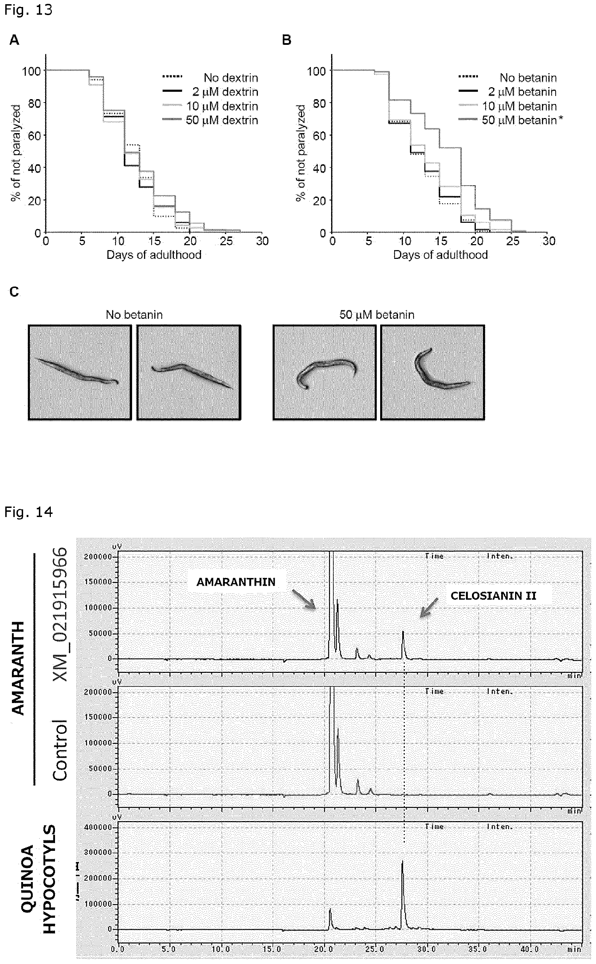

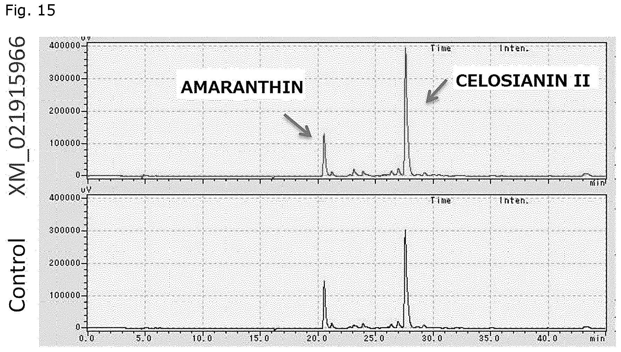

According to the results of the pigment analysis, the accumulation of celosianin II, which was not originally observed in amaranth, was able to be found in the amaranth caused to transiently express a gene (XM_021915966: SEQ ID NOS: 56 and 57) (FIG. 14). Further, in the quinoa caused to have a transient expression, the accumulation amount of celosianin II was increased about 2-fold as compared to the control (FIG. 15). Besides, XM_021905266 (SEQ ID NOS: 58 and 59) has high homology to XM_021915966 (amino acid homology: 94.75%), and hence has a similar effect. Further, 13 kinds belonging to the same SCPL gene family as XM_021915966 (XM_021889932, XM_021888895, XM_021888617, XM_021884200, XM_021884203, XM_021877796, XM_021900658, XM_021887381, XM_021868667, XM_021915969, XM_021891169, XM_021879737, and XM_021868668) also have similar effects.

### Example 6

### <Mass Production of Celosianin II in Tobacco Cultured Cells (BY-2 Cells)>

The celosianin II synthetase gene was successfully isolated, and hence an attempt is made to mass produce celosianin II in tobacco BY-2 cells. In order to produce amaranthin, vectors for expression in BY-2 cells are constructed, and transformants having introduced therein five kinds of betalain biosynthesis genes (CqCYP76AD1-1, CqDODA-1, CqCDOPA5GT, CqAmaSy1, and celosianin II synthetase) are generated. A strongly red line is selected from the resultant transformed BY-2 lines.

### [Analysis of Constitutive Expression in Tobacco BY2 Cells]

The plasmid harbored by the transformed *Agrobacterium* is introduced into tobacco BY2 cells through use of an *Agrobacterium* method (Hagiwara, Y., Komoda, K., Yamanaka, T., Tamai, A., Meshi, T., Funada, R., Tsuchiya, T., Naito, S. and Ishikawa, M. (2003) Subcellular localization of host and viral proteins associated with tobamovirus RNA replication. EMBO J. 22, 344-353.). The resultant transformed line is maintained and utilized for other analyses.

### [Results]

The selected celosianin II-producing line is subjected to HPLC and mass spectrometry to determine the production of celosianin II. In addition, a celosianin II production amount in 150 mL culture is investigated. Through this experiment, betalain pigments (amaranthin and betanin) can be mass produced in tobacco BY-2 cultured cells, i.e., a non-betalain-producing plant.

### Example 7

### <Betaxanthin Production in Tobacco Cultured Cells (BY-2 Cells)>

Through a homology search (Blastp) using the amino acid sequences of beet (*Beta vulgaris*)*-like* genes {BvCYP76AD5 (NCBI accession number: AJD87473) and BvCYP76AD6 (AJD87474)}, CqCYP76AD5 (XM_021920495, XM_021861483), and CqCYP76AD6 (XM_021861500) were selected and identified. Of those, XM_021920495 was used for expression in BY-2 cells.

In order to produce a betaxanthin, a vector for expression in BY-2 cells was constructed, and transformants having introduced therein two kinds of betaxanthin biosynthesis genes (CqCYP76AD5 and CqDODA-1) were generated. From the resultant transformed BY-2 lines, lines exhibiting yellow to orange colors were selected.

### [Analysis of Constitutive Expression in Tobacco BY2 Cells]

The plasmid harbored by the transformed *Agrobacterium* was introduced into tobacco BY2 cells through use of an *Agrobacterium* method (Hagiwara, Y., Komoda, K., Yamanaka, T., Tamai, A., Meshi, T., Funada, R., Tsuchiya, T., Naito, S. and Ishikawa, M. (2003) Subcellular localization of host and viral proteins associated with tobamovirus RNA replication. EMBO J. 22, 344-353.). The resultant transformed line was maintained and utilized for analyses.

### [Results]

The results are shown in FIG. 10.

The tobacco BY-2 cells transformed with CqCYP76AD5 and CqDODA1-1 were changed in color to yellow or orange, indicating that a betaxanthin was synthesized. The expression amounts of CqCYP76AD5 and CqDODA1-1 differ depending on differences in numbers and insertion sites of genes of interest inserted into the genome of the BY-2 cells in transformation, and hence the lines had colors, such as yellow and orange, different from each other. An orange line has a larger amount of a betaxanthin accumulated therein than a yellow one. The tobacco BY2 cells having an empty vector introduced therein remained white, indicating that no betaxanthin was synthesized.

The synthesized betaxanthin is a mixture of betaxanthins, and hence the betaxanthins are identified by performing HPLC and mass spectrometry.

### Example 8

### <Activity Evaluation of Betaxanthin synthesized in Cells>

The activity of a betaxanthin extracted from the betaxanthin-synthesizing tobacco BY-2 cells generated in Example 7 was evaluated.

### [Betaxanthin Extraction]

A BY-2 cell sample having the betaxanthin accumulated in the plant body was disrupted, and ethanol was added, followed by centrifugation (20,000 g, 10 minutes or 15 minutes). The centrifuged supernatant was collected, and a 4-fold amount of ethanol was added, followed by centrifugation (20,000 g, 10 minutes or 15 minutes). The supernatant obtained by the centrifugal operation was concentrated with a centrifugal concentrator (CC-105, Tomy Seiko). The resultant concentrate was used as a betaxanthin extract.

### [Effect of Betaxanthin on Amyloid-β Polymerization]

With regard to the polymerization of amyloid-β, no report about a betaxanthin has been found. Accordingly, whether the betaxanthin inhibits the polymerization of amyioid-β was evaluated by a ThT assay method.

### [Evaluation of Amyloid-β Polymerization Inhibitory Activity in Thioflavin T (ThT) Assay]

ThT assay involved evaluating polymerization using SensoLyte Thioflavin T β-Amyloid (1-40) Aggregation Kit (ANASPEC) in accordance with an accompanying protocol.

As an apparatus used for measurement, Varioskan LUX (Thermo Fisher Scientific) was used, and fluorescence derived from the polymerization of amyioid-β was measured for 4 hours at intervals of 5 minutes. A sample was stirred for 15 seconds before each measurement and subjected to the measurement. An excitation wavelength of 440 nm and a detection wavelength of 484 nm were used.

The sample used for the ThT assay was prepared to have a final concentration of 50 µM before being used for the experiment. An extracted sample from the BY-2 cells having an empty vector introduced therein (VC) and water (PC) in place of a sample were used as controls. In addition, betanin and curcumin were used as positive controls. Curcumin is a plant-derived substance reported to have amyioid-β polymerization inhibitory activity (Yang F, Lim GP, Begum AN, et al. Curcumin inhibits formation of amyloid betaoligomers and fibrils, binds plaques, and reduces amyloid in vivo. J BiolChem. 2005; 280(7): 5892-5901. doi: 10.1074/jbc.M404751200).

### [Results]

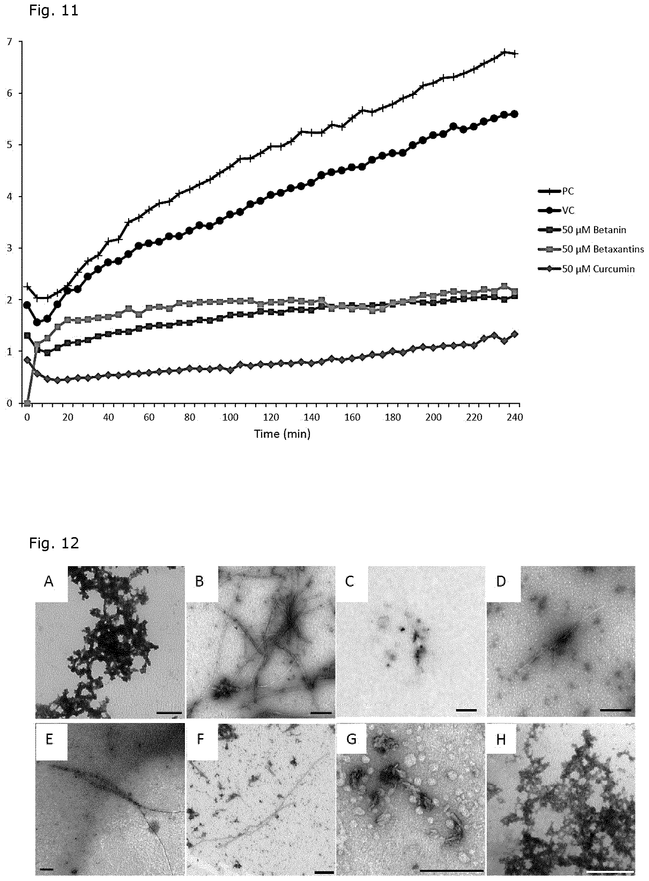

According to the results of the ThT assay, the betaxanthin possessed inhibitory activity comparable to that of betanin (FIG. 11).

Thus, it was recognized that the betaxanthin had activity of inhibiting amyloid polymerization.

### Example 9

### <Observation of Amyloid Peptide using Transmission Electron Microscope>

The influences of betalain pigments on the aggregation of an amyloid peptide were observed with a transmission electron microscope.

A lyophilized amyloid peptide (human amyioid-β 1-40, Aβ40) was purchased from Peptide Institute, Inc., dissolved as a stock solution at a concentration of 1 mM using dimethyl sulfoxide, and stored at -20°C until use.

As a betaxanthin, the betaxanthin extracted from the betaxanthin-producing BY2 cells (FIG. 10) was used.

With regard to reaction conditions, Aβ40 and a sample (betanin, amaranthin, celosianin II, ferulic acid, or the betaxanthin) were prepared with PBS (pH 7.4) so as to have a final concentration of 25 µM and a final concentration of 50 µM, respectively. As a control, distilled water was used in place of the sample. The prepared sample was left to stand still in a dark place at 37°C for 5 days.

The sample after the reaction was stained on a grid mesh for a transmission electron microscope by adding about 2 µL of a 2% uranyl acetate solution thereto. After the solution had dried, the aggregation of the amyloid peptide was observed using a transmission electron microscope (Hitachi 7650 TEM).

### [Results]

According to the results of the observation of the aggregation of the amyloid peptide with the transmission electron microscope, the control sample having added thereto water in place of a betalain pigment was observed to show amorphous aggregation of the amyloid peptide (FIG. 12A) and amyloid fibrils (FIG. 12B).

Meanwhile, when betanin, amaranthin, celosianin II, or ferulic acid (constituent component of celosianin II) was added, only short amyloid fibrils were observed, and hence the aggregation of the amyloid peptide was inhibited (FIG. 12C to FIG. 12G).

In addition, when the betaxanthin was added, amorphous aggregation was observed, but amyloid fibrils were not observed (FIG. 12H).

From the above-mentioned results, it was able to be recognized that betanin, amaranthin, celosianin, and ferulic acid each had an effect of inhibiting both of the polymerization and amorphous aggregation of amyloid fibrils, and the betaxanthin had an effect of inhibiting the polymerization of amyloid fibrils.

### Example 10

### <Test using Nematodes>

In a transgenic nematode expressing an amyloid peptide, aggregates of the amyloid peptide are formed in the body along with its growth period, and the nematode is paralyzed. In view of this, whether feeding with betanin changed a paralysis ratio was evaluated.

Nematodes were grown using NGM plates (nematode growth medium), and grown using *Escherichia coli* (OP50 strain) as food under the condition of 15°C unless otherwise indicated.

Transgenic nematodes (CL2006) expressing an amyloid peptide (human amyloid-β 1-42, Aβ42) were obtained from the Caenorhabditis Genetics Center (CGC), USA. The CL2006 expresses Aβ42 in the cells of the body wall muscle of the nematode, and then aggregation of the amyloid peptide occurs in the muscle. As a result, paralytic symptoms are induced in the CL2006 strain (C.D. Link, Expression of human beta-amyloid peptide in transgenic Caenorhabditis elegans, Proc. Natl. Acad. Sci. U. S. A., 92 (1995) 9368-9372.).

Groups of nematode individuals to be used for the experiment were grown on NGM plates for 2-3 generations without starvation in order that all the individuals were in the same growth stage. Young adult nematodes were collected therefrom and cleaned, and the nematodes were disintegrated using a lysis solution (0.6% sodium hypochlorite and 200 mM sodium hydroxide) to isolate eggs. After from 12 hours to 14 hours at 20°C, the isolated eggs were hatched, and the synchronized L1 larvae were used for the subsequent experiment.

The synchronized L1-stage larvae were grown with the food to young adults at 20°C. 5-Fluorodeoxyuridine (0.5 mg/ml) was added to prevent progeny production during the growth.

The individuals grown to adults through the synchronized growth were transferred to NGM plates containing various concentrations (2 µM, 10 µM, and 50 µM) of food having mixed therein dextrin or betanin, and were grown. After the nematodes had been transferred, the nematodes were each evaluated for paralysis at constant intervals. 20 synchronized adult individuals were transferred per plate. Dextrin was used as a control.

Paralysis was evaluated on the basis of the behavior of the nematode after the nose of the nematode had been lightly tapped with a platinum wire. According to a previous report, an individual that, after the nose of the nematode had been tapped, moved its nose but failed to move its body was considered paralyzed (E. Cohen, J. Bieschke, R.M. Perciavalle et al., Opposing activities protect against ageonset proteotoxicity, Science, 313 (2006) 1604-1610.).

### [Results]

As a result of the experiment using the transgenic nematodes overexpressing the amyloid peptide, the following results were able to be obtained: as compared to the group fed with dextrin (control), the group fed with 50 µM betanin showed significant amelioration (reduction) in paralysis ratio (FIG. 13B). FIG. 13C shows typical examples of paralyzed nematodes in the group fed with dextrin. FIG. 13D shows typical examples of normal nematodes in the group fed with betanin.

According to the above-mentioned results, the following result was able to be obtained: betanin inhibited the aggregation of the amyloid peptide even in vivo.

### Industrial Applicability

According to the present invention, the method of synthesizing celosianin II and the method of synthesizing a betaxanthin can be provided.

## Claims

1. A method of synthesizing celosianin II, comprising:
culturing a host that has introduced therein the following gene encoding a celosianin II synthetase, a gene encoding an enzyme having activity of hydroxylating a 3-position of a phenol ring of tyrosine, a gene encoding an enzyme having L-DOPA oxidase activity and/or a gene encoding an enzyme having DOPA 4,5-dioxygenase activity, a gene encoding an enzyme having activity of glycosylating a phenolic hydroxy group, and a gene encoding an enzyme having activity of adding glucuronic acid, and that has an ability to produce tyrosine or 3-hydroxy-L-tyrosine (L-DOPA), and extracting celosianin II from the host after the culturing; or
culturing a host that has introduced therein the following gene encoding a celosianin II synthetase, and that has enzyme activity of having activity of hydroxylating a 3-position of a phenol ring of tyrosine, L-DOPA oxidase activity and/or DOPA 4,5-dioxygenase activity, enzyme activity of having activity of glycosylating a phenolic hydroxy group, enzyme activity of having activity of adding glucuronic acid, and an ability to produce tyrosine or 3-hydroxy-L-tyrosine, and extracting celosianin II from the host after the culturing,
wherein the gene encoding a celosianin II synthetase is any one or more selected from the following:
(1) a gene encoding a polypeptide formed of an amino acid sequence set forth in SEQ ID NO: 57, 59, 61, 63, 65, 67, 69, 2, 4, 6, 8, 10, 12, 14, or 16;
(2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(4) a gene formed of DNA formed of a base sequence set forth in SEQ ID NO: 56, 58, 60, 62, 64, 66, 68, 1, 3, 5, 7, 9, 11, 13, or 15;
(5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
(6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
(7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
(8) a gene formed of DNA formed of a degenerate isomer of the base sequence set forth in SEQ ID NO: 56, 58, 60, 62, 64, 66, 68, 1, 3, 5, 7, 9, 11, 13, or 15.

2. A method of synthesizing celosianin II, comprising culturing a host that has introduced therein the following gene encoding a celosianin II synthetase and that has an ability to produce amaranthin, and extracting celosianin II from the host after the culturing,
wherein the gene encoding a celosianin II synthetase is any one or more selected from the following:
(1) a gene encoding a polypeptide formed of an amino acid sequence set forth in SEQ ID NO: 57, 59, 61, 63, 65, 67, 69, 2, 4, 6, 8, 10, 12, 14, or 16;
(2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(4) a gene formed of DNA formed of a base sequence set forth in SEQ ID NO: 56, 58, 60, 62, 64, 66, 68, 1, 3, 5, 7, 9, 11, 13, or 15;
(5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
(6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
(7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
(8) a gene formed of DNA formed of a degenerate isomer of the base sequence set forth in SEQ ID NO: 56, 58, 60, 62, 64, 66, 68, 1, 3, 5, 7, 9, 11, 13, or 15.

3. A method of synthesizing celosianin II, comprising:
culturing a host that has introduced therein the following gene encoding a celosianin II synthetase and a gene encoding an enzyme having activity of adding glucuronic acid, and that has an ability to produce betanin, and extracting celosianin II from the host after the culturing; or
culturing a host that has introduced therein the following gene encoding a celosianin II synthetase, and that has enzyme activity of having activity of adding glucuronic acid and an ability to produce betanin, and extracting celosianin II from the host after the culturing, or
wherein the gene encoding a celosianin II synthetase is any one or more selected from the following:
(1) a gene encoding a polypeptide formed of an amino acid sequence set forth in SEQ ID NO: 57, 59, 61, 63, 65, 67, 69, 2, 4, 6, 8, 10, 12, 14, or 16;
(2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(4) a gene formed of DNA formed of a base sequence set forth in SEQ ID NO: 56, 58, 60, 62, 64, 66, 68, 1, 3, 5, 7, 9, 11, 13, or 15;
(5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
(6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
(7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
(8) a gene formed of DNA formed of a degenerate isomer of the base sequence set forth in SEQ ID NO: 56, 58, 60, 62, 64, 66, 68, 1, 3, 5, 7, 9, 11, 13, or 15.

4. A method of synthesizing celosianin II, comprising:
culturing a host that has introduced therein the following gene encoding a celosianin II synthetase, a gene encoding an enzyme having activity of glycosylating a phenolic hydroxy group, and a gene encoding an enzyme having activity of adding glucuronic acid, and that has an ability to produce betanidin, and extracting celosianin II from the host after the culturing; or
culturing a host that has introduced therein the following gene encoding a celosianin II synthetase, and that has enzyme activity of having activity of glycosylating a phenolic hydroxy group, enzyme activity of having activity of adding glucuronic acid, and an ability to produce betanidin, and extracting celosianin II from the host after the culturing,
wherein the gene encoding a celosianin II synthetase is any one or more selected from the following:
(1) a gene encoding a polypeptide formed of an amino acid sequence set forth in SEQ ID NO: 57, 59, 61, 63, 65, 67, 69, 2, 4, 6, 8, 10, 12, 14, or 16;
(2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(4) a gene formed of DNA formed of a base sequence set forth in SEQ ID NO: 56, 58, 60, 62, 64, 66, 68, 1, 3, 5, 7, 9, 11, 13, or 15;
(5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
(6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
(7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
(8) a gene formed of DNA formed of a degenerate isomer of the base sequence set forth in SEQ ID NO: 56, 58, 60, 62, 64, 66, 68, 1, 3, 5, 7, 9, 11, 13, or 15.

5. The method of synthesizing celosianin II according to any one of claims 1 to 4, wherein the gene encoding a celosianin II synthetase is any one or more selected from genes formed of DNAs formed of the base sequences set forth in SEQ ID NOS: 56, 58, 60, 62, 64, 66, 68, 1, 3, 5, 7, 9, 11, 13, and 15.

6. A celosianin II synthesis composition, comprising a gene shown in any one of the following items or a vector carrying the gene:
(1) a gene encoding a polypeptide formed of an amino acid sequence set forth in SEQ ID NO: 57, 59, 61, 63, 65, 67, 69, 2, 4, 6, 8, 10, 12, 14, or 16;
(2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(4) a gene formed of DNA formed of a base sequence set forth in SEQ ID NO: 56, 58, 60, 62, 64, 66, 68, 1, 3, 5, 7, 9, 11, 13, or 15;
(5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
(6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15;
(7) a gene formed of DNA having 90% or more homology to DNA formed of the base sequence set forth in SEQ ID NO: 56, 58, 60, 62, 64, 66, 68, 1, 3, 5, 7, 9, 11, 13, or 15; and
(8) a gene formed of DNA formed of a degenerate isomer of the base sequence set forth in SEQ ID NO: 56, 58, 60, 62, 64, 66, 68, 1, 3, 5, 7, 9, 11, 13, or 15.

7. A celosianin II synthesis composition, comprising a peptide represented by any one of the following amino acid sequences:
(1) an amino acid sequence set forth in SEQ ID NO: 57, 59, 61, 63, 65, 67, 69, 2, 4, 6, 8, 10, 12, 14, or 16;
(2) an amino acid sequence that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that forms a polypeptide having a substantially equivalent ability to synthesize celosianin II to that of the amino acid sequence set forth in SEQ ID NO: 57, 59, 61, 63, 65, 67, 69, 2, 4, 6, 8, 10, 12, 14, or 16; and
(3) an amino acid sequence that has 90% or more homology to the amino acid sequence set forth in SEQ ID NO: 57, 59, 61, 63, 65, 67, 69, 2, 4, 6, 8, 10, 12, 14, or 16, and that forms a polypeptide having a substantially equivalent ability to synthesize celosianin II to that of the amino acid sequence set forth in SEQ ID NO: 57, 59, 61, 63, 65, 67, 69, 2, 4, 6, 8, 10, 12, 14, or 16.

8. A celosianin II-producing host having introduced therein the synthesis composition of claim 6 or 7.

9. A method of synthesizing celosianin II, comprising the following (1) or (2):
(1) a step of bringing amaranthin into contact with the celosianin II synthesis composition of claim 7; or
(2)
(a) a step of bringing betanin into contact with an enzyme having activity of adding glucuronic acid, and
(b) a step of bringing amaranthin obtained in the step (a) into contact with the celosianin II synthesis composition of claim 7.

10. A celosianin II-producing host,
the host having introduced therein the following gene encoding a celosianin II synthetase, a gene encoding an enzyme having activity of hydroxylating a 3-position of a phenol ring of tyrosine, a gene encoding an enzyme having L-DOPA oxidase activity and/or a gene encoding an enzyme having DOPA 4,5-dioxygenase activity, a gene encoding an enzyme having activity of glycosylating a phenolic hydroxy group, and a gene encoding an enzyme having activity of adding glucuronic acid, and having an ability to produce tyrosine or L-DOPA, or
the host having introduced therein the following gene encoding a celosianin II synthetase, and having enzyme activity of having activity of hydroxylating a 3-position of a phenol ring of tyrosine, L-DOPA oxidase activity and/or DOPA 4,5-dioxygenase activity, enzyme activity of having activity of glycosylating a phenolic hydroxy group, enzyme activity of having activity of adding glucuronic acid, and an ability to produce tyrosine or 3-hydroxy-L-tyrosine,
wherein the gene encoding a celosianin II synthetase is any one or more selected from the following:
(1) a gene encoding a polypeptide formed of an amino acid sequence set forth in SEQ ID NO: 57, 59, 61, 63, 65, 67, 69, 2, 4, 6, 8, 10, 12, 14, or 16;
(2) a gene encoding a polypeptide that has 1 to 20 amino acids substituted, deleted, inserted, and/or added in the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(3) a gene encoding a polypeptide that has 90% or more homology to the amino acid sequence set forth in SEQ ID NO: 57, 59, 61, 63, 65, 67, 69, 2, 4, 6, 8, 10, 12, 14, or 16, and that has a substantially equivalent ability to synthesize celosianin II to that of the amino acid sequence set forth in SEQ ID NO: 57, 59, 61, 63, 65, 67, 69, 2, 4, 6, 8, 10, 12, 14, or 16;
(4) a gene formed of DNA formed of a base sequence set forth in SEQ ID NO: 56, 58, 60, 62, 64, 66, 68, 1, 3, 5, 7, 9, 11, 13, or 15;
(5) a gene formed of DNA that hybridizes with DNA formed of a base sequence complementary to DNA formed of the base sequence set forth in SEQ ID NO: 56, 58, 60, 62, 64, 66, 68, 1, 3, 5, 7, 9, 11, 13, or 15 under stringent conditions, and that encodes a polypeptide having an ability to synthesize celosianin II;
(6) a gene formed of DNA having a 1- to 50-base sequence substituted, deleted, inserted, and/or added in DNA formed of the base sequence set forth in SEQ ID NO: 56, 58, 60, 62, 64, 66, 68, 1, 3, 5, 7, 9, 11, 13, or 15; and
(7) a gene formed of DNA having 90% or more homology to DNA formed of the base sequence set forth in SEQ ID NO: 56, 58, 60, 62, 64, 66, 68, 1, 3, 5, 7, 9, 11, 13, or 15.

11. An amyloid-β polymerization inhibitor or a therapeutic or preventive agent for Alzheimer's, comprising any one of the following:
(1) celosianin II;
(2) amaranthin;
(3) betanin;
(4) ferulic acid; and
(5) a betaxanthin.

12. An amyloid peptide aggregation inhibitor, comprising any one of the following:
(1) celosianin II;
(2) amaranthin;
(3) betanin;
(4) ferulic acid; and
(5) a betaxanthin.

13. A preventive and/or therapeutic agent for a neurodegenerative disease, Alzheimer's disease, systemic amyloidosis, Parkinson's disease, Huntington's disease, a prion disease, multiple sclerosis, or amyotrophic lateral sclerosis, the preventive and/or therapeutic agent comprising the amyloid peptide aggregation inhibitor of claim 12 as an active ingredient.

14. A method of synthesizing a betaxanthin, comprising:
culturing, with addition of an amine, a host that has introduced therein a gene encoding an enzyme having enzyme activity of hydroxylating a 3-position of a phenol ring of tyrosine but being free of L-DOPA oxidase activity, and a gene encoding an enzyme having DOPA 4,5-dioxygenase activity, and that has an ability to produce tyrosine or 3-hydroxy-L-tyrosine, and extracting a betaxanthin from the host after the culturing; or
culturing, with addition of an amine, a host that has introduced therein a gene encoding an enzyme having enzyme activity of hydroxylating a 3-position of a phenol ring of tyrosine but being free of L-DOPA oxidase activity, and that has enzyme activity of having DOPA 4,5-dioxygenase activity, and an ability to produce tyrosine or 3-hydroxy-L-tyrosine, and extracting a betaxanthin from the host after the culturing; or
culturing a host that has introduced therein a gene encoding an enzyme having enzyme activity of hydroxylating a 3-position of a phenol ring of tyrosine but being free of L-DOPA oxidase activity, a gene encoding an enzyme having DOPA 4,5-dioxygenase activity, and an amine synthesis gene, and that has an ability to produce tyrosine or 3-hydroxy-L-tyrosine, and extracting a betaxanthin from the host after the culturing; or
culturing a host that has introduced therein a gene encoding an enzyme having enzyme activity of hydroxylating a 3-position of a phenol ring of tyrosine but being free of L-DOPA oxidase activity, and that has enzyme activity of having DOPA 4,5-dioxygenase activity, enzyme activity of having amine synthesis activity, and an ability to produce tyrosine or 3-hydroxy-L-tyrosine, and extracting a betaxanthin from the host after the culturing,
wherein the gene encoding an enzyme having enzyme activity of hydroxylating a 3-position of a phenol ring of tyrosine but being free of L-DOPA oxidase activity is any one or more selected from the following:
(1) a gene encoding a polypeptide formed of an amino acid sequence set forth in SEQ ID NO: 51, 53, or 55;
(2) a gene encoding a polypeptide that has 1 to 20 amino acids substituted, deleted, inserted, and/or added in the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
(3) a gene encoding a polypeptide that has 90% or more homology to the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
(4) a gene formed of DNA formed of a base sequence set forth in SEQ ID NO: 50, 52, or 54;
(5) a gene formed of DNA that hybridizes with DNA formed of a base sequence complementary to DNA formed of the base sequence set forth in SEQ ID NO: 50, 52, or 54 under stringent conditions, and that encodes a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity;
(6) a gene formed of DNA having a 1- to 50-base sequence substituted, deleted, inserted, and/or added in DNA formed of the base sequence set forth in SEQ ID NO: 50, 52, or 54;
(7) a gene formed of DNA having 90% or more homology to DNA formed of the base sequence set forth in SEQ ID NO: 50, 52, or 54; and
(8) a gene formed of DNA formed of a degenerate isomer of the base sequence set forth in SEQ ID NO: 50, 52, or 54.

15. The method of synthesizing a betaxanthin according to claim 14, wherein the gene encoding an enzyme having enzyme activity of hydroxylating a 3-position of a phenol ring of tyrosine but being free of L-DOPA oxidase activity is any one or more selected from SEQ ID NOS: 50, 52, and 54.

16. A betalamic acid synthesis composition for betaxanthin synthesis, comprising a gene shown in any one of the following items or a vector carrying the gene:
(1) a gene encoding a polypeptide formed of an amino acid sequence set forth in SEQ ID NO: 51, 53, or 55;
(2) a gene encoding a polypeptide that has 1 to 20 amino acids substituted, deleted, inserted, and/or added in the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
(3) a gene encoding a polypeptide that has 90% or more homology to the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
(4) a gene formed of DNA formed of a base sequence set forth in SEQ ID NO: 50, 52, or 54;
(5) a gene formed of DNA that hybridizes with DNA formed of a base sequence complementary to DNA formed of the base sequence set forth in SEQ ID NO: 50, 52, or 54 under stringent conditions, and that encodes a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity;
(6) a gene formed of DNA having a 1- to 50-base sequence substituted, deleted, inserted, and/or added in DNA formed of the base sequence set forth in SEQ ID NO: 50, 52, or 54;
(7) a gene formed of DNA having 90% or more homology to DNA formed of the base sequence set forth in SEQ ID NO: 50, 52, or 54; and
(8) a gene formed of DNA formed of a degenerate isomer of the base sequence set forth in SEQ ID NO: 50, 52, or 54.

17. A betalamic acid synthesis composition for betaxanthin synthesis, comprising a peptide represented by any one of the following amino acid sequences:
(1) an amino acid sequence set forth in SEQ ID NO: 51, 53, or 55;
(2) an amino acid sequence that has 1 to 20 amino acids substituted, deleted, inserted, and/or added in the amino acid sequence set forth in SEQ ID NO: 51, 53, or 55, and that forms a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity; and
(3) an amino acid sequence that has 90% or more homology to the amino acid sequence set forth in SEQ ID NO: 51, 53, or 55, and that forms a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity.

18. A betaxanthin-producing host having introduced therein the synthesis composition of claim 16 or 17.

19. A betaxanthin-producing host,
the host having introduced therein a gene encoding an enzyme having enzyme activity of hydroxylating a 3-position of a phenol ring of tyrosine but being free of L-DOPA oxidase activity, and a gene encoding an enzyme having DOPA 4,5-dioxygenase activity, and having an ability to produce tyrosine or 3-hydroxy-L-tyrosine, or
the host having introduced therein a gene encoding an enzyme having enzyme activity of hydroxylating a 3-position of a phenol ring of tyrosine but being free of L-DOPA oxidase activity, and having enzyme activity of having DOPA 4,5-dioxygenase activity, and an ability to produce tyrosine or 3-hydroxy-L-tyrosine,
wherein the gene encoding an enzyme having enzyme activity of hydroxylating a 3-position of a phenol ring of tyrosine but being free of L-DOPA oxidase activity is any one or more selected from the following:
(1) a gene encoding a polypeptide formed of an amino acid sequence set forth in SEQ ID NO: 51, 53, or 55;
(2) a gene encoding a polypeptide that has 1 to 20 amino acids substituted, deleted, inserted, and/or added in the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
(3) a gene encoding a polypeptide that has 90% or more homology to the amino acid sequence set forth in SEQ ID NO: 51, 53, or 55, and that has a substantially equivalent ability to hydroxylate a 3-position of a phenol ring of tyrosine to that of the amino acid sequence set forth in SEQ ID NO: 51, 53, or 55 and is free of L-DOPA oxidase activity;
(4) a gene formed of DNA formed of a base sequence set forth in SEQ ID NO: 50, 52, or 54;
(5) a gene formed of DNA that hybridizes with DNA formed of a base sequence complementary to DNA formed of the base sequence set forth in SEQ ID NO: 50, 52, or 54 under stringent conditions, and that encodes a polypeptide having a substantially equivalent ability to hydroxylate a 3-position of a phenol ring of tyrosine to that of the amino acid sequence set forth in SEQ ID NO: 51, 53, or 55 and being free of L-DOPA oxidase activity;
(6) a gene formed of DNA having a 1- to 50-base sequence substituted, deleted, inserted, and/or added in DNA formed of the base sequence set forth in SEQ ID NO: 50, 52, or 54;
(7) a gene formed of DNA having 90% or more homology to DNA formed of the base sequence set forth in SEQ ID NO: 50, 52, or 54; and
(8) a gene formed of DNA formed of a degenerate isomer of the base sequence set forth in SEQ ID NO: 50, 52, or 54.

20. An HIV-1 protease activity inhibitor, comprising any one of the following:
(1) celosianin II;
(2) celosianin II obtained by the method of synthesizing celosianin II of any one of claims 1 to 5 and 9; and
(3) celosianin II obtained from the celosianin II-producing host of claim 8 or
